(19) Europäisches Patentamt
European Patent Office
Office européen des brevets

(11) EP 4 331 610 A1

(12) # EUROPEAN PATENT APPLICATION

(43) Date of publication:
**06.03.2024 Bulletin 2024/10**

(21) Application number: **22193416.9**

(22) Date of filing: **01.09.2022**

(51) International Patent Classification (IPC):
***A61K 47/59*** *(2017.01)* ***A61K 47/64*** *(2017.01)*
***A61K 47/68*** *(2017.01)* ***A61P 35/00*** *(2006.01)*
***A61P 7/00*** *(2006.01)*

(52) Cooperative Patent Classification (CPC):
**A61K 47/595; A61K 47/644; A61K 47/6455; A61K 47/6849; A61P 7/00; A61P 35/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Sapreme Technologies B.V.**
  **3721 MA Bilthoven (NL)**
- **Charité - Universitätsmedizin Berlin**
  **10117 Berlin (DE)**
- **Freie Universität Berlin**
  **14195 Berlin (DE)**
- **Universidade de Santiago de Compostela**
  **15782 Santiago de Compostela (ES)**

(72) Inventors:
- **POSTEL, Ruben**
  **3721 MA Bilthoven (NL)**
- **FUCHS, Hendrik**
  **10117 Berlin (DE)**
- **NAGEL, Gregor**
  **10117 Berlin (DE)**
- **WENG, Alexander**
  **14195 Berlin (DE)**
- **TRÖGER, Meike Karen**
  **14195 Berlin (DE)**
- **CORREA CHINEA, Juan Francisco**
  **15782 Santiago de Compostela (ES)**
- **FERNÁNDEZ MEGÍA, Eduardo Pedro**
  **15782 Santiago de Compostela (ES)**
- **LÓPEZ BLANCO, Roi**
  **15782 Santiago de Compostela (ES)**

(74) Representative: **Nederlandsch Octrooibureau**
**P.O. Box 29720**
**2502 LS The Hague (NL)**

Remarks:
The complete document including Reference Table(s) and the Sequence Listing(s) can be downloaded from the EPO website

(54) **POLYPLEX-SAPONIN COVALENT CONJUGATE**

(57) The invention lies in the field of delivery of nucleic acids into a cell. In particular, disclosed herein is a nucleic acid that is polyplexed with a polymeric scaffold, which scaffold is covalently bound to an endosomal escape-enhancing saponin by a linker configured to release the saponin from the scaffold under conditions present in an endosome. Surprisingly, the presence of the saponin and the linker does not interfere with or affect the ability of the polymeric scaffold to form or maintain the polyplex in the presence of the nucleic acid. Furthermore, such saponin-containing polyplexes not only enhance the cytosolic delivery of the nucleic acid into cells but also do not negatively impact the viability of these cells. The disclosed herein compositions and methods may further be cell-targeted by conjugation to cell- and/or tissue-specific ligands, and, advantageously, exploited in the treatment of various diseases and/or conditions by systemic delivery.

**FIG. 14**

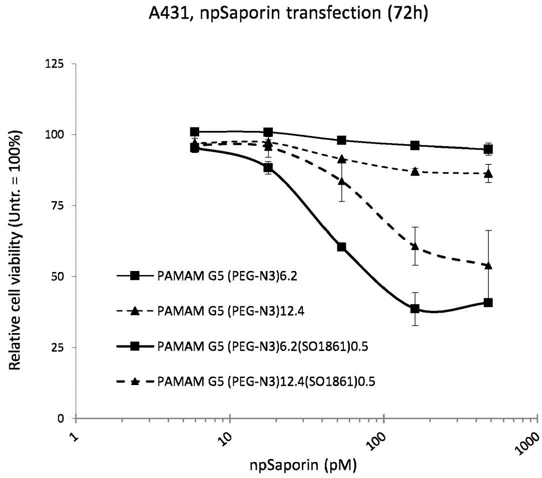


EP 4 331 610 A1

## Description

*The project leading to this application has received funding from the European Union's Horizon 2020 research and innovation programme under grant agreement No 825730.*

## FIELD

The invention lies in the field of delivery of nucleic acids into a cell. In particular, disclosed herein is a nucleic acid that is polyplexed with a polymeric scaffold, which scaffold is covalently bound to an endosomal escape-enhancing saponin by a linker configured to release the saponin from the scaffold under conditions present in an endosome. Surprisingly, the presence of the saponin and the linker does not interfere with or affect the ability of the polymeric scaffold to form or maintain the polyplex in the presence of the nucleic acid. Furthermore, such saponin-containing polyplexes not only enhance the cytosolic delivery of the nucleic acid into cells but also do not negatively impact the viability of these cells. The disclosed herein compositions and methods may further be cell-targeted by conjugation to cell- and/or tissue-specific ligands, and, advantageously, exploited in the treatment of various diseases and/or conditions by systemic delivery.

## BACKGROUND

Gene therapy is one of the most promising treatment options for future advanced therapies in a broad range of diseases. These include replacement of defective genes in inherited diseases (George et al., 2017), elimination of diseased cells such as cancer cells by suicide genes (Sama et al., 2018), gene repair or modification by CRISPR/Cas technology (Mali, et al. 2013), and gene control by micro ribonucleic acid (RNA) methodologies (Berkhout and Liu, 2014), for instance, to improve the body's response against acquired diseases. Currently, *in vivo* delivery of genes is predominantly accomplished via viral vectors (Lee et al., 2017), which still have major safety concerns. Importantly, their manufacturing process is complex and involves substantial production costs, especially on a large scale, which hinders their commercial implementation for the treatment of diseases with large patients' cohorts. Furthermore, viral gene carriers tend to induce adverse immune responses to viral structural proteins (Kwoh et al., 1999). Consequently, alternatives to viral delivery methods for gene therapeutics are intensively being sought for, but the existing approaches all face a major bottleneck caused by poor cytosolic and nucleosolic uptake rates of nucleic acids when delivered to or even into the cell (He et al., 2013).

Nucleic acids (NAs) are polymers of nucleotides - each nucleotide comprising a pentose sugar, a phosphate group and either purine or pyrimidine. The term "transfection" is generally understood as referring to the delivery into cells of an organism of a nucleic acid (Agarwal et al., 2012), for example ribonucleic acid (RNA) or deoxyribonucleic acid (DNA), or modified and/or synthetic equivalents thereof, typically carrying a "genetic information" such as a gene or a part thereof (e.g. an exon or a number thereof). To be able to carry such genetic information, gene therapeutics will typically involve relatively large genetic constructs encompassing more than thousand, frequently several thousands of consecutive nucleotides ("kilobases") or nucleotide pairs ("kbp" i.e. kilo base pairs). Naturally, transfection of such large and, furthermore, charged macromolecular compounds over the cellular membrane remains challenging.

To enable cellular delivery of large nucleic acids, typically, stable synthetic carriers with highaffinity to NAs are employed, such as cationic polymers as well as cationic lipids (He et al., 2013). Such cationic polymers have the ability to spontaneously form through the electrostatic condensation interpolyelectrolyte high-density complexes with NAs, termed polyplexes (Hess et al., 2017; Kabanov and Kabanov, 1995; Rumschöttel et al., 2016). As an illustration, polyplexes can be formed when DNA is mixed with a cationic polymer like poly(lysine) (PLL or polyK) or poly-D-lysine (PDL) (Wu and Wu, 1987; Vasiliu et al., 2017, Kwoh et al., 1999, Clifford et al., 2019), or another very commonly used poly(ethylenimine) (PEI) (Démoulins et al., 2016). These and many other cationic polymers are known to easily form polyplexes with NAs, notably including, to name a few more, polyamidoamine dendrimers (PAMAMs, Koping-Hoggard et al., 2004), chitosan (Koping-Hoggard et al., 2004, Puras et al., 2013), 2-dimethyl(aminoethyl) methacrylate (pDMAEM), and other natural or synthetic DNA-binding proteins or polypeptides (Tros de Ilarduya et al., 2010). Depending on the desired property or purpose for a given polyplex, like route of administration or half-life in plasma, there exist many different chemical modifications that have been tried and applied to various cationic polyplex-forming polymers. For example, instances of modified PLL particles have been reported for enhanced DNA delivery by e.g. addition of PEG groups (Kozielski et al., 2016, Ziady et al., 2003). Regardless of their chemical modification status, and because of their ability to condense and stabilise NA payloads in the form of a high-density polyplex, the cationic polymer carriers are frequently referred to as "scaffolds" or, simply, polyplexing agents.

The condensed nature of polyplexes leads to their easier cell internalization and enhanced protection from enzymatic degradation (Hess et al., 2017). Several mechanisms through which polyplexes interact with cells have been suggested. For example, it has been postulated that they may interact with the cell surface via electrostatic and non-specific interactions (Tros de Ilarduya et al., 2010). Despite the above, the therapeutic efficiency of polyplexes *in vivo* remains unsatisfactorily poor compared to viral vectors. Without wishing to be bound to any theory, it is possible that like with other non-viral approaches, this effect could potentially be related not to the inability of a polyplex to enter the cell *per se* but possibly to an insufficient endosomal escape after the engulfment by the cell.

Indeed, it has been estimated that the inability to escape the endosomal/lysosomal compartment within the cell remains an insurmountable hurdle against successful implementation of gene therapeutics in clinical settings. This perhaps is best reflected by the fact that more than 98% of genetic material that enters the cell was estimated to be transported and degraded in the lysosomes (Gilleron et al., 2013). Due to this effect, only limited and typically insufficient amounts of nucleic acid-based therapeutics manage to successfully enter the cytosol.

In line with the above, polyplexes were suggested to be transported into cells via endocytosis or endocytosis-like mechanisms and, once inside, when endosome pH decreases from pH 7 to 5.5, it was hypothesised that a part of the polyplex-bound nucleic acid dissociates from the polyplex and egresses into the cytosol (Tros de Ilarduya et al., 2010). For example, poly-lysine (poly-K) scaffolds were hypothesised to possess a very weak transfection efficiency because of their inefficient release from the endosomes (Zhang et al., 2010). Furthermore, as poly-K scaffolds do not possess a hydrophobic domain, they are inherently incapable of fusing with or destabilizing the endosome (Tros de Ilarduya et al., 2010).

In addition to the challenges related to insufficient cytosolic and nucleosolic uptake of gene therapeutics, in particular those of substantial sizes above one or more kilobases, successful gene therapy *in vivo* further frequently requires targeted delivery into specific tissue in which the gene therapy is supposed to take effect. These and other challenges are hereby addressed, as explained in the continuation.

## SUMMARY

To address the drawbacks of the prior art, the present disclosure aims to provide a non-viral gene therapy delivery system that will overcome the major and longstanding bottleneck in the field being the inefficient delivery of nucleic acid-based therapeutics across the endosomal membrane into the cytosol/nucleosol.

The presented herein universal gene delivery system is based on novel polyplex-saponin covalent conjugates wherein a potent endosomal escape enhancing (EEE) saponin is covalently bound to a polymeric scaffold that forms a polyplex with a nucleic acid, by a linker adapted to cleave and release the saponin from the polymeric scaffold under the specific conditions present in an endosome or a lysosome.

In line with the above, in a first general aspect, disclosed herein is a polyplex comprising

at least one nucleic acid,

and a polyplexing agent, wherein the polyplexing agent comprises

a polymeric scaffold, and

a saponin being a triterpenoid 12,13-dehydrooleanane-type saponin that in an unreacted state comprises an aldehyde group at position C-23 of the saponin's aglycone core structure,

wherein the saponin is covalently bound with the polymeric scaffold by a linker adapted to cleave and release the saponin from the polymeric scaffold under conditions present in an endosome or a lysosome.

The disclosed herein polyplexes and polyplexing agents comprise a potent EEE saponin bound by an acid-sensitive or endosomal-enzyme-cleavage-specific linker to a polymeric scaffold that has a high affinity to nucleic acids. Thanks to such designed linker, the EEE saponin will only, in theory, be released once the polyplex is in the endosome, which will ensure synchronisation of its destabilising effect on the endosomal membrane with the moment at which the nucleic acid is also present in the endosome.

Without wishing to be bound by any theory, the inventors hypothesised that specific inclusion by conjugation of the 12,13-dehydrooleanane type triterpenoid saponins as part of the polyplexing agents could stimulate efficient exiting of the therapeutic nucleic acids within the polyplex by such agents from the endosomes (in a very much desired for therapeutics but not fully understood phenomenon termed *endosomal escape)* to the cytosol. To the inventors' knowledge, such covalent conjugation of EEE saponins to a polymeric scaffold has never been tried and tested for the ability of such obtained novel polyplexing agent to successfully form a polyplex with a nucleic acid, and/or to stably maintain the nucleic acid under conditions similar to those present in cell culture and, most importantly, *in vivo,* like in blood plasma.

To the inventors' surprise, covalent conjugation of EEE saponins to polymeric scaffolds via acid-sensitive linkers did not impair the polymeric scaffold's ability to form a polyplex, and tests performed in human blood samples (shown below) indicated that such saponin-equipped polyplexes were stable in blood plasma and deprived from haemolytic

activity. Consequently, a seemingly safe and stable promising 1-component non-viral polyplexation-based system for nucleic acid delivery was developed with internally-incorporated endosomal escape enhancing properties for the increased efficacy of cytosolic delivery.

Advantageously, the system was further modified to provide an interface for linking ligands for targeted delivery into cells of choice, thus leading to creation of a targeted 1-component non-viral system wherein an endosomal escape enhancer, gene therapeutic product, and targeting ligand are all bound to one (polymeric) molecular scaffold. Such developed system has plenty of advantages for potential future simplified clinical applicability and receiving a streamlined therapeutic approval.

The above-discussed advantages suggest that the developed herein saponin-conjugated polyplexes and targeted saponin-conjugated polyplexes have an enormously promising potential for delivering gene therapy *in vivo* using systemic administration.

In sum, the disclosed herein polyplexes, polyplexing agents, compositions, polymeric scaffolds, and methods address the need of solving a longstanding problem of cytoplasmic and nucleic delivery of gene therapeutics, in particular *in vivo* and by minimizing the treatment risks through circumventing viral vector-mediated gene therapy.

The disclosed herein polyplexes, polyplexing agents, compositions, polymeric scaffolds, and methods address this need by exhibiting the highly desired property of enhancing delivery of nucleic acids specifically into target cells, and thus have the potential of increasing the efficacy of targeted gene therapeutic treatment in patients.

Furthermore, they are compatible with personalized gene therapies, e.g. by advanced CRISPR/Cas technology, and thanks to their greater simplicity as compared to viral approaches, they have the potential of being easily produced and thus of reducing the costs of gene therapy for making it available to a broad patient base.

The innovative concepts presented herein will be described with respect to particular embodiments that should be regarded as descriptive and not limiting beyond of what is described in the claims. These embodiments as described herein can operate in combination and cooperation, unless specified otherwise.

It is one of several objectives of embodiments of the present disclosure to provide a solution to the problem of insufficient delivery of nucleic acids into appropriate compartments of their target cells, and to the problem of the related therewith non-specificity encountered when administering nucleic acid-based therapeutics to human patients.

It is a further one of several objectives of the embodiments to provide a solution to the problem of insufficient safety characteristics of current nucleic-acid-based drugs, when administered to human patients in need thereof, in particular at side-effect inducing excessive doses.

It is yet a further one of several objectives of embodiments of the current invention to provide a solution to the problem of current nucleic acid-based therapies being less efficacious than desired, when administered to human patients in need thereof, due to not being sufficiently capable to reach and/or enter into to the diseased target cells with little to no off-target activity on non-diseased cells, when administered to human patients in need thereof.

At least one of the above objectives is achieved by providing any one of the polyplexes, polyplexing agents, polymeric scaffolds, kits, methods, and uses as described in continuation. In particularly advantageous instances, different particular embodiments are provided, comprising advantageous components such as the ones selected from preferred sub-types of endosomal-escape-enhancing saponins, different therapeutic nucleic acids, and advantageous ligands or combinations thereof for targeting said saponins and nucleic acids to cells of interest, as well as covalent linkers for connecting the ligands with the saponins and nucleic acids and configured for being cleavable under conditions present in human endosomes and or lysosomes.

These and other aspects of the disclosure are presented in detail in continuation.

## DEFINITIONS

As used herein, the term "polyplex" has its regular established in the art meaning and refers to a complex between a nucleic acid and cationic, likely polymeric, carrier having a sufficient degree of affinity to the nucleic acid and capable of binding to said nucleic acid primarily by electrostatic interactions. Typical examples of such cationic carriers include e.g. cationic polymers.

As used herein, a cationic carrier capable of forming a polyplex with a nucleic acid will further be termed as a "polymeric scaffold". Examples of such polymeric scaffolds include, but are not limited to compounds comprising or consisting of cationic polymers such as polyamidoamine dendrimer, further referred to as PAMAM, or polypeptides with a net cationic charge, for example comprising sufficient number of cationic amino acids such as lysine or e.g. arginine. An example of a polypeptide with a net cationic charge is a peptide comprising or consisting of a strip of lysines, which can be referred to as poly-lysine, PLL, poly-K, polyK, poly-Lys etc. Other examples of polymeric scaffolds include but are not limited to poly-D-lysine (PDL), poly(ethylenimine)(PEI), chitosan, 2-dimethyl(aminoethyl) methacrylate (pDMAEM), and any synthetic and/or modified forms thereof, such as any of these and other polymeric scaffolds conjugated with chemical modifications, including but not limited to polyethylene glycol (PEG), i.e. pegylated (also spelled as PEGylated).

As specifically used herein, the term "polyplexing agent" should be construed as referring to a polymeric scaffold conjugated with a saponin being a triterpenoid 12,13-dehydrooleanane-type saponin that in an unreacted state comprises an aldehyde (functional) group at position C-23 of the saponin's aglycone core structure, wherein the saponin is covalently bound with the polymeric scaffold by a linker adapted to cleave and release the saponin from the polymeric scaffold under conditions present in an endosome or a lysosome.

As used herein, typical polyplexes may comprise smaller nucleic acids (e.g. siRNA, microRNA, or generally understood oligonucleotides) but preferentially will comprise much larger nucleic acids (e.g., lager than 150 bp, preferably larger than 0.5 kb, more preferably larger than 1 kb, most preferably larger than 2 kb, e.g. 100 times larger than a typical 20-25 bp siRNA). While the sizes of the present polyplexes are not invariant, preferably they will fall within the range of 50-250 nm, more preferably within the range of 100-200 nm.

As used herein, the terms "nucleic acid" and "polynucleotide" are synonymous to one another and are to be construed as encompassing any polymeric molecule made of units, wherein a unit comprises at least a nucleobase (or simply "base" e.g. being a canonical nucleobase like adenine (A), cytosine (C), guanine (G), thymine (T), or uracil (U), or any known non-canonical, modified, or synthetic nucleobase like 5-methylcytosine, 5-hydroxymethylcytosine, xanthine, hypoxanthine, 7-methylguanine; 5,6-dihydrouracil etc.) or a functional equivalent thereof, which renders said polymeric molecule capable of engaging in hydrogen bond-based nucleobase pairing (such as Watson-Crick base pairing) under appropriate hybridisation conditions with naturally-occurring nucleic acids such as deoxyribonucleic acid (DNA) or ribonucleic acid (RNA), which naturally-occurring nucleic acids are to be understood being polymeric molecules made of units being nucleotides, whereby each nucleotide consists of a pentose sugar, a phosphate group and one of the nucleobases.

Hence, from a chemistry perspective, the term nucleic acid under the present definition can be construed as encompassing polymeric molecules that chemically are DNA or RNA, as well as polymeric molecules that are nucleic acid analogues, also known as xeno nucleic acids (XNA) or artificial nucleic acids, which are polymeric molecules wherein one or more (or all) of the units are modified nucleotides or are functional equivalents of nucleotides. Nucleic acid analogues are well known in the art and due to various properties, such as improved specificity and/or affinity, higher binding strength to their target and/or increased stability *in vivo,* they are extensively used in research and medicine. Typical examples of nucleic acid analogues include but are not limited to locked nucleic acid (LNA) (that is also known as bridged nucleic acid (BNA)), phosphorodiamidate morpholino oligomer (PMO also known as Morpholino), peptide nucleic acid (PNA), glycol nucleic acid (GNA), threose nucleic acid (TNA), hexitol nucleic acid (HNA), 2'-deoxy-2'-fluoroarabinonucleic acid (FANA or FNA), 2'-deoxy-2'-fluororibonucleic acid (2'-F RNA or FRNA); altritol nucleic acids (ANA), cyclohexene nucleic acids (CeNA) etc.

In line with the above, in some instance, the nucleic acid of the present disclosure may be modified. For example, the nucleic acid may be modified on its backbone. Examples of modifications that can be performed on the backbone of a nucleic acid include, but are not limited to, phosphorothioate (PS), boranophosphate, phosphonoacatate (PACE), morpholine, peptide nucleic acid backbone modification (PNA), and amid-linked bases. The nucleic acid may also be modified on the sugar moiety and/or on the base moiety. Examples of modifications that can be performed on the sugar and/or the base moieties include, but are not limited to, locked nucleic acid (LNA), phosphoramidate (NP), 2'F-RNA, 2'-0 methoxyethyl (2'MOE), 2'O-methyl (2'OMe), 2'-O-fluoro (2'-F) 5-bromouracil, 5-iodouracil, 5-methylcytosine, ethylene bridged nucleic acids (ENA), diaminopurine, 2-thiouracil, 4-thiouracil, pseudouracil, hypoxantine, 2-aminoadenine, 6-methyl or other alkyl derivates of adenine and guanine, 2-propyl and other derivative of adenine and guanine, 6-azo-uracil, 8-halo, 8-amino, 8-thiol, 8-hydroxyk and other 8-substituted adenines and guanines, constrained ethyl sugar moiety (cET), ribofuranosyl, 2'-0,4'-C-methylene and 2'-0,4'-C-ethylene bicyclic nucleotide analogues, acyclic nucleotides (UNA and PNA), and dihydrouridine modification. Other modifications that may be performed on nucleic acids are, but are not limited to, modifications that include deoxyribonucleotide bases incorporated in a ribonucleotide sequence. The incorporations may be limited to the overhang structure in the canonical siRNA architecture or may be distributed in the sequence. Modifications to RNA molecules include, but are not limited to blunt-ended siRNA, 25-27mer siRNA, single strand siRNA, short hairpin siRNA, dumbbell siRNA, asymetric siRNA, short interspaced siRNA, hybrid between siRNA and antisense oligonucleotides (ASO). Other analogue nucleic acids may be contemplated include those with nonribose backbones. In addition, mixtures of naturally occurring nucleic acids, analogues, and both may be made. Nucleic acids include but are not limited to DNA, RNA and hybrids where the nucleic acid contains any combination of deoxyribo- and ribo-nucleotides, and any combination of bases, including uracil, adenine, thymine, cytosine, guanine, inosine, xathanine hypoxathanine, isocytosine, isoguanine, 5-methylcytidine, pseudouridine etc. Modified 5' cap structures such as 3'-O-Me-m7G(5')ppp(5')G (antireverse cap analogue), may also be used for increased translation of mRNA. Nucleic acids include DNA in any form, RNA in any form, including triplex, duplex or single-stranded, antisense, siRNA, ribozymes, deoxyribozymes, polynucleotides, oligonucleotides, chimeras, and derivatives thereof.

In accordance with the cannon, length of a nucleic acid is expressed herein as the number of units from which a single strand of a nucleic acid is build. Because each unit corresponds to exactly one nucleobase capable of engaging in one base pairing event, the length is frequently expressed in so called "base pairs" or "bp" regardless of whether the

nucleic acid in question is a single stranded (ss) or double stranded (ds) nucleic acid. In naturally-occurring nucleic acids, the length expressed in bp usually corresponds to the length expressed in nucleotides (nt). For example, a single stranded nucleic acid made of 1000 nucleotides (or a double stranded nucleic acid made of two complementary strands each of which is made of 1000 nucleotides) can also be described as having a length of 1000 base pairs or 1000 bp, which length can also be expressed as 1000 nt or as 1 kilobase that is abbreviated to 1 kb. 2 kilobases or 2 kb are equal to the length of 2000 base pair which equates 2000 nucleotides of a single stranded RNA or DNA. To avoid confusion however, in view of the fact the nucleic acids as defined herein may comprise or consist of units not only chemically being nucleotides but also being functional equivalents thereof, the length of nucleic acids will preferentially be expressed herein in "bp" or "kb" rather than in the equally common in the art denotation "nt".

In certain embodiments, the nucleic acid can be an oligonucleotide (or simply an oligo) defined as nucleic acid being no longer than 150 bp, i.e. in accordance with the above provided definition, being any polymeric molecule comprising not more than 150 units (i.e. comprising maximally 150 units), wherein each unit comprises a nucleobase or a functional equivalent thereof, which renders said oligonucleotide capable of engaging in hydrogen bond-based nucleobase pairing under appropriate hybridisation conditions with DNA or RNA. Within the ambit of said definition, it will immediately be appreciated that the disclosed herein oligonucleotides can comprise or consist of units not only being nucleotides but also being synthetic equivalents thereof. In other words, from a chemistry perspective, as used herein the term oligonucleotide will be construed as possibly comprising or consisting of RNA, DNA, or a nucleic acid analogue such as but not limited to LNA (BNA), PMO (Morpholino), PNA, GNA, TNA, HNA, FANA, FRNA, ANA, CeNA and/or the like. However, in preferred embodiments, the nucleic acid will be larger than 150 bp, preferably larger than 500 bp (i.e. 0.5 kb or 0.5 kbp), more preferably larger than 1000 bp (i.e. 1 kb or 1 kbp).

The term "saponin" has its regular established meaning and refers herein to a group of amphipathic glycosides which comprise one or more hydrophilic saccharide chains combined with a lipophilic aglycone core which is a sapogenin. The saponin may be naturally occurring or synthetic (i.e. non-naturally occurring). The term "saponin" includes naturally-occurring saponins, functional derivatives of naturally-occurring saponins as well as saponins synthesized *de novo* through chemical and/or biotechnological synthesis routes. Saponin according to the polyplex of the invention has a triterpene backbone, which is a pentacyclic C30 terpene skeleton, also referred to as sapogenin or aglycone. Within the polyplex of the invention saponin is not considered an effector molecule nor an effector moiety in the polyplex according to the invention. In the context of the polyplex of the invention, the term saponin refers to those saponins which exert an endosomal/lysosomal escape enhancing activity, when present in the endosome and/or lysosome of a mammalian cell such as a human cell, towards an effector moiety, here the nucleic acid, comprised by the polyplex of the invention and present in said endosome/lysosome together with the saponin.

As used herein, the term "saponin derivative" (also known as "modified saponin") shall be understood as referring to a compound corresponding to a naturally-occurring saponin that has been derivatised on the aldehyde group of the aglycone core; or on the carboxyl group of a saccharide chain or on an acetoxy group of a saccharide chain. Typically, the saponin derivative does not have a natural counterpart, i.e. the saponin derivative is not produced naturally by e.g. plants or trees. The term "saponin derivative" includes derivatives obtained by derivatisation of naturally-occurring saponins as well as derivatives synthesized *de novo* through chemical and/or biotechnological synthesis routes resulting in a compound corresponding to a naturally-occurring saponin which has been derivatised by one or more chemical modifications. A saponin derivative in the context of the invention should be understood as a saponin functional derivative. "Functional" in the context of the saponin derivative is understood as the capacity or activity of the saponin or the saponin derivative to enhance the endosomal escape of an effector molecule which is contacted with a cell together with the saponin or the saponin derivative.

The term "aglycone core structure" shall be understood as referring to the aglycone core of a saponin without the carbohydrate antennae or saccharide chains (glycans) bound thereto. For example, quillaic acid is the aglycone core structure for SO1861, QS-7 and QS21. Typically, the glycans of a saponin are mono-saccharides or oligo-saccharides, such as linear or branched glycans.

The term "saccharide chain" has its regular scientific meaning and refers to any of a glycan, a carbohydrate antenna, a single saccharide moiety (mono-saccharide) or a chain comprising multiple saccharide moieties (oligosaccharide, polysaccharide). The saccharide chain can consist of only saccharide moieties or may also comprise further moieties such as any one of 4E-Methoxycinnamic acid, 4Z-Methoxycinnamic acid, and 5-O-[5-O-Ara/Api-3,5-dihydroxy-6-methyl-octanoyl]-3,5-dihydroxy-6-methyl-octanoic acid), such as for example present in QS-21.

The term "Api/Xyl-" or "Api- or Xyl-" in the context of the name of a saccharide chain has its regular scientific meaning and here refers to the saccharide chain either comprising an apiose (Api) moiety, or comprising a xylose (Xyl) moiety.

As used herein, the term "an endocytic receptor" is to be understood as any one of cell surface molecules, likely receptors or transporters that are accessible to their specific ligands from the external side or surface of cell membrane (also known as plasmalemma) and capable of undergoing internalisation via endocytic pathway e.g., upon external stimulation, such as ligand binding to the receptor. In some embodiments, an endocytic receptor can be internalized by clathrin-mediated endocytosis, but can also be internalized by a clathrin-independent pathway, such as, for example, phagocytosis, macropinocytosis, caveolae- and raft-mediated uptake or constitutive clathrin-independent endocytosis. In some embodiments, the endocytic receptor comprises an intracellular domain, a transmembrane domain, and/or (e.g., and) an extracellular domain, which may optionally further comprise a ligand-binding domain. In some embodiments, the endocytic receptor becomes internalized by the cell after ligand binding. In some embodiments, a ligand may be a specific-celltargeting agent, for example a natural ligand (or a synthetic fragment thereof) or an antibody or a binding fragment thereof.

As used herein, the term "ligand" is to be understood as any molecule that binds to or can be recognised by a receptor. Typical ligand can be an antibody, a binding fragment of an antibody, simply fragment of an antibody. Alternatively, a typical ligand can also be a protein, a peptide, a polysugar, a glycoprotein, or a fragment of any one thereof which fragment is capable of being recognised by an endocytic receptor.

As used herein, the term "antibody or a binding fragment thereof" refers to a polypeptide that includes at least one immunoglobulin variable domain or at least one antigenic determinant, e.g., paratope that specifically binds to an antigen. In some embodiments, an antibody is a full-length antibody. In some embodiments, an antibody is a chimeric antibody. In some embodiments, an antibody is a humanized antibody. However, in some embodiments, an antibody is a Fab fragment, a F(ab') fragment, a F(ab')2 fragment, a Fv fragment or a scFv fragment. In some embodiments, an antibody is a nanobody derived from a camelid antibody or a nanobody derived from shark antibody. In some embodiments, an antibody is a diabody. In some embodiments, an antibody comprises a framework having a human germline sequence. In another embodiment, an antibody comprises a heavy chain constant domain selected from the group consisting of IgG, IgG1, IgG2, IgG2A, IgG2B, IgG2C, IgG3, IgG4, IgAl, IgA2, IgD, IgM, and IgE constant domains. In some embodiments, an antibody comprises a heavy (H) chain variable region (abbreviated herein as VH), and/or (e.g., and) a light (L) chain variable region (abbreviated herein as VL). In some embodiments, an antibody comprises a constant domain, e.g., an Fc region. An immunoglobulin constant domain refers to a heavy or light chain constant domain. Human IgG heavy chain and light chain constant domain amino acid sequences and their functional variations are known. With respect to the heavy chain, in some embodiments, the heavy chain of an antibody described herein can be an alpha (a), delta (D), epsilon (e), gamma (g) or mu (m) heavy chain. In some embodiments, the heavy chain of an antibody described herein can comprise a human alpha (a), delta (D), epsilon (e), gamma (g) or mu (m) heavy chain. In a particular embodiment, an antibody described herein comprises a human gamma 1 CH1, CH2, and/or (e.g., and) CH3 domain. In some embodiments, the amino acid sequence of the VH domain comprises the amino acid sequence of a human gamma (g) heavy chain constant region, such as any known in the art. Non-limiting examples of human constant region sequences have been described in the art, e.g., see U.S. Pat. No. 5,693,780 and Kabat E A et al, (1991) supra. In some embodiments, the VH domain comprises an amino acid sequence that is at least 70%, 75%, 80%, 85%, 90%, 95%, 98%, or at least 99% identical to any of the variable chain constant regions provided herein. In some embodiments, an antibody is modified, e.g., modified via glycosylation, phosphorylation, sumoylation, and/or (e.g., and) methylation. In some embodiments, an antibody is a glycosylated antibody, which is conjugated to one or more sugar or carbohydrate molecules. In some embodiments, the one or more sugar or carbohydrate molecule are conjugated to the antibody via N-glycosylation, O-glycosylation, C-glycosylation, glypiation (GPI anchor attachment), and/or (e.g., and) phosphoglycosylation. In some embodiments, the one or more sugar or carbohydrate molecule are monosaccharides, disaccharides, oligosaccharides, or glycans. In some embodiments, the one or more sugar or carbohydrate molecule is a branched oligosaccharide or a branched glycan. In some embodiments, the one or more sugar or carbohydrate molecule includes a mannose unit, a glucose unit, an N-acetylglucosamine unit, an N-acetylgalactosamine unit, a galactose unit, a fucose unit, or a phospholipid unit. In some embodiments, an antibody is a construct that comprises a polypeptide comprising one or more antigen binding fragments of the disclosure linked to a linker polypeptide or an immunoglobulin constant domain. Linker polypeptides comprise two or more amino acid residues joined by peptide bonds and are used to link one or more antigen binding portions. Examples of linker polypeptides have been reported (see e.g., Holliger, P, et al. (1993) Proc. Natl. Acad. Sci. USA 90:6444-6448; Poljak, R. J., et al. (1994) Structure 2:1121-1123). Still further, an antibody may be part of a larger immunoadhesion molecule, formed by covalent or noncovalent association of the antibody or antibody portion with one or more other proteins or peptides. Examples of such immunoadhesion molecules include use of the streptavidin core region to make a tetrameric scFv molecule (Kipriyanov, S. M., et al. (1995) Human Antibodies and Hybridomas 6:93-101) and use of a cysteine residue, a marker peptide and a C-terminal polyhistidine tag to make bivalent and biotinylated scFv molecules (Kipriyanov, S. M., et al. (1994) Mol. Immunol. 31:1047-1058).

The term "single domain antibody", or "sdAb", in short, or 'nanobody', has its regular scientific meaning and here refers to an antibody fragment consisting of a single monomeric variable antibody domain, unless referred to as more than one monomeric variable antibody domain such as for example in the context of a bivalent sdAb, which comprises two of such monomeric variable antibody domains in tandem. A bivalent nanobody is a molecule comprising two single domain antibodies targeting epitopes on molecules present at the extracellular side of a cell, such as epitopes on the extracellular domain of a cell surface molecule that is present on the cell. Preferably the cell-surface molecule is a cell-surface receptor. A bivalent nanobody is also named a bivalent single domain antibody. Preferably the two different

single domain antibodies are directly covalently bound or covalently bound through an intermediate molecule that is covalently bound to the two different single domain antibodies. Preferably the intermediate molecule of the bivalent nanobody has a molecular weight of less than 10,000 Dalton, more preferably less than 5000 Dalton, even more preferably less than 2000 Dalton, most preferably less than 1500 Dalton.

As used herein, the term "covalently linked" refers to a characteristic of two or more molecules being linked together via at least one covalent bond, i.e. directly, or via a chain of covalent bonds, i.e. via a linker comprising at least one or more atoms.

As used herein, the term "conjugate" is to be construed as a combination of two or more different molecules that have been and are covalently bound. For example, different molecules forming a conjugate as disclosed herein may include a polymeric scaffold and one or more saponin molecules and/or one or more ligands that bind to an endocytic receptor, preferably wherein the ligand is an antibody or a binding fragment thereof, such as an IgG, a monoclonal antibody (mAb), a VHH domain or another nanobody type, a bivalent nanobody molecule comprising two single domain antibodies, etc. In some aspects, the disclosed herein conjugates may be made by covalently linking different molecules via one or more intermediate molecules such as linkers, such as for example via linking to a central or further linker. In a conjugate, not all of the two or more, such as three, different molecules need to be directly covalently bound to each other. Different molecules in the conjugate may also be covalently bound by being both covalently bound to the same intermediate molecule such as a linker or each by being covalently bound to an intermediate molecule such as a further linker or a central linker wherein these two intermediate molecules such as two (different) linkers, are covalently bound to each other. According to this definition even more intermediate molecules, such as linkers, may be present between the two different molecules in the conjugate as long as there is a chain of covalently bound atoms in between.

As used herein, the terms "administering" or "administration" means to provide a substance to a subject in a manner that is physiologically and/or (e.g., and) pharmacologically useful (e.g., to treat a condition in the subject).

As used herein, the term "approximately" or "about," as applied to one or more values of interest, refers to a value that is similar to a stated reference value. In certain embodiments, the term "approximately" or "about" refers to a range of values that fall within 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

The terms first, second, third and the like in the description and in the claims, are used for distinguishing between for example similar elements, compositions, constituents in a composition, or separate method steps, and not necessarily for describing a sequential or chronological order. The terms are interchangeable under appropriate circumstances and the embodiments of the invention can operate in other sequences than described or illustrated herein, unless specified otherwise.

The embodiments as described herein can operate in combination and cooperation, unless specified otherwise. Furthermore, the various embodiments, although referred to as "preferred" or "e.g." or "for example" or "in particular" and the like are to be construed as exemplary manners in which the disclosed herein concepts may be implemented rather than as limiting.

The term "comprising", used in the claims, should not be interpreted as being restricted to for example the elements or the method steps or the constituents of a compositions listed thereafter; it does not exclude other elements or method steps or constituents in a certain composition. It needs to be interpreted as specifying the presence of the stated features, integers, (method) steps or components as referred to, but does not preclude the presence or addition of one or more other features, integers, steps or components, or groups thereof. Thus, the scope of the expression "a method comprising steps A and B" should not be limited to a method consisting only of steps A and B, rather with respect to the present invention, the only enumerated steps of the method are A and B, and further the claim should be interpreted as including equivalents of those method steps. Thus, the scope of the expression "a composition comprising components A and B" should not be limited to a composition consisting only of components A and B, rather with respect to the present invention, the only enumerated components of the composition are A and B, and further the claim should be interpreted as including equivalents of those components.

In addition, reference to an element or a component by the indefinite article "a" or "an" does not exclude the possibility that more than one of the element or component are present, unless the context clearly requires that there is one and only one of the elements or components. The indefinite article "a" or "an" thus usually means "at least one".

The term "Saponinum album" has its normal meaning and here refers to a mixture of saponins produced by Merck KGaA (Darmstadt, Germany) containing saponins from *Gypsophila paniculata* and *Gypsophila arostii,* containing SA1657 and mainly SA1641.

The term "Quillaja saponin" has its normal meaning and here refers to the saponin fraction of Quillaja saponaria and thus the source for all other QS saponins, mainly containing QS-18 and QS-21.

"QS-21" or "QS21" has its regular scientific meaning and here refers to a mixture of QS-21 A-apio (-63%), QS-21 A-xylo (-32%), QS-21 B-apio (-3.3%), and QS-21 B-xylo (-1.7%).

Similarly, "QS-21A" has its regular scientific meaning and here refers to a mixture of QS-21 A-apio (-65%) and

QS-21 A-xylo (-35%).

Similarly, "QS-21B" has its regular scientific meaning and here refers to a mixture of QS-21 B-apio (-65%) and QS-21 B-xylo (-35%).

The term "Quil-A" refers to a commercially available semi-purified extract from Quillaja saponaria and contains variable quantities of more than 50 distinct saponins, many of which incorporate the triterpene-trisaccharide substructure Gal-(1→2)-[Xyl-(1→3)]-GlcA- at the C-3beta-OH group found in QS-7, QS-17, QS-18, and QS-21. The saponins found in Quil-A are listed in van Setten (1995), Table *2 [*Dirk C. van Setten, Gerrit van de Werken, Gijsbert Zomer and Gideon F. A. Kersten, Glycosyl Compositions and Structural Characteristics of the Potential Immuno-adjuvant Active Saponins in the Quillaja saponaria Molina Extract Quil A, RAPID COMMUNICATIONS IN MASS SPECTROMETRY, VOL. 9,660-666 (1995)]. Quil-A and also Quillaja saponin are fractions of saponins from Quillaja saponaria and both contain a large variety of different saponins with largely overlapping content. The two fractions differ in their specific composition as the two fractions are gained by different purification procedures.

The term "QS1861" and the term "QS1862" refer to QS-7 and QS-7 api. QS1861 has a molecular mass of 1861 Dalton, QS1862 has a molecular mass of 1862 Dalton. QS1862 is described in Fleck et *al.* (2019) in Table 1, row no. 28 *[*Juliane Deise Fleck, Andresa Heemann Betti, Francini Pereira da Silva, Eduardo Artur Troian, Cristina Olivaro, Fernando Ferreira and Simone Gasparin Verza, Saponins from Quillaja saponaria and Quillaja brasiliensis: Particular Chemical Characteristics and Biological Activities, Molecules 2019, 24, 171; doi:10.3390/molecules24010171]. The described structure is the api-variant QS1862 of QS-7. The molecular mass is 1862 Dalton as this mass is the formal mass including proton at the glucuronic acid. At neutral pH, the molecule is deprotonated. When measuring in mass spectrometry in negative ion mode, the measured mass is 1861 Dalton.

The terms "SO1861" and "SO1862" refer to the same saponin of *Saponaria officinalis,* though in deprotonated form or api form, respectively. The molecular mass is 1862 Dalton as this mass is the formal mass including a proton at the glucuronic acid. At neutral pH, the molecule is deprotonated. When measuring the mass using mass spectrometry in negative ion mode, the measured mass is 1861 Dalton.

## BRIEF DESCRIPTION OF FIGURES

**Figure 1:** (A) Reaction scheme for pH-cleavable derivative of SO1861, further termed "SO1861-EMCH"; (B) Skeletal structural formula of derivatized SO1861-EMCH; (C) Reaction scheme of PAMAM dendrimer with 2-iminothiolane resulting in generation of thiolated PAMAM dendrimer that can react with SO1861-EMCH leading to generation of a PAMAM scaffold covalently bound with SO1861 by means of an acid-sensitive linker that under acidic conditions is adapted to release free SO1861 from PAMAM;

**Figure 2:** Reaction scheme of PAMAM dendrimer with a PEG adapter ending with a click-chemistry reactive azide group that can be used for conjugation with a targeting ligand. The PEG adapter is shown using the example of NHS-$PEG_{12}$-$N_3$ adapter ending with the click-chemistry reactive azide but other designs of PEG adapters are also possible. (A) detailed schematic representation when the click adapter moiety is shown with a skeletal formula; (B) simplified schematic representation of the click adapter;

**Figure 3:** (A) Schematic 2-step reaction scheme for modifying PAMAM dendrimer by conjugation of click adapters (NHS-$PEG_{12}$-$N_3$) to create binding sites for e.g. targeting ligands (as shown in Figure 2), followed by conjugation with acid-cleavable SO1861-EMCH (using 2-iminothiolane *in situ* as shown in Figure 1C). Skeletal formulas of the thus-introduced binding sites attached to PAMAM are shown; (B) simplified schematic representation of the 2-step reaction scheme shown in A;

**Figure 4:** (A) Schematic representation of a possible strategy for preparing a targeted polyplex assembled with an effector gene (schematically represented by a circular double stranded nucleic acid, for example a plasmid) and a polyplexing agent based on a PAMAM scaffold that was conjugated with an endosomal escape enhancing (EEE) saponin (represented by SO1861) prior to mixing the scaffold with the plasmid. As a result of this sequence of preparation steps, the saponin has equal distribution throughout the entire polyplex. In the next step, the polyplexing agent is conjugated with a targeting ligand by means of a click-chemistry reaction of two compatible click adapters; (B) Schematic representation of a second possible strategy for preparing a targeted polyplex based on a PAMAM scaffold that was conjugated with an EEE saponin after mixing the scaffold with the plasmid. As a result, the saponin is only present on the surface of the polyplex. In the next step, the polyplexing agent is conjugated with a targeting ligand by means of a click-chemistry reaction as in A; (C) Schematic representation of the last step as shown in A and B, which involves conjugation of the polyplexing agent with a targeting ligand by means of a click-chemistry

reaction, wherein the targeting ligand is represented by transferrin and the click-chemical reaction is SPAAC; It should be noted that the conjugation with the ligand can either be performed before assembling of the polyplex or after mixing the effector gene with the non-targeted polymeric scaffold as shown in A-C;

**Figure 5:** (A) Schematic representation of a plasmid polyplexed with a PAMAM-based scaffold conjugated with an EEE saponin (represented by SO1861) obtained as shown in Figure 4A, wherein the saponin was conjugated to PAMAM before it was mixed with the plasmid. Consequently, the saponin is equally distributed throughout the entire polyplex, including even its interior; (B) schematic representation of a plasmid polyplexed with a PAMAM-based scaffold conjugated with an EEE saponin obtained as shown in Figure 4B, wherein the saponin was conjugated to PAMAM after it was mixed with the plasmid and, consequently, the saponin is only present on the polyplex' periphery;

**Figure 6:** (A) Schematic representation of one possible optimized peptide scaffold design with consecutive poly-lysine region at the N-terminal of the peptide and a C-terminal click-chemistry reactive azide group for conjugation of a targeting ligand (in this example introduced as part of a C-terminal azidolysine). C-terminal amidation of the peptide scaffold increases stability and prolongs its shelf life. The poly-lysine region and the C-terminal click-chemistry reactive azide group are separated by a spacer sequence primarily comprising glycine residues and (in present design) also a single cysteine that can be used for further various conjugation reactions; (B) Reaction scheme for addition of SO1861-EMCH to cysteine residue of the peptide scaffold shown in A, indicating the reaction conditions;

**Figure 7:** (A) $^1$H NMR spectra ($CD_3OD$, 500 MHz) of commercial PAMAM G5 and (B) G5-(PEG-$N_3$)$_5$; (C) 13C NMR spectra ($CD_3OD$, 125 MHz) of G5-(PEG-$N_3$)$_6$; (D) IR spectra (KBr) of PAMAM-G5 (a), PAMAM-G5-(PEG-$N_3$)$_{3.2}$ (b), PAMAM-G5-(PEG-$N_3$)$_{6.2}$ (c), and PAMAM-G5-(PEG-$N_3$)$_{12.4}$ (d); (E) DLS size distribution of PAMAM-G5-(PEG-$N_3$) with PEGylation degrees 3.2, 6.2, and 12.4; (F) Complexation efficiency of different PAMAM scaffolds as a function of N/P ratio used for polyplex formation. No free DNA was detected in gel retardation assay for all PAMAM scaffolds studied. (a) PAMAM-G5-(PEG$N_3$)$_{3.2}$, (b) PAMAM-G5-(PEG$N_3$)$_{6.2}$, (c) PAMAM-G5-(PEG$N_3$)$_{12.4}$, (d) PAMAM-G5-(PEG-$N_3$)$_{3.2}$-(SO1861)$_{0.5}$, (e) PAMAM-G5-(PEG$N_3$)$_{6.2}$-(SO1861)$_{0.5}$, (f) PAMAM-G5-(PEG$N_3$)$_{12.4}$-(SO1861)$_{0.5}$, (g) PAMAM-G5-(PEG-$N_3$)$_{3.2}$-(SO1861)$_5$, (h) PAMAM-G5-(PEG$N_3$)$_{6.2}$-(SO1861)$_5$, and (i) PAMAM-G5-(PEG$N_3$)$_{12.4}$-(SO1861)$_5$; (G) $^1$H NMR spectrum ($CD_3OD$, 500 MHz) of PAMAM G5-(PEG-$N_3$)$_5$-(SO1861)$_{0.38}$; (H) IR spectra (KBr) of (a) PAMAM-G5, (b) G5-(PEG-$N_3$)$_{12.4}$ and (c) G5-(PEG-$N_3$)$_{12.4}$-(SO1861)$_5$; (I) DLS size distribution of PAMAM G5-(PEG-$N_3$)$_x$-(SO1861)$_5$ with PEGylation degrees 3.2, 6.2, and 12.4 and a constant SO1861 loading of 5;

**Figure 8:** (A) DLS size distributions of polyplexes from pegylated and equipped PAMAM scaffolds and pEGFP-N3 (N/P 8) in 10 mM HEPES pH 7.1 before (continuous line) and after (dashed line) addition of salt solution (final NaCl concentration 150 mM): (a) PAMAM-G5-(PEG-$N_3$)$_{3.2}$, (b) PAMAM-G5-(PEG-$N_3$)$_{5.2}$, (c) PAMAM-G5-(PEG-$N_3$)$_{12.4}$, (d) PAMAM-G5-(PEG-$N_3$)$_{3.2}$-(SO1861)$_{0.5}$, (e) PAMAM-G5-(PEG-$N_3$)$_{6.2}$-(SO1861)$_{0.5}$, (f) PAMAM-G5-(PEG-$N_3$)$_{12.4}$-(SO1861)$_{0.5}$ (g) PAMAM-G5-(PEG-$N_3$)$_{3.2}$-(SO1861)$_5$, (h) PAMAM-G5-(PEG-$N_3$)$_{6.2}$-(SO1861)$_5$, (i) PAMAM-G5-(PEG-$N_3$)$_{12.4}$-(SO1861)$_5$; (B) Zeta-potential of polyplexes from equipped PAMAM scaffolds and pEGFP-N3 (N/P 8) in 10 mM HEPES pH 7.1: (a) PAMAM-G5-(PEG-$N_3$)$_{3.2}$-(SO1861)$_{0.5}$, (b) PAMAM-G5-(PEG-$N_3$)$_{6.2}$-(SO1861)$_{0.5}$, (c) PAMAM-G5-(PEG-$N_3$)$_{12.4}$-(SO1861)$_{0.5}$, (d) PAMAM-G5-(PEG-$N_3$)$_{3.2}$-(SO1861)$_5$, (e) PAMAM-G5-(PEG-$N_3$)$_{6.2}$-(S01861)$_5$, (f) PAMAM-G5-(PEG-$N_3$)$_{12.4}$-(SO1861)$_5$; (C) TEM images of polyplexes from equipped PAMAM scaffolds and pEGFP-N3 (N/P 8) in 10 mM HEPES pH 7.1: (a) PAMAM-G5-(PEG-$N_3$)$_{3.2}$-(SO1861)$_{0.5}$, (b) PAMAM-G5-(PEG-$N_3$)$_{6.2}$-(SO1861)$_{0.5}$, (c) PAMAM-G5-(PEG-$N_3$)$_{12.4}$-(SO1861)$_{0.5}$, (d) PAMAM-G5-(PEG-$N_3$)$_{3.2}$-(SO1861)$_5$, (e) PAMAM-G5-(PEG-$N_3$)$_{6.2}$-(S01861)$_5$, (f) PAMAM-G5-(PEG-$N_3$)$_{12.4}$-(SO1861)$_5$;

**Figure 9:** of SDS-PAGE of polyplexes conjugated with labelled ligand compared to fluorescence of the unreacted free protein at known concentrations visualized in Cy3 (A) or Cy5 (B+C) channel: (A) PAMAM polyplexes of (1) PAMAM-G5-(PEG$N_3$)$_{3.2}$, (2) PAMAM-G5-(PEG-$N_3$)$_{3.2}$-(SO1861)$_{0.5}$, and (3) PAMAM-G5-(PEG-$N_3$)$_{3.2}$-(SO1861)$_5$ conjugated with Cy3-transferrin-DBCO; (B) PAMAM polyplexes of (1) PAMAM-G5-(PEG$N_3$)$_{3.2}$, (2) PAMAM-G5-(PEG$N_3$)$_{6.2}$, (3) PAMAM-G5-(PEG$N_3$)$_{12.4}$, and (4) PAMAM G5 conjugated with Cy5-cetuximab-DBCO: (C) (1) PAMAM-G5-(PEG-$N_3$)$_{3.2}$-(SO1861)$_{0.5}$, (2) PAMAM-G5-(PEG$N_3$)$_{6.2}$-(S01861)$_{0.5}$, (3) PAMAM-G5-(PEG$N_3$)$_{12.4}$-(SO1861)$_{0.5}$, (4) PAMAM-G5-(PEG-$N_3$)$_{3.2}$-(SO1861)$_5$, (5) PAMAM-G5-(PEG$N_3$)$_{6.2}$-(S01861)$_5$, and (6) PAMAM-G5-(PEG$N_3$)$_{12.4}$-(SO1861)$_5$ conjugated with Cy5-cetuximab-DBCO.

**Figure 10:** Complexation efficiency of different peptide scaffolds as a function of N/P ratio used for polyplex formation. ≤ 2% free DNA was detected for all studied peptide scaffolds at N/P 10. K16 = $K_{16}$, K16C = $K_{16}G_4$CGGYK($N_3$), $K_{16}$Ceq0.25 = $K_{16}G_4$CGGYK($N_3$)-S01861$_{0.25}$, $K_{16}$Ceq0.5 = $K_{16}G_4$CGGYK($N_3$)-S01861$_{0.5}$, K16CPEG = $K_{16}G_4$CGGY-PEG8-K($N_3$), $K_{16}$CPEGeq0.25 = $K_{16}G_4$CGGY-PEG8-K($N_3$)-SO1861$_{0.25}$, $K_{16}$CPEGeq0.5 =

$K_{16}G_4$CGGY-PEG8-K($N_3$)-SO1861$_{0.5}$.;

**Figure 11:** Transfection of HEK cells with 40 pM eGFP plasmid polyplexed with either (A) low pegylated PAMAM G5 (PEG-$N_3$)$_{3.5}$ alone; (B) the same scaffold but provided with 4 μM SO1861-EMCH as a transfection agent; (C) the scaffold of A in addition equipped with 0.5 equivalents of surface SO1861 (conjugation after polyplexation); or with (D) the scaffold of A in addition equipped with 5 equivalents SO1861 (equally distributed per PAMAM core;, conjugation before polyplexation). The bright spots mark cells expressing GFP indicating successful transfection. The results show that the highest transfection augmentation at no noticeable side-effect or toxicity was achieved with polyplexes made with PAMAM scaffolds conjugated with 0.5 equivalents of surface SO1861 (pane C). Although transfection rate was also high in cells where 4 μM SO1861-EMCH was used, these cells suffered from severe growth inhibition effects and/or cell death (pane B); (E) Percent of HEK293FT cell confluency at 72 hrs post treatments A-D + in Lipofectamine transfected control; (F) Percent of GFP expression at 72 hrs post treatments A-D + in Lipofectamine transfected control. The percentages of confluency were calculated by eSIGHT sofware, based on masks that cover all cells or only the GFP cells;

**Figure 12:** Live imaging of transfection dynamics in HEK293FT cells using pEGFP polyplexed as described in Figure 10 with or without SO1861. The readings were taken at intervals of 6 hrs for a total of 72 hrs; (A) bright-field (% confluency) readings providing an indication of toxicity of the polyplex compositions to cells; (B) eGFP expression (% green confluency) readings providing an indication for the general transfection efficiency to cells; (C) Normalized eGFP expression (% green confluency/% confluency), readings providing an indication for the transfection efficiency relative to the amount of cell viability;

**Figure 13:** Transfection of A2058 cells with eGFP plasmid polyplexed with either (A) low pegylated PAMAM G5 (PEG-$N_3$)$_{3.5}$ alone; (B) the same scaffold equipped with 0.5 equivalents of surface SO1861; or with (C) 0.5 equivalents of equally distributed SO1861; or with low pegylated PAMAM G5 (PEG-$N_3$)$_{3.5}$ with either (D) 40 nM SO1861-EMCH; or (E) 40 nM SO1861; or (F) lipofectamine transfection of eGFP plasmid. The bright spots mark cells expressing GFP indicating successful transfection. The results show that transfection augmentation was the highest with polyplexes made with scaffolds conjugated either with 0.5 equivalents of equally distributed SO1861 or with 0.5 equivalents of surface SO1861, both of which achieved similar effects (C and B, respectively);

**Figure 14:** Transfection of A431 cells with nanoplasmid encoding for saporin (npSaporin) polyplexed with PAMAM G5 (PEG-$N_3$)$_{6.2}$ or PAMAM G5 (PEG-$N_3$)$_{12.4}$ or PAMAM G5 (PEG-$N_3$)$_{6.2}$(SO1861)$_{0.5}$ or PAMAM G5 (PEG-$N_3$)$_{12.4}$(SO1861)$_{0.5}$;

**Figure 15:** Transfection of a nanoplasmid encoding for saporin (npSaporin) polyplexed with K16C peptide scaffold into A431 cells alone, with free SO1861-EMCH, or wherein the K16C peptide scaffold was covalently conjugated by means of an acid-sensitive linker with 0.25 equivalents of SO1861 (K16C(SO1861)$_{0.25}$);

**Figure 16:** Transfection efficiency in different cell lines of polyplexes made with pEGFP-N3 and different peptide scaffolds (as indicated), + or - SO1861-EMCH denotes supplementation or lack thereof, respectively, with free SO1861 -EMCH for enhancing transfection efficiency. The cell lines are as follows: (A) N2A; (B) HEK293FT; (C) MDA-MB 468; and (D) Hepa1-6;

**Figure 17:** Transfection of murine primary hepatocytes with a plasmid encoding human Factor IX (hFIX), which was polyplexed with PAMAM G5 (PEG-$N_3$)$_{4.0}$ scaffold, and optionally conjugated with SO1861 (external) and/or GalNAc as a liver cell targeting ligand;

**Figure 18:** (A) Experimental setup of the assay for assessing stability of polyplexes in blood. (B) Gel electrophoresis of samples treated according to the scheme shown in A: (1.) PCR positive control, (2.) negative control (DNA-free sample), (3.) peptide-based polyplex ($K_{16}C$ = $K_{16}G_4$CGGYK($N_3$)) without heparin, (4.) dendriplex (PAMAM G5) without heparin, (5.) $K_{16}C$ polyplex after release through heparin addition, (6.) PAMAM G5-based polyplexes after heparin addition. White numbers on the DNA ladder indicate base pairs;

**Figure 19:** (A) Procedure of the hemolysis assay and possible results. The term "Nanomaterials" refers to the different PAMAM- or peptide-based scaffolds; (B) hemolytic activity of scaffolds and polyplexes at different concentrations. For better comparison the concentrations are referred to the scaffold molecules PAMAM and K16C and not to the polyplexes; (C) morphology of RBCs treated with either of (1.) PAMAM, (2.) $K_{16}C$ = $K_{16}G_4$CGGYK($N_3$), (3.) polyplexes made with PAMAM, (4.) polyplexes made with $K_{16}C$ (as indicated) at a final test concentration of 80

μg/mL.

## DETAILED DESCRIPTION

In a first general aspect, disclosed herein are saponin-containing polyplexes that have the improved ability of enhancing endosomal escape and thus also effective delivery into the cell of nucleic acids comprised within said polyplexes. The improved endosomal escape is achieved thanks to the use of an endosomal escape enhancing (EEE) saponin and a specific mode of its covalent conjugation to the polymeric scaffold that forms the polyplex in the presence of a nucleic acid. This mode of conjugation is configured such to ensure the release of the EEE saponin from the polymeric scaffold in response to conditions present in the endosome/lysosome (e.g. in response to acidic pH or can be cleaved of by a specifically endosomal/lysosomal enzyme etc.) and further such that the released saponin is restored to its EEE form and can exert its destabilising activity towards the endosomal/lysosomal membrane, thus stimulating the escape of the nucleic acid into the cytosol. The advantage of such polyplex-saponin conjugate is that by virtue of the saponin being covalently linked to the polymeric scaffold that assembles the polyplex, the EEE saponin is stably linked to and can co-migrate with the polyplexed nucleic acid like a portable transfection agent that only becomes active to enhance the nucleic acid's cytosolic delivery when present in the endosome. Consequently, in contrast to other transfection agents, the saponin forms an integral part of the polyplexing agent that holds the nucleic acid and it only activates its membrane destabilising properties when inside the endosome, thus ensuring not only efficient cotransportation with the gene therapeutic but also specific release of the gene therapeutic into the cell's cytosol via specifically enhanced endosomal escape.

The disclosed herein endosomal escape enhancing polyplexes can advantageously be targeted to cells of interest by conjugation with selected ligands recognised by endocytic receptors present on the target cell's surface. The ligands can e.g. be naturally existing ligands or parts thereof or modified versions of any of those. For example, they can be proteins, peptides, or polysugars, but advantageously they can also be antibodies or binding fragments thereof. Such ligands can also be conjugated to the polymeric scaffold and thus they may become a part of the polyplexing agent that polyplexes with the nucleic acid to be delivered to the target cell. The disclosed herein ligand-targeted polyplex-saponin conjugates have the advantage of being able to specifically deliver and efficiently release therapeutic nucleic acid in the target cells of interest. Consequently, they have the promising potential of providing a novel targeted one-component non-viral gene therapy system with enhanced endosomal escape properties for improved nucleic acid delivery, which is currently needed to reduce the doses of gene therapeutics and thus improve their safety profiles and clinical application.

The innovative concepts presented herein will be described with respect to particular embodiments or aspects of the disclosure, that should be regarded as descriptive and not limiting beyond of what is described in the claims. The particular aspects as described herein can operate in combination and cooperation, unless specified otherwise. While the invention has been described with reference to these embodiments, it is contemplated that alternatives, modifications, permutations and equivalents thereof will become apparent to one having ordinary skill in the art upon reading the specification and upon study of the drawings and graphs. The invention is not limited in any way to the illustrated embodiments. Changes can be made without departing from the scope which is defined by the appended claims.

It is one of several objectives of embodiments of present disclosure to provide a solution to the problem of inefficient delivery encountered when administering nucleic acid-based therapeutics to human patients and in need of such therapeutics. It is a further one of several objectives of the embodiments to provide a solution to the problem of current nucleic acid-based therapies being less efficacious than desired, when administered to human patients in need thereof, due to not being sufficiently capable to reach and/or enter into the target cell, e.g. a diseased cell, with little to no off-target activity on non-targeted cells, e.g. non-diseased cells, when administered to human patients in need thereof.

Without wishing to be bound by any theory, the disclosed herein novel pharmaceutical compositions were conceived based on the observation that a specific group of triterpenoid 12,13-dehydrooleanane-type saponins appears to exhibit potent endosomal-escape enhancing properties for nucleic acid-based therapeutics, for example when the nucleic acid-based therapeutic is provided as a polyplex according to the invention.

Endocytic pathways are complex and not fully understood. Nowadays, it is hypothesized that they involve stable compartments connected by vesicular traffic. A compartment is a complex, multifunctional membrane organelle that is specialized for a particular set of essential functions for the cell. Vesicles are considered to be transient organelles, simpler in composition, and are defined as membrane-enclosed containers that form *de novo* by budding from a pre-existing compartment. In contrast to compartments, vesicles can undergo maturation, which is a physiologically irreversible series of biochemical changes. Early endosomes and late endosomes represent stable compartments in the endocytic pathway while primary endocytic vesicles, phagosomes, multivesicular bodies (also called endosome carrier vesicles), secretory granules, and even lysosomes represent vesicles.

An endocytic vesicle, which arises at the plasma membrane, most prominently from clathrin-coated pits, first fuses with the early endosome, which is a major sorting compartment of approximately pH 6.5. A large part of the

internalized cargo and membranes are recycled back to the plasma membrane through recycling vesicles (recycling pathway). Components that should be degraded are transported to the acidic late endosome (pH lower than 6) via multivesicular bodies. Lysosomes are vesicles that can store mature lysosomal enzymes and deliver them to a late endosomal compartment when needed. The resulting organelle is called the hybrid organelle or endolysosome. Lysosomes bud off the hybrid organelle in a process referred to as lysosome reformation. Late endosomes, lysosomes, and hybrid organelles are extremely dynamic organelles, and distinction between them is often difficult. Degradation of the endocytosed molecules occurs inside the endolysosomes.

*Endosomal escape* is the active or passive release of a substance from the inner lumen of any kind of compartment or vesicle from the endocytic pathway, preferably from clathrin-mediated endocytosis, or recycling pathway, into the cytosol. Endosomal escape thus includes but is not limited to release from endosomes, endolysosomes or lysosomes, including their intermediate and hybrid organelles. After entering the cytosol, said substance might move to other cell units such as the nucleus.

As will be demonstrated by the presented herein data, the inclusion of a triterpenoid 12,13-dehydrooleanane-type saponin comprising an aldehyde group at position C-23 of the saponin's aglycone core structure, in the polyplexes and polyplexing agents of the disclosure appeared to stimulate efficient cytosolic delivery of the therapeutic nucleic acids once present in the endosomes of the cells to which these saponins were delivered and released from the polymeric scaffold in the form comprising the aldehyde group at position C-23 thanks to appropriately designed covalent linkers.

In line with these promising observations and findings, in a first general aspect, the invention provides a polyplex comprising

at least one nucleic acid,

and a polyplexing agent, wherein the polyplexing agent comprises

a polymeric scaffold, and

a saponin being a triterpenoid 12,13-dehydrooleanane-type saponin that in an unreacted state comprises an aldehyde group at position C-23 of the saponin's aglycone core structure,

wherein the saponin is covalently bound with the polymeric scaffold by a linker adapted to cleave and release the saponin from the polymeric scaffold under conditions present in an endosome or a lysosome.

The above-referred to group of saponins possess triterpene 12,13-dehydrooleanane-type backbone core structure (also referred to as sapogenin or aglycone), usually shown as a pentacyclic C30 terpene skeleton, and further comprise an aldehyde group at position C-23 in their native (i.e. nonmodified or non-reacted) state. Examples of such known saponins are shown in Table 1 and in Scheme Q.

**TABLE 1. Saponins displaying (late) endosomal/lysosomal escape enhancing activity with an aglycone core of the 12,13-dehydrooleanane type[4)]**

| Saponin Name | Aglycone core with an aldehyde group at the C-23 position | Carbohydrate substituent at the C-3beta-OH group | Carbohydrate substituent at the C-28-OH group |
|---|---|---|---|
| NP-017777 | Gypsogenin | Gal-(1→2)-[Xyl-(1→3)]-GlcA- | Xyl-(1→4)-Rha-(1→2)-[R-(→4)]-Fuc- (R = 4E-Methoxycinnamic acid) |
| NP-017778 | Gypsogenin | Gal-(1→2)-[Xyl-(1→3)]-GlcA- | Xyl-(1→4)-Rha-(1→2)-[R-(→4)]-Fuc- (R = 4Z-Methoxycinnamic acid) |
| NP-017774 | Gypsogenin | Gal-(1→2)-[Xyl-(1→3)]-GlcA- | Xyl-(1→4)-[Gal-(1→3)]-Rha-(1→2)-4-OAc-Fuc- |
| NP-018110[c], NP-017772[d] | Gypsogenin | Gal-(1→2)-[Xyl-(1→3)]-GlcA- | Xyl-(1→4)-[Glc-(1→3)]-Rha-(1→2)-3,4-di-OAc-Fuc- |
| NP-018109 | Gypsogenin | Gal-(1→2)-[Xyl-(1→3)]-GlcA- | Xyl-(1→4)-[Glc-(1→3)]-Rha-(1→2)-[R-(→4)]-3-OAc-Fuc- (R = 4E-Methoxycinnamic acid) |

(continued)

| TABLE 1. Saponins displaying (late) endosomal/lysosomal escape enhancing activity with an aglycone core of the 12,13-dehydrooleanane type[4)] | | | |
|---|---|---|---|
| **Saponin Name** | **Aglycone core with an aldehyde group at the C-23 position** | **Carbohydrate substituent at the C-3beta-OH group** | **Carbohydrate substituent at the C-28-OH group** |
| NP-017888 | Gypsogenin | Gal-(1→2)-[Xyl-(1→3)]-GlcA- | Glc-(1→3)-Xyl-(1→4)-[Glc-(1→3)]-Rha-(1→2)-4-OAc-Fuc- |
| NP-017889 | Gypsogenin | Gal-(1→2)-[Xyl-(1→3)]-GlcA- | Glc-(1→3)-Xyl-(1→4)-Rha-(1→2)-4-OAc-Fuc- |
| NP-018108 | Gypsogenin | Gal-(1→2)-[Xyl-(1→3)]-GlcA- | Ara/Xyl-(1→3)-Ara/Xyl-(1→4)-Rha/Fuc-(1→2)-[4-OAc-Rha/Fuc-(1→4)]-Rha/Fuc- |
| SA1641[a], AE X55[b] | Gypsogenin | Gal-(1→2)-[Xyl-(1→3)]-GlcA- | Xyl-(1→3)-Xyl-(1→4)-Rha-(1→2)-[Qui-(1→4)]-Fuc- |
| S01658 | Gypsogenin | Gal-(1→2)-[Xyl-(1→3)]-GlcA- | Glc-(1→3)-[Xyl-(1→3)-Xyl-(1→4)]-Rha-(1→2)-Fuc- |
| gypsoside A[6)] | Gypsogenin | Gal-(1→4)-Glc (1→4)-[Ara-(1→3)]-GlcA- | Xyl-(1→3)-Fuc-(1→4)-[Xyl-(1→3)-Xyl-(1→3)]-Rha- |
| phytolaccagenin | Gypsogenin | absent | absent |
| | | | |
| Gypsophila saponin 1 (Gyp1) | Quillaic acid | Gal-(1→2)-[Xyl-(1→3)]-GlcA | Glc-(1→3)-[Xyl-(1→4)]-Rha-(1→2)-Fuc- |
| NP-017674 | Quillaic acid | Gal-(1→2)-[Xyl-(1→3)]-GlcA- | Api-(1→3)-Xyl-(1→4)-[Glc-(1→3)]-Rha-(1→2)-Fuc- |
| NP-017810 | Quillaic acid | Gal-(1→2)-[Xyl-(1→3)]-GlcA- | Xyl-(1→4)-[Gal-(1→3)]-Rha-(1→2)-Fuc |
| AG1 | Quillaic acid | Gal-(1→2)-[Xyl-(1→3)]-GlcA- | Xyl-(1→4)-[Glc-(1→3)]-Rha-(1→2)-Fuc- |
| NP-003881 | Quillaic acid | Gal-(1→2)-[Xyl-(1→3)]-GlcA- | Ara/Xyl-(1→4)-Rha/Fuc-(1→4)-[Glc/Gal-(1→2)]-Fuc- |
| NP-017676 | Quillaic acid | Gal-(1→2)-[Xyl-(1→3)]-GlcA- | Api-(1→3)-Xyl-(1→4)-[Glc-(1→3)]-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| NP-017677 | Quillaic acid | Gal-(1→2)-[Xyl-(1→3)]-GlcA- | Api-(1→3)-Xyl-(1→4)-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| NP-017706 | Quillaic acid | Gal-(1→2)-[Xyl-(1→3)]-GlcA- | Api-(1→3)-Xyl-(1→4)-Rha-(1→2)-[Rha-(1→3)]-4-OAc-Fuc- |

(continued)

| TABLE 1. Saponins displaying (late) endosomal/lysosomal escape enhancing activity with an aglycone core of the 12,13-dehydrooleanane type[4] | | | |
|---|---|---|---|
| **Saponin Name** | **Aglycone core with an aldehyde group at the C-23 position** | **Carbohydrate substituent at the C-3beta-OH group** | **Carbohydrate substituent at the C-28-OH group** |
| NP-017705 | Quillaic acid | Gal-(1→2)-[Xyl-(1→3)]-GlcA- | Api-(1→3)-Xyl-(1→4)-[Glc-(1→3)]-Rha-(1→2)-[Rha-(1→3)]-4-OAc-Fuc- |
| NP-017773 | Quillaic acid | Gal-(1→2)-[Xyl-(1→3)]-GlcA- | 6-OAc-Glc-(1→3)-Xyl-(1→4)-Rha-(1→2)-[3-OAc-Rha-(1→3)]-Fuc- |
| NP-017775 | Quillaic acid | Gal-(1→2)-[Xyl-(1→3)]-GlcA- | Glc-(1→3)-Xyl-(1→4)-Rha-(1→2)-[3-OAc-Rha-(1→3)]-Fuc |
| SA1657 | Quillaic acid | Gal-(1→2)-[Xyl-(1→3)]-GlcA- | Xyl-(1→3)-Xyl-(1→4)-Rha-(1→2)-[Qui-(1→4)]-Fuc- |
| AG2 | Quillaic acid | Gal-(1→2)-[Xyl-(1→3)]-GlcA- | Glc-(1→3)-[Xyl-(1→4)]-Rha-(1→2)-[Qui-(1→4)]-Fuc- |
| GE1741 | Quillaic acid | Gal-(1→2)-[Xyl-(1→3)]-GlcA- | Xyl-(1→3)-Xyl-(1→4)-Rha-(1→2)-[3,4-di-OAc-Qui-(1→4)]-Fuc- |
| SO1542 | Quillaic acid | Gal-(1→2)-[Xyl-(1→3)]-GlcA- | Glc-(1→3)-[Xyl-(1→4)]-Rha-(1→2)-Fuc |
| S01584 | Quillaic acid | Gal-(1→2)-[Xyl-(1→3)]-GlcA- | 6-OAc-Glc-(1→3)-[Xyl-(1→4)]-Rha-(1→2)-Fuc- |
| SO1674 | Quillaic acid | Gal-(1→2)-[Xyl-(1→3)]-GlcA- | Glc-(1→3)-[Xyl-(1→3)-Xyl-(1→4)]-Rha-(1→2)-Fuc- |
| SO1700[3] | Quillaic acid | Gal-(1→2)-[Xyl-(1→3)]-GlcA- | Xyl-(1→4)-Rha-(1→2)-[Xyl-(1→3)-4-OAc-Qui-(1→4)]-Fuc- |
| Saponarioside B[1] | Quillaic acid | Gal-(1→2)-[Xyl-(1→3)]-GlcA- | Xyl-(1→3)-Xyl-(1→4)-Rha-(1→2)-[4-OAc-Qui-(1→4)]-Fuc- |
| SO1730[3] | Quillaic acid | Gal-(1→2)-[Xyl-(1→3)]-GlcA- | Glc-(1→3)-Xyl-(1→4)-Rha-(1→2)-[-4-OAc-Qui-(1→4)]-Fuc- |
| SO1772[3] | Quillaic acid | Gal-(1→2)-[Xyl-(1→3)]-GlcA- | 6-OAc-Glc-(1→3)-[Xyl-(1→4)]-Rha-(1→2)-[4-OAc-Qui-(1→4)]-Fuc-- |
| SO1832[1] (protonated S01831) = Saponarioside A | Quillaic acid | Gal-(1→2)-[Xyl-(1→3)]-GlcA- | Xyl-(1→3)-Xyl-(1→4)-Rha-(1→2)-[Xyl-(1→3)-4-OAc-Qui-(1→4)]-Fuc- |
| S01861 (deprotonated S01862) | Quillaic acid | Gal-(1→2)-[Xyl-(1→3)]-GlcA- | Glc-(1→3)-Xyl-(1→4)-Rha-(1→2)-[Xyl-(1→3)-4-OAc-Qui-(1→4)]-Fuc- |
| S01862 (protonated SO1861), also referred to as *Sapofectosid*[5] | Quillaic acid | Gal-(1→2)-[Xyl-(1→3)]-GlcA- | Glc-(1→3)-Xyl-(1→4)-Rha-(1→2)-[Xyl-(1→3)-4-OAc-Qui-(1→4)]-Fuc- |

(continued)

| TABLE 1. Saponins displaying (late) endosomal/lysosomal escape enhancing activity with an aglycone core of the 12,13-dehydrooleanane type[4)] | | | |
|---|---|---|---|
| **Saponin Name** | **Aglycone core with an aldehyde group at the C-23 position** | **Carbohydrate substituent at the C-3beta-OH group** | **Carbohydrate substituent at the C-28-OH group** |
| SO1904[3)] | Quillaic acid | Gal-(1→2)-[Xyl-(1→3)]-GlcA- | 6-OAc-Glc-(1→3)-[Xyl-(1→4)]-Rha-(1→2)-[Xyl-(1→3)-4-OAc-Qui-(1→4)]-Fuc- |
| QS-7 (also referred to as QS1861) | Quillaic acid | Gal-(1→2)-[Xyl-(1→3)]-GlcA- | Api/Xyl-(1→3)-Xyl-(1→4)-[Glc-(1→3)]-Rha-(1→2)-[Rha-(1→3)]-4OAc-Fuc- |
| QS-7 api (also referred to as QS1862) | Quillaic acid | Gal-(1→2)-[Xyl-(1→3)]-GlcA- | Api-(1→3)-Xyl-(1→4)-[Glc-(1→3)]-Rha-(1→2)-[Rha-(1→3)]-4OAc-Fuc- |
| QS-17 | Quillaic acid | Gal-(1→2)-[Xyl-(1→3)]-GlcA- | Api/Xyl-(1→3)-Xyl-(1→4)-[Glc-(1→3)]-Rha-(1→2)-[R-(→4)]-Fuc-(R = 5-O-[5-O-Rha-(1→2)-Ara/Api-3,5-dihydroxy-6-methyl-octanoyl]-3,5-dihydroxy-6-methyl-octanoic acid) |
| QS-18 | Quillaic acid | Gal-(1→2)-[Xyl-(1→3)]-GlcA- | Api/Xyl-(1→3)-Xyl-(1→4)-[Glc-(1→3)]-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| QS-21 A-apio | Quillaic acid | Gal-(1→2)-[Xyl-(1→3)]-GlcA- | Api-(1→3)-Xyl-(1→4)-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| QS-21 A-xylo | Quillaic acid | Gal-(1→2)-[Xyl-(1→3)]-GlcA- | Xyl-(1→3)-Xyl-(1→4)-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| QS-21 B-apio | Quillaic acid | Gal-(1→2)-[Xyl-(1→3)]-GlcA- | Api-(1→3)-Xyl-(1→4)-Rha-(1→2)-[R-(→3)]-Fuc-(R = 5-O-[5-O-Ara/Api-3,5-dihydroxy-6-methyl-octanoyl]-3,5-dihydroxy-6-methyl-octanoic acid) |
| QS-21 B-xylo | Quillaic acid | Gal-(1→2)-[Xyl-(1→3)]-GlcA- | Xyl-(1→3)-Xyl-(1→4)-Rha-(1→2)-[R-(→3)]-Fuc-(R = 5-O-[5-O-Ara/Api-3,5-dihydroxy-6-methyl-octanoyl]-3,5-dihydroxy-6-methyl-octanoic acid) |

(continued)

| **TABLE 1. Saponins displaying (late) endosomal/lysosomal escape enhancing activity with an aglycone core of the 12,13-dehydrooleanane type[4)]** | | | |
|---|---|---|---|
| **Saponin Name** | **Aglycone core with an aldehyde group at the C-23 position** | **Carbohydrate substituent at the C-3beta-OH group** | **Carbohydrate substituent at the C-28-OH group** |
| QS-21 | Quillaic acid | Gal-(1→2)-[Xyl-(1→3)]-GlcA- | Combination of the carbohydrate chains depicted for QS-21 A-apio, A-xylo, B-apio, B-xylo, for this position at the aglycone (see also the structure depicted as (Scheme Q)) |
| Agrostemmoside E (AG1856, AG2.8)[2)] | Quillaic acid | Gal-(1→2)-[Xyl-(1→3)]-GlcA- | [4,6-di-OAc-Glc-(1→3)]-[Xyl-(1→4)]-Rha-(1→2)-[3,4-di-OAc-Qui-(1→4)]-Fuc- |

a, b: Different names refer to different isolates of the same structure

c, d: Different names refer to different isolates of the same structure

1) Jia et al., Major Triterpenoid Saponins from Saponaria officinalis, J. Nat. Prod. 1998, 61, 11, 1368-1373, Publication Date: September 19, 1998, https://doi.org/10.1021/np980167u

2) The structure of Agrostemmoside E (also referred to as AG1856 or AG2.8) is given in Fig. 4 of J. Clochard et al, A new acetylated triterpene saponin from Agrostemma githago L. modulates gene delivery efficiently and shows a high cellular tolerance, International Journal of Pharmaceutics, Volume 589, 15 November 2020, 119822.

3) Structures of SO1700, SO1730, SO1772, SO1904 are given in Moniuszko-Szajwaj et al., Highly Polar Triterpenoid Saponins from the Roots of Saponaria officinalis L., Helv. Chim. Acta, V99, pp. 347 - 354, 2016 (doi.org/10.1002/hlca.201500224).

4) See for example:

- thesis by Dr Stefan Böttger (2013): Untersuchungen zur synergistischen Zytotoxizität zwischen Saponinen und Ribosomen inaktivierenden Proteinen Typ I, and
- Sama et al., Structure-Activity Relationship of Transfection-Modulating Saponins - A Pursuit for the Optimal Gene Trafficker, Planta Med. Volume 85, pp. 513-518, 2019 (doi:10.1055/a-0863-4795) and
- Fuchs et al., Glycosylated Triterpenoids as Endosomal Escape Enhancers in Targeted Tumor Therapies, Biomedicine, Volume 5, issue 14, 2017 (doi: 10.3390/biomedicines5020014).

5) Sama et al., Sapofectosid - Ensuring non-toxic and effective DNA and RNA delivery, International Journal of Pharmaceutics, Volume 534, Issues 1-2, 20 December 2017, Pages 195-205 (dx.doi.org/10.1016/j.ijpharm.2017.10.016) & Moniuszko-Szajwaj et al., Highly Polar Triterpenoid Saponins from the Roots of Saponaria officinalis L., Helv. Chim. Acta, V99, pp. 347 - 354, 2016 (doi.org/10.1002/hlca.201500224).

6) See for example: doi:10.1016/s0040-4039(01)90658-6, Tetrahedron Letters No. 8, pp. 477-482, 1963 and pubchem.ncbi.nlm.nih.gov/compound/Gipsoside

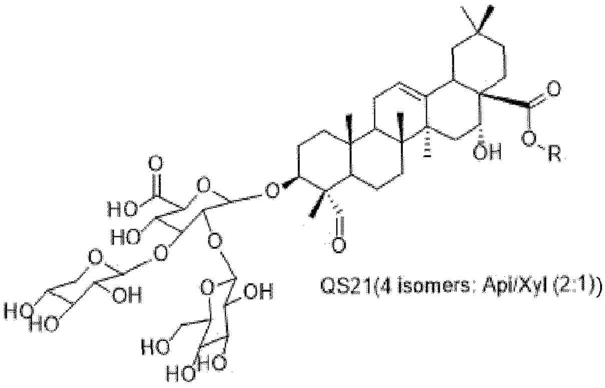

QS21(4 isomers: Api/Xyl (2:1))

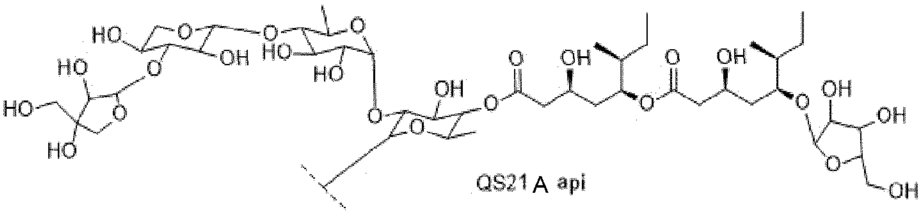

QS21 A api

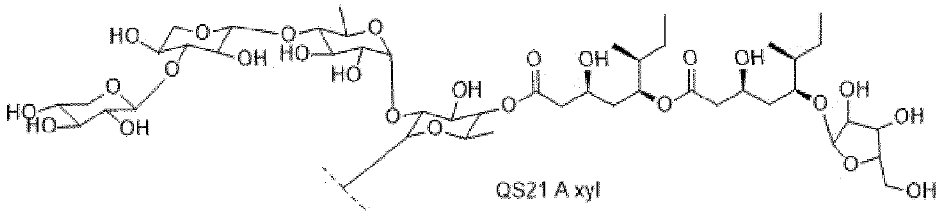

QS21 A xyl

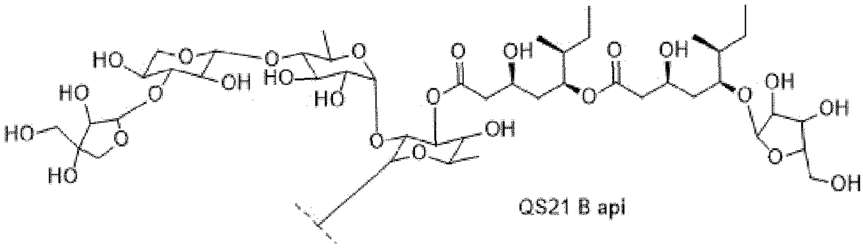

QS21 B api

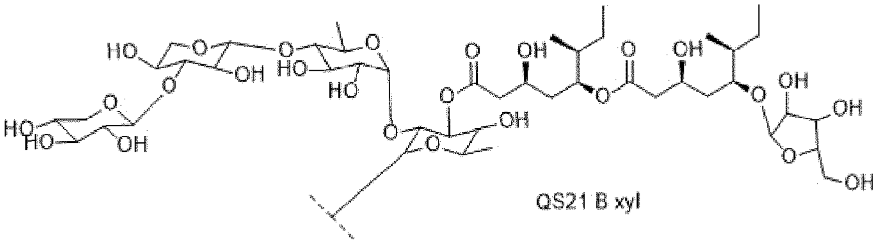

QS21 B xyl

**(Scheme Q)**

A notable feature of these saponins is the aldehyde group at position C-23 of the saponin's aglycone core structure. Without wishing to be bound by any theory, it was observed that presence of said aldehyde group in the aglycone core structure of the saponin (here, also referred to as 'aglycone') is particularly beneficial for the capacity of the saponin to stimulate and/or potentiate the endosomal escape of the therapeutic nucleic acids comprised by the polyplex of the invention or by the cell-surface molecule targeted polyplex of the invention.

Consequently, in possible advantageous embodiments, the disclosed herein polyplexes are prepared such that the aldehyde group at position C-23 of the saponin's aglycone core structure is present or restored under acidic or specific-enzymatic conditions present in mammalian endosomes and/or lysosomes.

Advantageously, the saponin can be covalently bound to the polymeric scaffold by an acid-sensitive linker through the position C-23 of the saponin's aglycone core structure and wherein the acid-sensitive linker is adapted to cleave and release the saponin from the polymeric scaffold (and thus also from the polyplex formed using said polymeric scaffold) such that the aldehyde group at position C-23 of the saponin's aglycone core structure is restored under acidic conditions present in mammalian endosomes and/or lysosomes.

Hence, in a preferred embodiment, a polyplex is provided, wherein the linker is an acid-sensitive linker or is a linker configured to be enzymatically cleaved by an enzyme present in the endosome or the lysosome, preferably wherein the linker is an acid-sensitive linker adapted to cleave and release the saponin from the polymeric scaffold under acidic conditions present in the endosome or the lysosome, i.e. in particular conditions present in mammalian endosomes and/or lysosomes, defined as conditions of pH < 6, preferably pH < 5.5, more preferably pH < 5; even more preferably pH < 4.5, most preferably pH < 4.

For the endosomal escape enhancing properties, it appears to be particularly beneficial that the aldehyde group at position C-23 of the saponin's aglycone core structure is restored to a free aldehyde (functional) group once inside of the endosome, and hence any chemical modifications that uncap or restore the free aldehyde functional group at the position at position C-23 of the saponin's aglycone core structure under acidic and/or enzymatic conditions present in endosomes and/or lysosomes of human cells can in principle be applied to the disclosed herein suitable saponins including those saponins shown in their native form in Table 1.

For example, endosomal-escape-enhancing properties of such saponins are still also very pronounced when the aldehyde group is e.g. substituted by a maleimide-comprising moiety attached at said position C-23 with a cleavable covalent bond that cleaves off under acidic conditions present in endosomes and/or lysosomes of human cells, whereby said aldehyde group at position C-23 of the saponin's aglycone core structure is restored upon said cleavage under acidic conditions present in endosomes and/or lysosomes of human cells.

In advantageous embodiments, such cleavable covalent bond can be selected from a hydrazone bond, or a imine bond.

For example, in further possible embodiments, the maleimide-comprising moiety can be a part of a molecule comprising or consisting of N-ε-maleimidocaproic acid hydrazide that is attached at position C-23 of the saponin's aglycone core structure upon forming a hydrazone bond (further referred to as EMCH).

Hence, in a possible embodiment, a polyplex is disclosed, wherein the aldehyde group at position C-23 of the saponin's aglycone core structure is either a free aldehyde group, or is an aldehyde group substituted by a maleimide-comprising moiety attached at said position C-23 with a cleavable covalent bond that cleaves off under acidic conditions present in endosomes and/or lysosomes of human cells, wherein said aldehyde group at position C-23 of the saponin's aglycone core structure is restored upon said cleavage under acidic conditions present in endosomes and/or lysosomes of human cells; preferably wherein the cleavable covalent bond is selected from a hydrazone bond, or an imine bond.

As explained above, in particularly attractive and relevant for safe systemic delivery embodiments, the disclosed herein polyplexes can be endowed, e.g. covalently conjugated with targeting ligands recognised by endocytic receptors present on target tissues or cells, for example diseased cells like cancer cells.

Consequently, in an advantageous embodiment, a polyplex or a polyplexing agent is provided further comprising a targeting ligand recognised by an endocytic receptor, which targeting ligand can, for example and preferably be linked, more preferably covalently bound directly or via a linker to the polyplexing agent, most preferably to the polymeric scaffold.

The endocytic receptor can for example be a receptor expressed on a diseased cell, like the highly-expressed Her2 or EGFR receptors on certain cancer cells. Alternatively, it can be for example a receptor abundant in a given tissue that under certain conditions could require or benefit from a gene therapy. A good example of such tissue-abundant endocytic receptor is the asialoglycoprotein receptor found on liver cells (hepatocytes). Liver cells form the target tissue for e.g. haemophilia gene therapy using clotting factor IX (FIX, synonymous with hFIX for human factor IX). The asialoglycoprotein receptor recognises and has high affinity to ligands comprising N-acetylgalactosamine (GalNAc).

For example, a possible embodiment of the presented herein targeted polyplexes could comprise any of the EEE-saponin equipped polyplexing agents as disclosed herein and a nucleic acid, for example a plasmid encoding for hFIX and a targeting ligand comprising GalNAc, which is a ligand recognised by an endocytic asialoglycoprotein receptor abundantly expressed on liver cells. However, many other examples of possible EEE-saponin-conjugated polyplexes in accordance with the present disclosure can be envisaged.

In another possible exemplary embodiment, a polyplex or a polyplexing agent can be provided, wherein the targeting ligand is an antibody or a binding fragment thereof, preferably is a monoclonal antibody such as Cetuximab or Panitumumab.

In line with the latter, in related possible embodiments, a polyplex or a polyplexing agent can be provided, wherein the endocytic receptor is selected from any of the following: epidermal growth factor receptor (EGFR) and receptor tyrosine-protein kinase erbB-2 (Her-2).

In another possible embodiment, the presented herein scaffold can further be conjugated with other type of molecules such as dyes, e.g. fluorescent dyes, or quenchers, which can be useful for performing e.g. subcellular localisation studies of polyplexes labelled with scaffolds and/or studies of how such localisation is affected by e.g. the size and nature of the polyplexed nucleic acid (e.g. plasmid of about 2kb versus a plasmid > 5kb vs PMO or siRNA or microRNA) and/or by the presence of different ligands. There exist many fluorescent dyes that can be attached to the scaffold according to any coupling strategy known in the art, so that the scaffold becomes labelled similarly as it is typically done with other molecular biology, for example fluorescently-labelled antibodies. Suitable fluorescent dyes can be dyes belonging to any of the known families like coumarin, rhodamine, cyanine, or xanthene like fluorescein family dyes, or modifications thereof like Alexa Flour dyes. Good results were obtained with cyanine dyes, which can be e.g. of non-sulfonated type including Cy3, Cy3.5, Cy5, Cy5.5, Cy7, and Cy7.5, as well as of sulfonated type such as sulfo-Cy3, sulfo-Cy5, and sulfo-Cy7 etc.

Using two different polymeric scaffold models, with varying degrees of their surface chemical modifications, it was observed that for all the conditions tested, when the amount of the saponin molecule equivalents was comprised between 0.05 and 4 per amount of molecules of the polymeric scaffold in the polyplexing agent was giving best transfection efficiency.

Hence, in an advantageous embodiment, a polyplex or a polyplexing agent is provided, wherein the amount of the saponin molecule equivalents per amount of molecules of the polymeric scaffold in the polyplexing agent is comprised between 0.05 and 4, preferably between 0.1 and 2, more preferably between 0.15 and 1, even more preferably between 0.2 and 0.75, and most preferably is either equal to or about 0.5.

The experimental data (shown below) confirmed that the disclosed herein polymeric scaffolds can be based on a dendrimer. Dendrimers can provide hyperbranched structures comprising layers of monomers radiating from a central core (Kodama et al., 2014), which makes them particularly suitable for being fine-tuned by chemical modifications. Many dendrimers are known in the art, with different degrees of branching with functional groups that are easy to modify. Different dendrimers are already used for gene delivery and gene transfection purposes (Dufes et al., 2005) and can be selected based on whether they exhibit hydrodynamic diameters in the suitable nanometer ranges. In the present disclosure, polyamidoamine dendrimer (PAMAM) of fifth generation (G5, or generation 5) was employed with promising success, in particular when modified by addition of PEG adapters (acting like pegylation), most advantageously, when the pegylation level was on a lower degree to retain sufficient positively charged amine groups for the reminder of the PAMAM to efficiently polyplex with the nucleic acid.

Hence, in a further preferred embodiment, a polyplex or a polyplexing agent is provided, wherein the polymeric scaffold is or comprises polyamidoamine dendrimer, further referred to as PAMAM. Preferably the PAMAM can comprise ethylenediamine core and/or is at least generation 3 PAMAM or higher, preferably is generation 5 PAMAM.

Possibly and preferably, the PAMAM may comprise at least one pegylated group, preferably comprising at least one PEG adapter. Preferably, the PEG adapter may comprise 5-15 PEG units, more preferably being 6-12 PEG units. Even more preferably, the pegylation degree of the PAMAM is comprised between on average 1.5-20 equivalents of PEG adapter per PAMAM core, preferably being on average 2-16 equivalents of PEG adapter per PAMAM core, even more preferably being on average 3-13 equivalents of PEG adapter per PAMAM core, most preferably being on average selected from any one of 3.2, 6.2, and 12.4 PEG adapter equivalents per PAMAM core.

In an advantageous specific embodiment, a polyplex or a polyplexing agent is provided wherein the amount of the saponin molecule equivalents per amount of molecules of the PAMAM in the polyplexing agent is comprised between 0.1 and 4, preferably between 0.15 and 2, more preferably between 0.2 and 1, even more preferably between 0.25 and 0.75, and most preferably is either equal to or about 0.5.

Alternatively, the polymeric scaffold is or comprises a polypeptide, for example comprising sufficient amount of lysines and/or arginines to provide the required for nucleic acid binding net positive charge. Peptides containing repeating lysine or arginine motifs are known to complex genetic material and have certain advantages. For example they are usually biocompatible and fully degradable but have limited binding sites. However, poly-lysine peptides are also known to lack the so called "proton sponge" ability (Vasiliu et al., 2017), which makes them inferior in gene delivery as compared to other polymeric scaffolds, e.g. based on PEI, due to not being able to destabilise endosomes. Advantageously, this weak interaction with membranes also results in lower toxicity of PLL compared to other cationic polymers. As in the present context the EEE-property is conferred by the conjugated saponin, polymeric scaffolds comprising or consisting of a polypeptide comprising lysines can be particularly advantageous.

In line with this, in an alternative possible embodiment, a polyplex or a polyplexing agent is provided, wherein

the polymeric scaffold is or comprises a polypeptide comprising between 5 to 25 lysines and preferably also at least one cysteine, more preferably being exactly one cysteine.

Preferably, such polypeptide comprises exactly one cysteine flanked by a string of 1-7 glycines from each side, preferably 1-5 glycines from each side, more preferably 2-4 glycines, even more preferably wherein the exactly one cysteine is flanked by 3-7 glycines from N-terminal side, preferably being 4 glycines; and by 1-4 glycines from C-terminal side, preferably being 2 glycines.

Optionally, the polypeptide may further comprise at least one C-terminal tyrosine.

As another option, the polypeptide may be terminated with a residue comprising an azide group, preferably being azidolysine, preferably 6-azido-L-lysine, which is beneficial for various ligation purposes using e.g. click-chemistry.

In a particularly advantageous embodiment, the polypeptide is represented by a general formula of, from N-terminus to C-terminus: 2-25 Lys; 3-7 Gly; 1 Cys; 2-4 Gly, and possibly 1 or more Tyr; preferably wherein the polypeptide is represented by an amino acid sequence given by SEQ ID NO. 1: KKKKKKKKKKKKKKKKGGGGCGGY or SEQ ID NO. 2: KKKKKKKKKKKKKKKKGGGGCGGYK* wherein K* is azidolysine, preferably 6-azido-L-lysine.

In a related particularly advantageous embodiment, a polyplex or a polyplexing agent is provided wherein the amount of the saponin molecule equivalents per amount of molecules of the polypeptide in the polyplexing agent is comprised between 0.1 and 1, preferably between 0.15 and 0.75, more preferably between 0.2 and 0.6, and most preferably is either equal to or about 0.5.

Naturally, other beneficial parameters can be envisaged when describing polyplexes, which include but are not limited to:

- N/P ratio, which enables calculation of the minimum ratio required to complex 100% of the nucleic acid and which will naturally differ depending on the polymeric scaffold used;
- charge/molar ratio, which is also scaffold-dependent and can be determined by e.g. gel electrophoresis shift;
- hydrodynamic diameter when in a solution, measurable via dynamic light scattering (DLS); and
- size and shape of the complexes in dried/frozen state that can be determined using high-resolution microscopy technique such as TEM, SEM, or AFM.

These and many other polyplex characteristics are variable and dependent on the final polyplex composition, which is known to the skilled person who is capable of determining them by any of the suitable techniques available in the art, if needed.

Most of the known saponins of the 12,13-dehydrooleanane-type that naturally comprise the aldehyde group in position C-23 in their native or unconjugated form are saponins for which the aglycone core structure is either quillaic acid or gypsogenin. An exemplary such saponin is depicted as SAPONIN A and illustrated by the following structure:

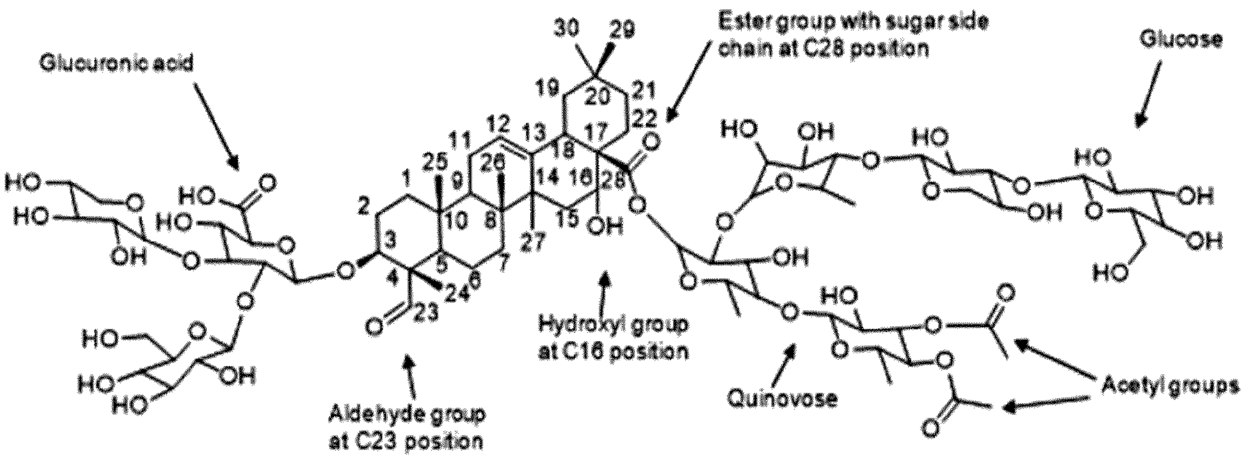


(SAPONIN A)

In line with this, it was observed that saponins comprising a quillaic acid aglycone or a gypsogenin aglycone core structure are particularly suitable for the purposes of the present disclosure.

Hence, in a further embodiment, a polyplex or a polyplexing agent is provided, wherein the saponin comprises

an aglycone core structure selected from quillaic acid and gypsogenin, more preferably wherein the aglycone core structure is quillaic acid.

Saponins can comprise one or more saccharide chains attached to the aglycone core structure. Preferred saponins of the polyplex or the polyplexing agent of the disclosure comprise a single chain (i.e. are monodesmosidic) or two chains (i.e. are bidesmosidic) attached to the triterpene 12,13-dehydrooleanane aglycone core structure that comprises an aldehyde (functional) group in position C-23.

It was postulated that the sugar chains also play a role in the endosomal-escape-enhancing properties.

In line with the above, in a further embodiment, a polyplex or a polyplexing agent is provided, wherein the saponin is at least a monodesmosidic saponin comprising at least a first saccharide chain comprising at least three sugar residues in a branched configuration, or is at least a bidesmosidic saponin further comprising a second saccharide chain comprising a glucuronic acid residue, preferably being a terminal glucuronic acid residue.

In a preferred embodiment, the first branched saccharide chain comprises a terminal fucose residue and/or a terminal rhamnose residue and preferably comprises at least four sugar residues, and/or wherein the second saccharide chain is Gal-(1→2)-[Xyl-(1→3)]-GlcA.

Advantageously, the saponin may comprise the first saccharide chain at position C-28 of the saponin's aglycone core structure, and/or the second saccharide chain at position C-3 of the saponin's aglycone core structure.

In a related advantageous embodiment, the first saccharide chain is a carbohydrate substituent at the C-28-OH group of the saponin's aglycone core structure and/or wherein the second saccharide chain is a carbohydrate substituent at the C-3beta-OH group of the saponin's aglycone core structure.

In a related specific embodiment, a polyplex or a polyplexing agent is provided, wherein the saponin comprises the first saccharide chain at position C-28 of the saponin's aglycone core structure and the second saccharide chain at position C-3 of the saponin's aglycone core structure; preferably wherein the first saccharide chain is a carbohydrate substituent at the C-28-OH group of the saponin's aglycone core structure and/or wherein the second saccharide chain is a carbohydrate substituent at the C-3beta-OH group of the saponin's aglycone core structure.

Particularly preferred saponins include SO1861 and/or GE1741, which are purified from plant roots of either *Saponaria officinalis L.* or *Gypsophila elegans M. BIEB,* respectively.

In an embodiment, a polyplex or a polyplexing agent is provided, wherein the saponin is any one or more of:

a) saponin selected from any one or more of list A:

- *Quillaja saponaria* saponin mixture, or a saponin isolated from *Quillaja saponaria,* for example Quil-A, QS-17-api, QS-17-xyl, QS-21, QS-21A, QS-21B, QS-7-xyl;
- *Saponinum album* saponin mixture, or a saponin isolated from *Saponinum album;*
- *Saponaria officinalis* saponin mixture, or a saponin isolated from *Saponaria officinalis;* and
- Quillaja bark saponin mixture, or a saponin isolated from Quillaja bark, for example Quil-A, QS-17-api, QS-17-xyl, QS-21, QS-21A, QS-21B, QS-7-xyl; or

b) a saponin comprising a gypsogenin aglycone core structure, selected from list B:
SA1641, gypsoside A, NP-017772, NP-017774, NP-017777, NP-017778, NP-018109, NP-017888, NP-017889, NP-018108, SO1658 and Phytolaccagenin; or

c) a saponin comprising a quillaic acid aglycone core structure, selected from list C:
AG1856, AG1, AG2, Agrostemmoside E, GE1741, Gypsophila saponin 1 (Gyp1), NP-017674, NP-017810, NP-003881, NP-017676, NP-017677, NP-017705, NP-017706, NP-017773, NP-017775, SA1657, Saponarioside B, SO1542, SO1584, SO1674, SO1700, SO1730, SO1772, SO1832, SO1861, SO1862, SO1904, QS-7, QS-7 api, QS-17, QS-18, QS-21 A-apio, QS-21 A-xylo, QS-21 B-apio and QS-21 B-xylo; or

preferably, wherein the saponin is any one or more of a saponin selected from list B or C, more preferably, a saponin selected from list C.

In a particular embodiment, a polyplex or a polyplexing agent is provided, wherein the saponin is any one or more of AG1856, GE1741, a saponin isolated from *Quillaja saponaria,* Quil-A, QS-17, QS-21, QS-7, SA1641, a saponin isolated from *Saponaria officinalis,* SO1542, SO1584, SO1658, SO1674, SO1700, SO1730, SO1772, Saponarioside B, SO1832, SO1861, SO1862 and SO1904; preferably wherein the saponin is any one or more of QS-21, SO1832, SO1861, SA1641 and GE1741; more preferably wherein the saponin is QS-21, SO1832 or SO1861; most preferably being SO1861.

In a more particular embodiment, a polyplex or a polyplexing agent is provided, wherein the saponin is a saponin isolated from *Saponaria officinalis,* preferably wherein the saponin is any one or more of SO1542, SO1584, SO1658, SO1674, SO1700, SO1730, SO1772, Saponarioside B, SO1832, SO1861, SO1862 and SO1904; more preferably wherein the saponin is any one or more of SO1832, SO1861 and SO1862;even more preferably wherein the saponin is SO1832 and SO1861; most preferably being SO1861.

In a further particular embodiment, a polyplex or a polyplexing agent is provided, wherein the saponin is or comprises at least one molecule of any of SO1861 or SO1861-EMCH.

In further embodiments, polyplexes or polyplexing agents as disclosed herein can be prepared with the suitable triterpenoid 12,13-dehydrooleanane-type saponins comprising an aldehyde group at position C-23 of the saponin's aglycone core structure, wherein one or more, preferably one of:

i. an aldehyde group in the aglycone core structure of the at least one saponin has been derivatised,
ii. a carboxyl group of a glucuronic acid moiety in a second saccharide chain of the at least one saponin has been derivatised when present in the at least one saponin, and
iii. at least one acetoxy (Me(CO)O-) group in a first saccharide chain of the at least one saponin has been derivatised if present.

In more particular embodiments, polyplexes or polyplexing agents can be provided wherein the at least one of the triterpenoid 12,13-dehydrooleanane-type saponins comprising an aldehyde group at position C-23 of the saponin's aglycone core structure further comprises:

i. an aglycone core structure comprising an aldehyde group which has been derivatised by:

- reduction to an alcohol;
- transformation into a hydrazone bond through reaction with N-ε-mateimidocaproic acid hydrazide (EMCH) wherein the maleimide group of the EMCH is optionally derivatised by formation of a thioether bond with mercaptoethanol;
- transformation into a hydrazone bond through reaction with N-[β-maleimidopropionic acid] hydrazide (BMPH) wherein the maleimide group of the BMPH is optionally derivatised by formation of a thioether bond with mercaptoethanol; or
- transformation into a hydrazone bond through reaction with N-[κ-maleimidoundecanoic acid] hydrazide (KMUH) wherein the maleimide group of the KMUH is optionally derivatised by formation of a thioether bond with mercaptoethanol; or

ii. a second saccharide chain comprising a carboxyl group, preferably a carboxyl group of a glucuronic acid moiety, which has been derivatised by transformation into an amide bond through reaction with 2-amino-2-methyl-1,3-propanediol (AMPD) or *N-(2*-aminoethyl)maleimide (AEM); or
iii. a first saccharide chain comprising an acetoxy group (Me(CO)O-) which has been derivatised by transformation into a hydroxyl group (HO-) by deacetylation; or
iv. any combination of two or three derivatisations i., ii. and/or iii., preferably any combination of two derivatisations of i., ii. and iii.

In a specific embodiment, provided are polyplexes or polyplexing agents, wherein the at least one saponin comprises a first saccharide chain and a second saccharide chain, wherein the first saccharide chain comprises more than one saccharide moiety and the second saccharide chain comprises more than one saccharide moiety, and wherein the aglycone core structure is quillaic acid or gypsogenin, more preferably is quillaic acid, wherein one, two or three, preferably one or two, of:

i. an aldehyde group in the aglycone core structure has been derivatised,
ii. a carboxyl group of a glucuronic acid moiety in the second saccharide chain has been derivatised, and
iii. at least one acetoxy (Me(CO)O-) group in the first saccharide chain has been derivatised.

An embodiment is the conjugate of the invention, wherein one, two or three, preferably one or two, more preferably one, of:

i. an aldehyde group in the aglycone core structure of the at least one saponin has been derivatised,
ii. a carboxyl group of a glucuronic acid moiety in a second saccharide chain of the at least one saponin has been derivatised when present in the at least one saponin, and

at least one acetoxy (Me(CO)O-) group in a first saccharide chain of the at least one saponin has been derivatised if present.

In another embodiment, a polyplex is provided wherein the nucleic acid is selected from a plasmid, a minicircle, cDNA, mRNA, siRNA, oligonucleotide, and any modified equivalent thereof comprising one or more nucleotide analogues, preferably wherein the nucleic acid is a plasmid, more preferably wherein the nucleic acid comprises at least 0.25 kbp, or at least 0.5 kbp; or at least 0.75 kbp, more preferably at least 1 kbp, or at least 1.5 kbp; or at least 2 kbp, even more preferably at least 2.5 kbp, or at least 3 kbp, or at least 4, kbp, most preferably at least 5 kbp. In preferred embodiments, the nucleic acid will comprise DNA, most preferably will be a plasmid or another form of a circular double-stranded DNA, such as a minicircle.

However, in an alternative embodiment, the nucleic acid can be an oligonucleotide (or a modified oligonucleotide, frequently referred to as an "oligo"), defined as polynucleotide having a maximal length of 150 bp, preferably of 120 bp, more preferably of 100 bp.

In preferred implementations, the nucleic acid is a circular, preferably double stranded DNA (dsDNA), such as a plasmid, mini-circle DNA, or other circular vector DNA. In some instances, the circular ds DNA such as plasmid or other circular vector DNA comprises a sequence that encodes for a therapeutic molecule such a therapeutic protein. For example, the sequence may encode for a component involved in gene editing such as a component of the clustered regularly interspaced short palindromic repeats (CRISPRs)/Cas gene system. Alternatively, the sequence may encode for a therapeutic RNA, which can be defined as an RNA molecule capable of exerting a therapeutic effect in a mammalian cell. Therapeutic RNAs include antisense RNAs, siRNAs, short hairpin RNAs, and enzymatic RNAs. The sequence may include nucleic acids intended to form triplex molecules, protein binding nucleic acids, ribozymes, deoxyribozymes, or small nucleotide molecules. In advantageous embodiments, the sequence can encode for a therapeutic peptide or a therapeutic protein, including cytotoxic proteins such as toxins (e.g. saporin or dianthin) or prodrugs; ribozymes; antisense or the complement thereof; or other such molecules.

In another embodiment, the nucleic acid may be used to effect gene therapy (also referred to as "genetic therapy"), for example by serving as a replacement or enhancement for a defective gene or to compensate for lack of a particular gene product, e.g. by encoding a therapeutic product. Alternatively, the nucleic acid may also inhibit expression of an endogenous gene or may encode all or a portion of a translation product, or may function by recombining with DNA already present in a cell, thereby replacing a defective portion of a gene. It further may also encode a portion of a protein and exert its effect by virtue of co-suppression of a gene product.

As already mentioned above, in possible embodiments, a polyplex or a polyplexing agent is provided, wherein the linker is subject to cleavage under conditions present in endosomes or lysosomes, preferably acidic or enzymatic conditions present in endosomes or lysosomes, possibly wherein the linker comprises a cleavable bond selected from:

- a bond subject to cleavage under acidic conditions such as a hydrazone bond, acetal bond, a dioxalan, and/or an imine bond,
- a bond susceptible to proteolysis, for example amide or peptide bond, preferably subject to proteolysis by Cathepsin B;
- red/ox-cleavable bond such as disulfide bond, or thiol-exchange reaction-susceptible bond such as thio-ether bond.

Advantageously, a polyplex or a polyplexing agent is provided, wherein the linker is an acid-sensitive linker that comprises a covalent bond selected from any one or more of: a hydrazone bond, an imine bond, an ester bond, a dioxalane bond, and an oxime bond, preferably wherein the acid-sensitive linker comprises a hydrazone bond.

Preferably, a polyplex or a polyplexing agent is provided, wherein the aldehyde group at position C-23 of the saponin's aglycone core structure has been engaged in forming the covalent bond with the linker.

It is advantageous that such a cleavable bond is not susceptible, or only to a minor extent, to cleavage when the polyplex-saponin conjugate (i.e. the polyplex of the invention) is present outside the endosome and/or lysosome of the cell, such as outside the cell or in the endocytosed vesicle after the polyplex specifically engaged with the endocytic machinery of the cell, either non-specifically (untargeted) or by binding of the ligand to its target endocytic receptor. For example, the cleavable bond is preferably less susceptible to cleavage when the polyplex is present in the circulation of a human subject and/or is present extracellularly in an organ of the human subject, compared to the susceptibility for cleavage of the bond when the polyplex is in the endosome or in the lysosome of a target cell.

In a particular embodiment, a polyplex or a polyplexing agent can be provided, wherein another linker as described above is directly or indirectly covalently linked to the targeting ligand, whereby the ligand can preferably be bound to the polyplexing agent, more preferably to the polymeric scaffold.

In a specific embodiment, a pharmaceutical composition is provided comprising the polyplex as disclosed herein, and preferably further comprising a pharmaceutically acceptable excipient and/or pharmaceutically acceptable diluent.

Advantageously, a pharmaceutical composition accordingly to any embodiment as described above is provided

and can be selected from any one or more of the following:

- a composition comprising the polyplex as disclosed herein, wherein the polyplex is conjugated, (preferably via the polymeric scaffold) to the targeting ligand that is Cetuximab, and wherein the nucleic acid preferably encodes for a toxin, more preferably saporin or dianthin for use in the treatment of EGFR positive cancer, possibly being selected from any one of colon cancer, breast cancer, and head and neck cancer; or

- a composition comprising the polyplex as disclosed herein, wherein the nucleic acid is encoding for factor IX, preferably human factor IX (hFIX) or a functional fragment thereof for use in the treatment of haemophilia B, preferably wherein the polyplex is conjugated (preferably via the polymeric scaffold) to the targeting ligand capable of recognising an endocytic receptor on a liver cell; and/or

- a composition comprising the polyplex as disclosed herein, wherein the polyplex is conjugated, (preferably via the polymeric scaffold) to a targeting ligand that is GalNAc for use in the treatment of conditions in liver cells (hepatocytes), wherein the nucleic acid is encoding for a gene or gene fragment, capable of improving the condition in liver cells.

In another aspect, a polyplexing agent is provided comprising a polymeric scaffold and a saponin being a triterpenoid 12,13-dehydrooleanane-type saponin that in an unreacted state comprises an aldehyde group at position C-23 of the saponin's aglycone core structure,

wherein the saponin is covalently bound with the polymeric scaffold by a linker adapted to cleave and release the saponin from the polymeric scaffold under conditions present in an endosome or a lysosome, preferably being an acid-sensitive linker adapted to cleave and release the saponin from the polymeric scaffold under acidic conditions present in an endosome or a lysosome, and

preferably wherein the amount of the saponin molecule equivalents per amount of molecules of the polymeric scaffold in the polyplexing agent is comprised between 0.05 and 4, preferably between 0.1 and 2, more preferably between 0.15 and 1, even more preferably between 0.2 and 0.75, and most preferably is either equal to or about 0.5.

In a possible embodiment of the above aspect, a polyplexing agent is provided wherein the polymeric scaffold is or comprises PAMAM, preferably being at least generation 3 PAMAM or higher, and/or preferably is a PAMAM of ethylenediamine core, more preferably wherein the PAMAM comprises at least one pegylated group comprising at least one PEG adapter preferably comprising 5-15 PEG units, and/or preferably wherein the pegylation degree of the PAMAM is comprised between on average 1.5-20 equivalents of PEG adapter per PAMAM core; and
preferably wherein the amount of the saponin molecule equivalents per amount of molecules of the PAMAM in the polyplexing agent is comprised between 0.1 and 4, preferably between 0.15 and 2, more preferably between 0.2 and 1, even more preferably between 0.25 and 0.75, and most preferably is either equal to or about 0.5.

Alternatively, in a different possible embodiment of the above aspect, a polyplexing agent is provided, wherein the polymeric scaffold is or comprises a polypeptide comprising between 5 to 25 lysines and at least one cysteine, preferably being exactly one cysteine, more preferably wherein the polypeptide comprises exactly one cysteine, preferably wherein the exactly one cysteine is flanked by a string of 1-7 glycines from each side, preferably 1-5 glycines from each side, more preferably 2-4 glycines, most preferably is flanked by 3-7 glycines from N-terminal side, preferably being 4 glycines; and by 1-4 glycines from C-terminal side, preferably being 2 glycines, and wherein optionally the polypeptide comprises a C-terminal tyrosine, optionally wherein the polypeptide is further modified to comprise at least an azide group, preferably configured to display said single azide group for conjugating of the targeting ligand, most preferably wherein the polypeptide is represented by a general formula of, from N-terminus to C-terminus: 2-25 Lys; 3-7 Gly; 1 Cys; 2-4 Gly, and possibly at least one Tyr, possibly followed by a residue comprising azide group, preferably being azidolysine, preferably 6-azido-L-lysine; preferably wherein the polypeptide is represented by an amino acid sequence given by SEQ ID NO. 1: KKKKKKKKKKKKKKKGGGGCGGY or SEQ ID NO. 2: KKKKKKKKKKKKKKKGGGGCGGYK* wherein K* is azidolysine, preferably 6-azido-L-lysine,
preferably wherein the amount of the saponin molecule equivalents per amount of molecules of the polypeptide in the polyplexing agent is comprised between 0.1 and 1, preferably between 0.15 and 0.75, more preferably between 0.2 and 0.6, and most preferably is either equal to or about 0.5.

In a preferred embodiment of any one of the possible embodiments of the above aspect, a polyplexing agent is provided comprising a targeting ligand recognised by an endocytic receptor, for example being any one of the receptors mentioned already above.

In a related aspect a kit of parts is provided for delivering a nucleic acid into a cell *in vitro,* the kit of parts comprising the polyplexing agent as described herein above, and optionally further comprising instructions for use,

preferably containing instructions for combining of the polyplexing agent with a nucleic acid for preparation of a polyplex for delivering the nucleic acid into a cell.

In a yet another aspect, a method of preparation of the disclosed herein polyplex is provided, the method comprising the step of conjugating of a polymeric scaffold with a saponin to obtain a polyplexing agent, wherein the saponin is a triterpenoid 12,13-dehydrooleanane-type saponin that in an unreacted state comprises an aldehyde group at position C-23 of the saponin's aglycone core structure, wherein said conjugating is either

- performed after mixing an at least one nucleic acid with the polymeric scaffold to obtain the polyplex; or
- performed before mixing the at least one nucleic acid with the polymeric scaffold to obtain the polyplex.

In a preferred embodiment, the above method is provided, wherein the amount of the saponin molecule equivalents per amount of molecules of the polymeric scaffold in the polyplexing agent is adjusted during the conjugating to be between 0.05 and 4, preferably between 0.1 and 2, more preferably between 0.15 and 1, even more preferably between 0.2 and 0.75, and most preferably is either equal to or about 0.5.

In a preferred embodiment, the above method is further provided comprising conjugation of a targeting ligand recognised by an endocytic receptor to the polymeric scaffold, wherein the targeting ligand either is covalently linked to the polymeric scaffold before the preparation of the polyplex, or is covalently linked to the polymeric scaffold after the preparation of the polyplex, preferably wherein the conjugation of targeting ligand uses click-chemistry; more preferably wherein the conjugation of targeting ligand comprises reaction with an azide group, possibly being a reaction involving a terminal azidolysine residue, preferably being azido-L-lysine residue.

In another preferred embodiment, the above method is provided, wherein the polymeric scaffold is or comprises PAMAM, preferably being at least generation 3 PAMAM or higher, and/or preferably being a PAMAM of ethylenediamine core, more preferably wherein the PAMAM comprises at least one pegylated group comprising at least one PEG adapter preferably comprising 5-15 PEG units, and/or preferably wherein the pegylation degree of the PAMAM is comprised between on average 1.5-20 equivalents of PEG adapter per PAMAM core, and preferably wherein the amount of the saponin molecule equivalents per amount of molecules of the PAMAM in the polyplexing agent is adjusted during the conjugating to be between 0.1 and 4, preferably between 0.15 and 2, more preferably between 0.2 and 1, even more preferably between 0.25 and 0.75, and most preferably is either equal to or about 0.5; or

wherein the polymeric scaffold is or comprises a polypeptide comprising between 5 to 25 lysines and at least one cysteine, preferably being exactly one cysteine, more preferably wherein the polypeptide comprises exactly one cysteine, preferably wherein the exactly one cysteine is flanked by a string of 1-7 glycines from each side, preferably 1-5 glycines from each side, more preferably 2-4 glycines, most preferably is flanked by 3-7 glycines from N-terminal side, preferably being 4 glycines; and by 1-4 glycines from C-terminal side, preferably being 2 glycines, and wherein optionally the polypeptide comprises a C-terminal tyrosine, most preferably wherein the polypeptide is represented by a general formula of, from N-terminus to C-terminus: 2-25 Lys; 3-7 Gly; 1 Cys; 2-4 Gly, and possibly at least one Tyr, possibly followed by a residue comprising azide group, preferably being azidolysine, preferably 6-azido-L-lysine; preferably wherein the polypeptide is represented by an amino acid sequence given by SEQ ID NO. 1: KKKKKKKKKKKKKKKKGGGGCGGY or SEQ ID NO. 2: KKKKKKKKKKKKKKKKGGGGCGGYK* wherein K* is azidolysine, preferably 6-azido-L-lysine; and preferably wherein the amount of the saponin molecule equivalents per amount of molecules of the polypeptide in the polyplexing agent is adjusted during the conjugating to be between 0.1 and 1, preferably between 0.15 and 0.75, more preferably between 0.2 and 0.6, and most preferably is either equal to or about 0.5.

Finally, a method of preparation of the disclosed herein polyplexing agent is also provided, the method comprising the step of conjugating of a polymeric scaffold with a saponin being a triterpenoid 12,13-dehydrooleanane-type saponin that in an unreacted state comprises an aldehyde group at position C-23 of the saponin's aglycone core structure, preferably whereby said conjugating is done by a linker adapted to cleave and release the saponin from the polymeric scaffold under conditions present in an endosome or a lysosome, preferably being an acid-sensitive linker adapted to cleave and release the saponin from the polymeric scaffold under acidic conditions present in an endosome or a lysosome, more preferably wherein the amount of the saponin molecule equivalents per amount of molecules of the polymeric scaffold in the thus obtained polyplexing agent is adjusted during the conjugating to be between 0.05 and 4, preferably between 0.1 and 2, more preferably between 0.15 and 1, even more preferably between 0.2 and 0.75, and most preferably is either equal to or about 0.5.

**EXAMPLES**

***Materials:***

SO1861 was isolated and purified by Analyticon Discovery GmbH from raw plant extract obtained from *Saponaria officinalis L.*

Cetuximab was purchased from the pharmacy (Charité, Berlin)

PAMAM (Dendritech, Midland, Michigan)

Peptide scaffolds K16, K16C and K16CPEG were purchased as custom synthesis from GeneCust (Boynes, France).

Saporin-Nanoplasmid (NTC9385R-Sap-BGH pA, 2585 bp) was produced by Nature Technology Corporation, Lincoln, USA.

pEGFP-N3 plasmid (Gene Bank Accession: U57609) was amplificated in Dh5a-cells and isolated using Qiagen®Plasmid Mega Kit (Qiagen, Hilden, Germany).

6-Maleimidocaproic Acid Hydrazide, Trifluoroacetic Acid Salt (EMCH*TFA, >95%, TCI)

Tris(2-carboxyethyl)phosphine hydrochloride (TCEP, 98%, Sigma-Aldrich),

5,5-Dithiobis(2-nitrobenzoic acid) (DTNB, Ellman's reagent, 99%, Sigma-Aldrich),

N-Ethylmaleimide (NEM, 98%, Sigma-Aldrich),

1,4-Dithiothreitol (DTT, 98%, Sigma-Aldrich),

Isopropyl alcohol (IPA, 99.6%, VWR),

Tris(hydroxymethyl)aminomethane (Tris, 99%, Sigma-Aldrich),

Tris(hydroxymethyl)aminomethane hydrochloride (Tris.HCL, Sigma-Aldrich),

Polyethylene glycol sorbitan monolaurate (TWEEN 20, Sigma-Aldrich),

Dulbecco's Phosphate-Buffered Saline (DPBS, Thermo-Fisher),

Ethylenediaminetetraacetic acid disodium salt dihydrate (EDTA-$Na_2$, 99%, Sigma-Aldrich),

sterile filters 0.2 $\mu$m and 0.45 $\mu$m (Sartorius),

Dimethyl sulfoxide (DMSO, 99%, Sigma-Aldrich),

N-(2-Aminoethyl)maleimide trifluoroacetate salt (AEM, 98%, Sigma-Aldrich),

L-Cysteine (98.5%, Sigma-Aldrich),

deionized water (DI) was freshly taken from Ultrapure Lab Water Systems (MilliQ, Merck).

**Abbreviations**

| | |
|---|---|
| DIPEA | N,N-diisopropylethylamine |
| DLS | dynamic light scattering |
| DMEM | Dulbecco's modified Eagles medium |
| DMF | N,N-dimethylformamide |
| DTME | Dithiobismaleimidoethane |
| DTT | Dithiothreitol |
| EDCI.HCl | 3-((Ethylimino)methyleneamino)-N,N-dimethylpropan-1-aminium hydrochloride |

| | |
|---|---|
| EDTA | Ethylenediaminetetraacetic acid |
| EMCH.TFA | N-(ε-maleimidocaproic acid) hydrazide, trifluoroacetic acid salt |
| FBS | Fetal bovine serum |
| GPC | Gel Permeation Chromatography |
| HATU | 1-[Bis(dimethylamino)methylene]-1H-1,2,3-triazolo[4,5-b]pyridinium 3-oxid hexafluorophosphate |
| IR | Infrared |
| min | Minutes |
| MWCO | molecular weight cut-off |
| NHS | N-hydroxysuccinimid |
| NMM | 4-Methylmorpholine |
| NMR | Nuclear magnetic resonance |
| PAMAM | Poly(amidoamine) |
| PCR | polymerase chain reaction |
| PDI | Poly dispersity index |
| PDT | Pyridine 2-thione |
| PEG | Polyethylene glycol |
| PEG4-SPDP | 2-Pyridyldithiol-tetraoxatetradecane-N-hydroxysuccinimide |
| SDS | Sodium dodecyl sulfate |
| SEC | Size exclusion chromatography |
| SMCC | Succinimidyl 4-(N-maleimidomethyl)cyclohexane-1-carboxylate |
| TBEU | (Tris-(hydroxymethyl)-aminomethan)-Borat-EDTA-Urea |
| TCEP | Tris(2-carboxyethyl)phosphine hydrochloride |
| Temp | Temperature |
| TFA | Trifluoroacetic acid |
| THPP | Tris(hydroxypropyl)phosphine |
| TNBS | 2,4,6-trinitrobenzene sulfonic acid |
| Tris | Tris(hydroxymethyl)aminomethane |

***Methods:***

**S01861-EMCH**

SO1861 was obtained from *Saponaria officinalis L* (Analyticon Discovery GmbH), and was coupled to respective handles according to methods known in the art, e.g. as described in patent EP3773737 of SAPREME TECHNOLOGIES. Figure 1A schematically shows derivatization of the aldehyde group at position C-23 of the saponin SO1861 aglycone core structure with an acid-sensitive maleimide-andhydrazide (EMCH) crosslinker for conjugating e.g. thiols such as cysteines. Figure 1B shows a skeletal structural formula of derivatized SO1861-EMCH with the acid-sensitive hydrazone bone at C-23 of the saponin SO1861 aglycone core structure, which bond cleaves under acidic conditions resulting in recreation of the aldehyde group at position C-23, which participates in endosomal escape enhancing properties of SO1861 and other similar saponins as listed in Table 1.

**Synthesis of PAMAM scaffolds covalently bound with S01861 by acid-sensitive linkers**

As a proof-of-principle model system, generation 5 of PAMAM dendrimers (PAMAM G5) with ethylenediamine core were chosen for preparation of dendrimer-based scaffolds and for investigation of the impact of the scaffold modifications on crucial properties like polyplex solubility and DNA binding. PAMAM generation 5 (Dendritech, Midland, Michigan) was purchased lyophilized. Introduction of SO1861 via acid sensitive linkers to PAMAM was performed by first using 2-iminothiolane to transform a part of the PAMAM amines to thiol groups, which readily react with the maleimide present in the EMCH linker. The reaction scheme is shown in Figure 1C. In an exemplary protocol for conducting the reaction, PAMAM (14.8 mg, 1.0 equiv.) was dissolved in phosphate buffered saline (PBS, 1 mL) and 2-iminothiolane hydrochloride (0.73 mg, 10 equiv., Sigma-Aldrich) was added from a stock solution in PBS (1.0 mg/mL). The mixture was shaken (800 rpm) for 1 h at room temperature. Afterwards, SO1861-EMCH (3.65 mg, 5 equiv.) dissolved in PBS was directly added to the reaction without intermediate purifications. The mixture was stirred at room temperature for 3 h. The conjugate was purified from unreacted SO1861-EMCH using dialysis tubes (12 kDa MWCO, Roth) against PBS. After 24 h, PBS was exchanged by 10 mM $NaCO_3$ solution to deprotonate the amine groups, before media was changed to 150 mM NaCl solution to ensure solely chloride ions as counter ions. After another 24 h, dialysis was continued with Milli-Q water for 12 h. The dialysis fraction was lyophilized to afford the equipped dendrimer as a white solid (10.8 mg) in more than 75% yield, which was then stored at -20°C.

### Synthesis of pegylated PAMAM scaffolds including PAMAM-PEG-$N_3$

It was hypothesised that it would be advantageous for gene therapy *in vivo,* and in particular for systemic delivery, that the polyplexes could be targeted by cell-/tissue-/organ-specific ligands. Consequently, a scaffold modification synthesis strategy was established for conjugating such ligands, which is schematically shown in Figure 2A and B, in detail and in simplification, respectively.

Firstly, libraries of PAMAM scaffolds were prepared and included modified scaffolds with varying modifications such as dye conjugation (not shown) and different numbers of PEG-based click adapters for conjugation of various targeting ligands like antibodies or proteins.

For the introduction of the mentioned modifications, the amines of PAMAM were utilized. PAMAM G5 contains 128 amine groups. However, as these positively charged groups provide the cationic surface charge for polyplex formation through their ionic interactions with the negatively charged phosphate backbones of the nucleic acids, it was decided to modify only a proportion of them.

Consequently, only a limited number of the amines were subjected to pegylation in order to guarantee that a sufficient positive charge remains for effective polyplexation of the modified PAMAM with nucleic acid such as a plasmid. To do so, the modifications of amine groups were controlled stoichiometrically. As the first modification of PAMAM it was decided to introduce binding sites for conjugation of a targeting ligand. To do so, a heterobifunctional polyethylene glycol (PEG)-spacer was employed that contains an active N-hydroxysuccinimide (NHS) ester on one side and an azide functionality on the other side. Then, optimization testing was performed to investigate PEG-spacers having length of 6 or 12 PEG units, and to investigate the properties of scaffolds containing different numbers of spacers (2.5 to 80 PEG-spacers per PAMAM). The length of the PEG-spacer was selected to ensure the accessibility for the ligand in order to ensure efficient conjugation thereof to the modified PEG scaffold. In a typical reaction, PAMAM G5 (20 mg, 1.0 equiv.) was dissolved in DMSO (1.0 mL) and NHS-$PEG_{12}$-$N_3$ (2.57 mg, 5.0 equiv.) dissolved in DMSO (0.26 mL) was added. The reaction was shaken (800 rpm) at room temperature for 2 h. Subsequently, the reaction was diluted with water (1:8 v/v) and placed in a dialysis membrane (12 kDa MWCO, Roth). The diluted reaction mixture was dialyzed against water for 72 h to remove DMSO, N-hydroxysuccinimide (NHS) and unreacted PEG linker. During this time, dialysis medium was changed 6 times. The dialysis fraction was lyophilized, and the product was obtained as a colorless solid (19.9 mg, 90% yield). The product was characterized using infrared (IR) spectroscopy and $^1$H NMR. Different dendrimer-based scaffolds were characterised by standard laboratory methods for the pegylation degree, size, stability, purity, and polydispersity.

### Synthesis of pegylated PAMAM scaffolds conjugated with endosomal escape enhancing saponin

After establishing the respective strategies for conjugating the dendrimer scaffolds with saponins and with PEG spacers, a protocol was optimised for conjugating SO1861-EMCH to PAMAM dendrimer endowed with different amounts of PEG spacers. Initially, we have observed that thiolation for PAMAM led to aggregation, as a consequence a one-pot thiolation-Michael addition protocol was devised. A schematic representation of an example of such 2-step conjugation protocol is shown in Figure 3A and B, in more detail and in simplification, respectively. An exemplary protocol for conjugating pegylated PAMAM with saponin is as follows: PAMAM G5-(PEG-$N_3$)$_5$ (14.8 mg, 1.0 equiv.) was dissolved in PBS (1 mL) and 2-iminothiolane hydrochloride (0.73 mg, 10 equiv., Sigma-Aldrich) was added from a stock solution in PBS (1.0 mg/mL). The mixture was shaken (800 rpm) for 1 h at room temperature. Afterwards, SO1861-EMCH (3.65 mg, 5 equiv.) dissolved in PBS was added to the reaction. The mixture was stirred at room temperature for 3 h. The conjugate was purified from unreacted SO1861-EMCH using dialysis tubes (12 kDa MWCO, Roth) against PBS. After 24 h, PBS was exchanged by 10 mM $NaCO_3$ solution to deprotonate the amine groups, before media was changed to 150 mM NaCl solution to ensure solely chloride ions as counter ions. After another 24 h, dialysis was continued with Milli-Q water for 12 h. The dialysis fraction was lyophilized to afford the equipped dendrimer as a white solid (10.8 mg) in 77% yield. The product was characterized using $^1$H NMR.

### Polyplex formation between PAMAM-PEG-$N_3$ and plasmid DNA

Polyplexes were formed by mixing e.g. plasmid DNA with the PAMAM conjugates (PAMAM G5, PAMAM-(PEG-$N_3$)$_x$ or PAMAM-(PEG-$N_3$)$_x$-(SO1861)$_y$ including different degrees of pegylation and SO1861 loading). For example, PAMAM conjugates were dissolved in HEPES (10 mM, pH 7.1) at 5 mg/mL one day before polyplex formation. The plasmid DNA was diluted to 0.2 $\mu$g/$\mu$L with HEPES buffer and PAMAM solutions were diluted in HEPES (10 mM HEPES, pH 7.1) to the calculated concentration which depended on the desired N/P ratio. For the N/P ratio, primary amines of PAMAM and the positive ion introduced by 2-iminothiolane were considered. For 20 $\mu$L of polyplexes, 12.5 $\mu$L PAMAM solutions in HEPES buffer were added to 7.5 $\mu$L (1.5 $\mu$g) DNA solution and mixed thoroughly by pipetting for 5 seconds, followed by brief vortexing and subsequent centrifugation. The polyplexes were allowed to incubate for at least 30 min

at room temperature before being used in subsequent experiments. Polyplexes were characterized by gel retardation (DNA binding, Figure 7F), dynamic light scattering (hydrodynamic diameter), laser Doppler electrophoresis (LDE, for zeta potential), and transmission electron microscopy (TEM) (Figure 8C).

When testing the polyplexes, it was observed that the pegylation of PAMAM improved their stability. Interestingly, we also observed that the pegylation of PAMAM mitigated toxicity of the formulated therewith polyplexes.

Notably, it was also observed that coupling of EMCH-SO1861 to PAMAM not only can be performed before the formation of a polyplex (i.e. before the scaffold is mixed with the nucleic acid) but also, that it can efficiently progress also on an already assembled polyplex. Here, the Michael coupling of the SO1861-EMCH is done after the scaffold was mixed with the nucleic acid using the same conditions as for the reaction before polyplexation. Both of these modes of coupling are shown in Figure 4A and B. In the latter case (Figure 4B), the distribution of such saponin coupled only after the polyplex formation, is expected to primarily be superficial (cf Figure 5B for schematic representation). Conversely, in the instance of conjugating the PAMAM and saponin before polyplexation (Figure 4A), the saponin will be equally distributed throughout the scaffold, and consequently, will remain at such distribution in a polyplex formed by such scaffold after addition of the nucleic acid (cf Figure 5A for schematic representation of such polyplex).

**Conjugation of PAMAM scaffolds with ligands**

As the next modification, the pegylated and equipped-with-a-click-adapter PAMAM scaffolds, with or without saponins, were conjugated with a ligand attached to a second click adapter, compatible with the one equipped as part of the modified PAMAM. Many of such compatible click adapters are known in the art and suitable for performing click-chemistry reactions, such as, to name a few, azide-alkyne Huisgen cycloaddition, strain-promoted azide-alkyne cycloaddition (SPAAC), or the inverse electron-demand Diels-Alder (IEDDA) reaction. The conjugation with a second click adapter equipped targeting ligand (for example being an antibody, a peptide, a protein, a carbohydrate, a vitamin) can be done before the polyplex formation, or because of the high thermodynamic driving force and specificities of click-chemical syntheses, it can even be performed on an already formed polyplex as shown in Figure 4A-C. The latter mode of conjugation has the additional advantage of ensuring that the targeting ligand is presented on the surface of the polyplex. According to this procedure, the following targeted PAMAM scaffolds were prepared:

**Transferrin-targeted polyplex**

First, the PAMAM conjugate (PAMAM G5, PAMAM-$(PEG-N_3)_x$ or PAMAM-$(PEG-N_3)_x$-$(SO1861)_y$) was polyplexed with the desired plasmid as described in the "Polyplex formation" section above. For a polyplex of PAMAM-$(PEG-N_3)_{6.2}$-$(S01861)_{0.4}$) at N/P 8 and volume of 240 μL (3.5 nmol, 108.8 μg PAMAM conjugate), a solution of Cy3-transferrin-PEG-DBCO in 10 mM PBS pH 7.4 (97.9 μL, 0.3 mg/mL, 0.35 nmol equivalent to 10% of mol PAMAM conjugate) was added to the polyplex solution and SPAAC bio-conjugation was allowed to proceed for 48 h at room temperature under shaking (800 rpm). The progress of the reaction was followed by quantifying the fraction of unreacted Cy3-transferrin-DBCO using SDS-PAGE electrophoresis (10%, 200 V, 60 min) by comparison with a standard calibration curve made with solutions of Cy3-transferrin-DBCO (100, 50, 25, 12.5%) of known concentration prepared under identical conditions (Figure 9A). Gel quantification was done by using a Versadoc imaging system equipped with a Cy3 filter. The functionalized polyplex was used without further purification.

**Cetuximab-targeted polyplex**

First, the PAMAM conjugate (PAMAM G5, or PAMAM-$(PEG-N_3)_x$) was polyplexed with the desired plasmid as described in the "Polyplex formation" section above. For a polyplex of PAMAM-$(PEG-N_3)_6$ at N/P 8 and volume of 100 μL (1.1 nmol, 42.2 μg PAMAM conjugate), a solution of Cy5-cetuximab-PEG-DBCO in 10 mM PBS pH 7.4 (54.1 μL, 0.3 mg/mL, 0.011 nmol, equivalent to 10 mol% of PAMAM conjugate i.e., 0.1 equivalents relative to PAMAM conjugate) was added to the polyplex solution and SPAAC bio-conjugation was allowed to proceed for 48 h at room temperature under shaking (800 rpm). The progress of the reaction was followed by quantifying the fraction of unreacted Cy5-cetuximab-DBCO using SDS-PAGE electrophoresis (10%, 200 V, 90 min) by comparison with a standard calibration curve made with solutions of Cy5-cetuximab-DBCO (100, 50, 25, 12.5%) of known concentration prepared under identical conditions (Figure 9B+C). Gel quantification was done by using a Versadoc imaging system equipped with a Cy5 filter. The functionalized polyplex was used without further purification.

**GalNAc-targeted polyplex**

First, the PAMAM conjugate (PAMAM G5, PAMAM-(PEG-N3)x or PAMAM-(PEG-N3)x-(SO1861)y) was polyplexed with the desired plasmid as described in the "Polyplex formation" section above. For a polyplex of PAMAM-(PEG-

$N_3)_{6.2}$ at N/P 8 and volume of 200 μL (3.0 nmol, 95.0 μg PAMAM conjugate), a solution of $(GalNAc)_3$-DBCO in 10 mM HEPES pH 7.1 (19.3 μL, 0.03 mg/mL, 0.3 nmol, equivalent to 10 mol% of PAMAM conjugate i.e., 0.1 equivalents relative to PAMAM conjugate) was added to the polyplex solution and SPAAC bio-conjugation was allowed to proceed for 48 h at room temperature under shaking (800 rpm). The functionalized polyplex was used without further purification. Conjugation of another example of a targeting ligand not being an antibody is shown in Figure 4C, whereby transferrin is schematically shown to be conjugated to the click chemical adapters of PAMAM present in a polyplex by SPAAC bioconjugation reaction.

**Generation of peptide scaffolds and pegylated peptide scaffolds**

As a second proof-of-principle model system, following a triblock copolymer strategy, several peptide scaffolds containing a poly-lysine strip were designed and tested with or without the presence of a PEG8-spacer. Based on the analytical characterisation of the PEG-equipped scaffolds, the peptide sequence was modified. In the initial designs, the peptides included several cysteines for the introduction of free thiol groups into the peptide scaffold for conjugation purposes, for example by means of thiol-maleimide coupling chemistry. However, due to aggregate formation, possibly due to interpeptide disulfide formation, the number of cysteines was reduced to only one. Further modifications of the initial peptide sequence included the introduction of several glycines directly preceding and directly following the cysteine to avoid or reduce steric hindrances, and the incorporation of tyrosine to facilitate the detection and quantitation using ultraviolet-visible (UV/Vis) spectrophotometry. Lastly, a terminal azide group was introduced in a form of an azidolysine (6-azido-L-lysine) for conjugation purposes with potential targeting ligands. For certain types of ligands that could suffer of steric hindrance, a PEG-spacer was introduced just before the azidolysine. Satisfactory results were observed with a PEG8-spacer. The two optimized designs of peptide scaffolds ($K_{16}G4CG_2Y\text{-}PEG_8\text{-}K(N_3)\text{-}NH_2$ and H-$K_{16}G_4CG_2YK(N_3)\text{-}NH_2$) are schematically shown in Figure 6A. The peptide scaffolds are C-terminally amidated to increase their stability and prolong their shelf life.

Coupling of SO1861-EMCH to the single cysteine of those designed peptide scaffolds using classic Michael addition with thiol-maleimide coupling chemistry is schematically shown in Figure 6B (in the example using an *N*-ε-maleimidocaproic acid hydrazide linker present in the SO1861-EMCH), with a simplified indication of the reaction conditions. SO1861-EMCH was conjugated to the modified peptide in DPBS at pH 6.5 to promote the protonation of the lysine residues, thus minimizing the cross-reactivity of the maleimide group with the amine group of the lysines. Additionally, the amount of SO1861-EMCH was drastically reduced in relation to the initial experiments. A molar peptide to SO1861-EMCH ratio of 4:1 was chosen, theoretically corresponding to fourthiol groups per maleimide group. This was assumed as suitable to minimize the undesired cross-reaction with lysine residues while retaining enough accessible thiols. MALDI-MS analysis confirmed the formation of equipped peptide scaffolds under the described conditions. The Michael addition of SO1861-EMCH was optimized to be performed in DPBS at pH 6.5 using less than one equivalent (0.25-0.5) of SO1861-EMCH. In previous attempts to perform the conjugation reaction at pH 6.0 in citrate buffer, the conjugation reaction was equally efficient. However, despite intensive dialysis, complete exchange of the citrate anions for chloride anions was not achieved. The presence of the trivalent citrate anions in the final SO1861-functionalized peptide resulted in a significant deterioration in DNA complexation efficiency. In addition, the polyplexes prepared with those peptide scaffolds exhibited a strong aggregation tendency and low stability.

***Analytical and Preparative methods***

**Spectrophotometry*:*** Concentrations were determined using either a Thermo Nanodrop 2000 spectrometer or Perkin Elmer Lambda 365 Spectrophotometer.

**DLS measurements:** DLS measurements were performed on a Malvern Nano ZS (Malvern Instruments, U.K.), operating at 633 nm with a 173° scattering angle, at 25 or 37 °C. DLS mean diameters were obtained from the intensity particle size distribution provided by Malvern Zetasizer Software. DLS histograms were obtained from the intensity particle size distributions.

**Zeta-potential:** The zeta-potential values of the polyplexes were obtained by laser doppler anemometry (LDA), measuring the mean electrophoretic mobility (Malvern Zetasizer Nano ZS, Malvern Instruments, U.K.). Measurements were performed in 10 mM HEPES pH 7.1, 150 mM NaCl.

**TEM measurements:** TEM measurements were performed on a JEOL JEM-1011 operated at 100 kV electron microscope, equipped with a camera S5 MegaView G2. A drop of a solution of polyplexes (0.1 mg/mL) was settled on a PELCO® TEM carbon type-B film copper grid and allowed to dry at room temperature for 1 h.

**Infrared Spectroscopy:** FT-IR spectra were recorded on a Bruker Vertex 70v, equipped with a RockSolid interferometer, after preparing the samples (1-2 mg) as KBr pellets. Spectra were processed using OPUS 7.8 software (Bruker).

**NMR Spectroscopy:** $^1H$ NMR spectra were recorded on a 11.7 T Bruker DRX-500 spectrometer. Chemical

shifts are reported in ppm (δ units) downfield from the HOD residual solvent peak ($CD_3OD$). Experiments were recorded acquiring 64-512 scans, with a pre-scan delay (d1) of 12 s, and an acquisition time (aq) of 4 s at 300 K. $^{13}C$ NMR spectrum was recorder on a 9.39 T Varian Innova 400 spectrometer. MestReNova 14.2 software (Mestrelab Research) was used for spectra processing.

**Cell culture conditions:** HEK293FT, A431, A2058, Neuro2A, Hepa1-6, MDA-MB 468 were maintained in 10% FBS-supplemented DMEM medium (PAN-Biotech GmbH, or Lonza Group, Basel, Switzerland) + Pen/Strep at 37°C with 5% $CO_2$. Cells were treated with different concentrations of polyplex (as indicated); made with a polymeric scaffold (comprising of PAMAM or K16-polypeptide) and a DNA plasmid. SO1861-EMCH was conjugated into/onto these polyplexes or co-administrated at 966 or 4000 nM to the different cell lines *in vitro* (as indicated). For this, cells were seeded in a 96 well plate at 5.000-10.000 c/w in 100 μL/well and incubated overnight or for 24 hrs at 37°C. Before treatment, the SO1861 and polyplexes were diluted separately in DPBS (PAN-Biotech GmbH) to 10x the required final concentration. The cells received 80 μL/well fresh media, followed by 20 μL/well polyplex solution and 20 μL/well saponin solution. In particular, before K16-polyplex treatments, K16-polyplexes were formulated in 10 mM HEPES, incubated for 30 min at room temperature and then diluted with complete cell culture medium. The complete cell culture medium was exchanged against 100 μL transfection medium. For co-administration of SO1861-EMCH, the transfection medium was supplemented with 2 μg/mL (= 966 nM) SO1861-EMCH. Control transfections were performed with Lipofectamine™ 3000 Transfection Reagent (Thermo Fisher Scientific) according to the manufacturer's instruction, using either 120 pM or 100ng plasmid and with a media change 4-5 hrs after transfection.

**Cell viability assay:** Cells were seeded in a 96 well plate at 5.000-10.000 cells/well in 100 μL/well and incubated overnight at 37°C.

Before treatment, the mAb-SO1861 and polyplexes were diluted separately in DPBS (PAN-Biotech GmbH) to 10x the required concentration. The cells received 80 μL/well fresh media, followed by 20 μL/well polyplex solution and 20 μL/well saponin solution. Control transfections were performed with Lipofectamine™ 2000 Transfection Reagent (Thermo Fisher Scientific) according to the manufacturer's instruction, using 160 pM plasmid and with a media change 4-5 hrs after transfection.

After treatment the cells were incubated for 72 hr at 37°C before the cell viability was determined by a MTS-assay performed according to the manufacturer's instruction (CellTiter 96® AQueous One Solution Cell Proliferation Assay, Promega). Briefly, the MTS solution was diluted 20x in DMEM without phenol red (PAN-Biotech GmbH) supplemented with 10% FBS (PAN-Biotech GmbH). Media was removed, after which 100 μL diluted MTS solution was added per well. The plate was incubated for approximately 20-30 minutes at 37°C. Subsequently, the optical density at 492 nm was measured according to instructions, using a SpectraMax ID5 plate reader (Molecular Devices). For quantification the background signal of 'medium only' wells was subtracted from all other wells, before the ratio of untreated/treated cells was calculated by dividing the background corrected signal of untreated wells over the background corrected signal of the treated wells.

**Live cell imaging (cancer cell lines):** Directly after treatment the cell plates were placed into the xCELLigence RTCA eSight (Aligent), to simultaneous image cell growth and cell transfection. 4 images/well were taken every 60 min for up to 72 hr, on the brightfield and green fluorescent channel (300 ms exposure time). Cell growth was determined, using the xCELLigence RTCA eSight sofware, based on the average % confluency/well using the brightfield images, while the GFP-signal (calculated as average % green confluence/well) was used to determine transfection efficiency.

### *Results and Discussion*

### Example 1. Characterisation of PAMAM-based scaffolds before loading with saponins

The dendrimer-based scaffolds were characterised with respect to the degrees of their substitution, size, stability, purity, polydispersity, and structural analysis. Pegylation and thiolation were determined by $^1H$ nuclear magnetic resonance (NMR). As an example, analysis of a PAMAM G5-$(PEG\text{-}N_3)_5$ by $^1H$ NMR revealed the incorporation of the $PEG_{12}$ linkers associated to a new multiplet at 3.80-3.64 ppm, characteristic of the ethylene glycol monomers, not present in the spectrum of commercial PAMAM (Figure 7A and B). A pegylation degree of 5 for this sample was determined by integration of these characteristic multiplet signals at 3.80-3.64 ppm, accounting for 48 of the 52 protons in the $PEG_{12}$ linker, relative to that of the multiplet at 2.71-2.59 ppm due to the 252 internal protons present in G5 PAMAM. The presence of the $PEG_{12}$ linker was also confirmed by $^{13}C$ NMR that showed the expected characteristic signals of the $PEG_{12}\text{-}N_3$ groups (Figure 7C).

Due to the characteristic, intense signal of the azide functional group by IR spectroscopy, IR spectra for PAMAM G5 dendrimers after functionalization with PEG-$N_3$ linkers were recorded. Figure 7D shows the IR spectra of a commercial PAMAM and three pegylated derivatives with pegylation degrees of 3.2, 6.2, and 12.4 (as indicated). The presence of a new band at ca. 2100 $cm^{-1}$ reveals the azide incorporation in the pegylated dendrimers. In addition, a new band is observed at ca. 1100 $cm^{-1}$ corresponding to the ether functional group indicative of the presence of PEG. Notably, both

bands increase their intensity as the degree of pegylation increases, confirming the efficient incorporation of PEG-$N_3$.

The hydrodynamic diameter of PAMAM G5-(PEG-$N_3$) with pegylation degrees between 3.2 and 12.4 was determined by DLS as shown in Figure 7E. Namely, sizes calculated in these histograms using volume distributions revealed a range of 8.1-8.8 nm, confirming no substantial difference in size within the range of pegylation degree. Table 2 shows sizes and PDI by DLS of polyplexes prepared from equipped PAMAM G5 with increasing degree of pegylation.

***Table 2.***

| dissolved in PBS (5 mg/mL) | + HEPES to N/P 8 | | + NaCl | | |
|---|---|---|---|---|---|
| **$PEG_{12}$-$N_3$ per PAMAM by $^1$H NMR** | **$D_H$ [nm]** | **PDI** | **$D_H$ [nm]** | **PDI** | **delta with NaCl** |
| 4.6 | 152.3 | 0.201 | 204.0 | 0.190 | 51.7 |
| 9.2 | 170.7 | 0.218 | 264.0 | 0.210 | 93.3 |
| 20.6 | 218.0 | 0.242 | 243.3 | 0.229 | 25.3 |
| 35.2 | 170.0 | 0.311 | 189.0 | 0.188 | 19.0 |

For the dendrimer system, it was found that all pegylated PAMAMs were able to efficiently bind DNA at amine to phosphate ratios (N/P) equal or higher to four. To determine the N/P ratios, the PAMAM scaffold libraries equipped with increasing amount of click adapter were tested for their capacity to complex the effector gene and form stable polyplexes. The capability to complex plasmid DNA was studied using pUC19 plasmid via gel retardation assays with the fluorescent dye pico488, which fluorescence is quenched upon intercalation into double-stranded DNA. All PAMAM conjugates were able to efficiently complex the effector plasmid at low N/P ratios (Figure7F a-c).

Regarding polyplex stability, it was confirmed that polyplexes of unmodified PAMAM tend to aggregate at higher ionic strength. However, $PEG_{12}$ linkers (5-40 equivalents per PAMAM) seemed to stabilize the polyplexes at a physiological salt concentration, whereas $PEG_6$ linkers were only able to stabilize the polyplex at the highest degree of pegylation (40 equivalents per PAMAM). Therefore, PAMAM scaffolds modified with $PEG_{12}$-linkers were used for transfection experiments. Here, unmodified PAMAM and pegylated PAMAM showed significant increase in transfection efficiency when cell medium was supplemented with SO1861 (up to 20-fold increase). With increasing degree of pegylation, this effect was reduced indicating that higher pegylation advantageously impaired non-specific internalization of polyplexes. Importantly, lower toxicity was also observed for systems with higher degree of pegylation. However, scaffolds with higher than 20 degrees of pegylation of PAMAM exhibited poorer transfection efficiencies and the enhancer effect of endosomal escape-enhancing saponin like SO1861 was weak or absent. Consequently, pegylation degrees being on average about 3-13 equivalents of PEG adapter per PAMAM core were evaluated as advantageous.

## Example 2. Characterisation of PAMAM-based scaffolds loaded with saponins

Dendrimer-based scaffolds equipped with SO1861 were characterized by standard laboratory methods for their structure, size, stability, purity, and polydispersity. For instance, the incorporation of SO1861 onto the dendritic scaffold has been clearly demonstrated by $^1$H NMR. As shown in Figure 7G, the spectrum of a PAMAM G5-(PEG-$N_3$)$_5$-(SO1861)$_{0.38}$ reveals the presence of signals corresponding to both the dendritic PAMAM G5-(PEG-$N_3$)$_5$ precursor and SO1861-EMCH. The SO1861 loadings in these samples were determined by relative integration of the characteristic 252 internal PAMAM dendrimer protons relative to the SO1861 hydrazone proton, a singlet at 7.89 ppm, and a multiplet at 5.37-5.26 ppm due to three protons at the SO1861 structure. IR spectroscopy also gives relevant information that confirm the incorporation of SO1861 onto the dendritic scaffold. Figure 7H shows new characteristic bands in the IR spectrum of PAMAM G5-(PEG-$N_3$)$_{12.4}$-(SO1861)$_5$ associated to the carbonyl, ethers and alcohol groups in SO1861. The incorporation of SO1861 onto the dendritic periphery was also confirmed by an increase, up to ca. 11 nm, in the hydrodynamic diameter of equipped scaffolds with PEGylation degree in the range 3.2-12.4 and a SO1861 loading of 5 (Figure 71).

Initially, PAMAM molecules were equipped with a constant amount of five equivalents of SO1861 per PAMAM. This constant number was selected in view of the fact that in polyplexes, many PAMAMsaponin units will be present per nucleic acid molecule, thus yielding a substantial amount of endosomal escape enhancing (EEE) saponin per polyplex. Therefore, five saponin units per PAMAM were initially estimated as sufficient for concerting endosomal escape activity while at the same time allowing stable polyplexation with plasmids. In general, all equipped dendrimers were able to efficiently complex DNA (Figure 7F d-i). However, for all tested PAMAM conjugates with five or more SO1861-equivalents per PAMAM, the observed transfection was very poor compared to lipofectamine and the augmentation by SO1861 was absent. To investigate possible solutions for improving the transfection efficiency, a library of equipped PAMAMs with a constant and low degree of pegylation and decreasing content of SO1861 (4, 2, 1, 0.5) was prepared.

It was observed that transfection ability of polyplexes was recovered for lower saponin loadings and by adding e.g. SO1861 to the cell medium, indicating that high SO1861 loading may lead to too dense packaging of the polyplexes. To address this, the location of SO1861 was moved from equal distribution in the entire polyplex (Figure 4A and 5A) to the polyplex periphery (Figure 4B and 5B), under the assumption that the release of the EEE saponins from the polyplex would be facilitated when presented on the surface. In this manner, 0.5 and 2 equivalents of SO1861-EMCH per PAMAM were conjugated to the polyplex surface. It was found that polyplex sizes were not affected by surface conjugation and that PAMAM with low loadings of conjugated SO1861 was able to substantially increase the transfection efficiency by non-specific binding mechanism (in the absence of targeting ligand) compared to polyplexes made with PAMAM alone and compared to when free SO1861 is used at the same concentration. However, it was noteworthy that this observed transfection augmentation was very similar irrespective whether 0.5 equivalents of SO1861 was conjugated to the surface or equally distributed by conjugation to PAMAM, indicating that at low saponin-conjugation levels, the saponin distribution is not an important factor for increasing transfection efficiency.

In view of the above, PAMAM conjugates with low to medium pegylation and low SO1861 content of 0.5 equivalents per PAMAM were characterized again for the polyplexing capability. The hydrodynamic diameters of the polyplexes before and after addition of salt are shown in Table 3, which lists sizes and PDI by DLS of polyplexes prepared from PAMAM G5 with increasing pegylation degree and at low (0.5) or high (5) SO1861 loading (DLS histograms shown in Figure 8A).

***Table 3:***

| **dissolved in PBS (5 mg/mL)** | **+ HEPES to N/P 8** | | | **+ NaCl** | | | | **zeta-potential (mV)** |
|---|---|---|---|---|---|---|---|---|
| **$PEG_{12}$-$N_3$ per PAMAM by NMR** | **$D_H$ [nm]** | **ST D** | **PDI** | **$D_H$ [nm]** | **STD** | **PDI** | **delta with NaCl** | |
| **3.2** | 132.3 | 2.3 | 0.163 | 505.7 | 32.1 | 0.288 | 373.4 | |
| **6.2** | 173.7 | 14.1 | 0.260 | 247.1 | 12.1 | 0.211 | 73.3 | |
| **12.4** | 157.0 | 3.0 | 0.216 | 158.9 | 9.2 | 0.162 | 2.0 | |
| **0.5 equivalents S01861** | **$D_H$ [nm]** | **ST D** | **PDI** | **$D_H$ [nm]** | **STD** | **PDI** | **delta with NaCl** | |
| **3.2** | 136.1 | 3.5 | 0.172 | 389.4 | 17.6 | 0.222 | 253.2 | 21.3 ± 0.9 |
| **6.2** | 137.5 | 1.9 | 0.256 | 153.5 | 0.9 | 0.215 | 16.0 | 22.4 ± 1.6 |
| **12.4** | 198.3 | 13.3 | 0.255 | 187.2 | 1.4 | 0.191 | -11.1 | 19.8 ± 3.7 |
| **5 equivalents S01861** | **$D_H$ [nm]** | **ST D** | **PDI** | **$D_H$ [nm]** | **STD** | **PDI** | **delta with NaCl** | |
| **3.2** | 157.1 | 0.3 | 0.170 | 198.9 | 5.1 | 0.181 | 41.8 | 24.3 ± 0.1 |
| **6.2** | 158.4 | 6.9 | 0.199 | 220.6 | 1.4 | 0.182 | 62.1 | 22.4 ± 1.1 |
| **12.4** | 215.8 | 3.0 | 0.247 | 349.0 | 7.3 | 0.262 | 133.2 | 25.6 ± 1.1 |

The results indicate that pegylation improves stability against salt and prevents swelling, even at low SO1861 loading of 0.5 equivalents per PAMAM. Interestingly, the results also show that lower SO1861 loading appears to be beneficial since at high SO1861 loadings the particles appear to swell more.

The zeta-potential of the equipped polyplexes was determined by laser Doppler electrophoresis (LDE) that measures the mean electrophoretic mobility. Very similar values, in the range 20-25 mV, were obtained for all polyplexes (Table 3 and Figure 8B) that reveal a positive charge, in accordance with the excess of cationic PAMAM scaffold used in the polyplexing process (N/P 8). No significant differences were observed varying the pegylation degree or SO1861 loading.

While DLS provides information about the hydrodynamic diameter of the polyplexes in solution, transmission electron microscopy (TEM) gives information about their size and morphology in dried state. Figure 8 shows the DLS histograms (Figure 8A) and TEM images (Figure 8C) of polyplexes prepared from PAMAM-G5-$(PEG\text{-}N_3)_x$-$(SO1861)_5$ (PEGylation degrees 3.2, 6.2, 12.4) and pEGFP-N3 at N/P 8. The intensity size distribution measured by DLS revealed polyplex diameters in the range 140-220 nm, which are in accordance with those observed by TEM. Interestingly, TEM reveals spherical and compact polyplexes, indicative of an efficient DNA complexation by the PAMAM scaffolds.

**Example 3. Characterisation of peptide-based scaffolds**

Based on *in vitro* polyplex formation experiments, it is believed that the sequence of the peptide scaffold for polyplexation and ligand conjugation was optimized. In summary, the core structure of the peptide scaffold was selected to be a linear polypeptide containing a polylysine tail, a single cysteine surrounded by several glycines followed by a tyrosine, an optional $PEG_8$-spacer, and, optionally, a terminal azidolysine (i.e. a lysine carrying an azide group) for conjugation purposes using click-chemistry, for example of targeting ligands.

Initial studies indicated that the scaffolds having the selected sequences $H-K_{16}G_4CG_2Y-PEG_8-K(N_3)-NH_2$ and $H-K_{16}G_4CG_2YK(N_3)-NH_2$ (as shown in Figure 6) when equipped with 0.25 and 0.5 equivalents SO1861 respectively, showed higher transfection efficiencies *in vitro* than their non-equipped equivalents in combination with saponin supplemented transfection medium, with no observed toxicity. To further characterise polyplexes made with such peptide scaffolds, a set thereof varying in the incorporation of the $PEG_8$-spacer and the EEE loading (0.25/0.5 equivalents) was prepared and tested for their capability to complex pEGFP-N3 as model plasmid. Different N/P ratios were used for the complexation of a constant amount of DNA in 10 mM HEPES, pH 7.1. The proportion of free DNA was quantified using a fluorescent DNA-binding dye that enables sensitive quantitation of small amounts of double-stranded DNA in solution. The results are shown in Figure 10 demonstrating that all studied scaffolds complexed ≥ 98% of the DNA at an N/P ratio of 10.

**Example 4. Transfection of non-targeted polyplexes made with PAMAM scaffold in HEK cells**

First, transfection experiments were performed in HEK293FT cells using 40 pM or 120 pM pEGFP-N3 plasmid polyplexed with any one of the following scaffolds at an N/P ratio of 8:

A. PAMAM G5-(PEG $N3)_{3.5}$,
B. PAMAM G5-(PEG $N3)_{3.5}$ + 4000 nM SO1861-EMCH,
C. PAMAM G5-(PEG $N3)_{3.5}$-$(SO1861)_{0.5eq}$ (SO1861 conjugated after DNA plasmid polyplexation),
D. PAMAM G5-(PEG $N3)_{3.5}$-$(SO1861)_5$ (SO1861 conjugated before DNA plasmid polyplexation).

Lipofectamin transfection was used as control. The results are shown in Figure 11, and include light microscopy photographs of HEK cells transfected with polyplexes made with A-D (Figure 11A-D, respectively, 40 pM pEGFP-N3 plasmid), as well as readings of the HEK confluencies (Figure 11E, indicated as % confluency) and percent of GFP-expressing cells (Figure 11F) on day 3 post transfection with both concentrations of the plasmid.

The data shows that, in the absence of the saponin, pEGFP-N3 polyplexed with PAMAM G5-(PEG $N3)_{3.5}$ could only transfect very few cells, whereas nearly all cells were transfected with PAMAM G5-(PEG $N3)_{3.5}$ polyplexes co-administered with 4000 nM SO1861-EMCH. However, in the latter instance strong toxicity was observed. Interestingly, polyplexes made with PAMAM G5-(PEG $N3)_{3.5}$-$(SO1861)_{0.5eq}$ or PAMAM G5-(PEG $N3)_{3.5}$-$(SO1861)_5$ were very effective in pEGFP-N3 transfection and showed a relatively high GFP protein expression without substantial toxicity. Consequently and surprisingly, lower loading in fact resulted in higher transfection efficiency.

The transfection efficiency and viability of the cells were also measured via live imaging, which readings are at each 6 hr interval post transfection in total of 73 hrs are shown in Figure 12. During these experiments, it was observed that transfection with unconjugated 4000 nM SO1861-EMCH showed strong decrease in cell viability that correlated with the increased concentration of the plasmid (Figure 12A), even though SO1861-EMCH alone is not toxic at this concentration (data not shown). Conversely, pEGFP polyplexed with either of PAMAM-G5 scaffolds conjugated with SO1861 (external or internal) showed efficient transfection (Figure 12B or C) without any decrease in cell viability (Figure 12A).

**Example 5. Transfection of non-targeted polyplexes made with PAMAM scaffold in A2058 cells**

Next, transfection of pEGFP-N3 plasmid was performed in A2058 cancer cells and the expression of eGFP was captured using light microscopy 72 h after incubation with (A) PAMAM G5-$(PEG)_{3.5}$, (B) PAMAM G5-$(PEG)_{3.5}$ polyplexes with 0.5 equiv. surface SO1861 (conjugated after DNA plasmid polyplexation), (C) PAMAM G5(-$PEG)_{3.5}$-$(SO1861)_{0.5}$ (conjugated before DNA plasmid polyplexation), (D) PAMAM G5-$(PEG)_{3.5}$ + 40 nM SO1861-EMCH, (E) PAMAM G5-$(PEG)_{3.5\,+}$ 40 nM SO1861 (corresponding to 0.5 equivqlents SO1861), or (F) Lipofectamine as a control. Pictures of brightfield images with an overlay of the GFP signal from these transfections are shown in Figure 13A-F, respectively.

The results show that polyplexes of PAMAM G5(PEG-$N3)_{3.5}$ were successful in transfecting only very few cells (Figure 13A). Similarly, marginal transfection was observed with PAMAM G5-(PEG $N3)_{3.5}$ supplemented with 40 nM SO1861-EMCH or SO1861 (Figure 13D-E, respectively). The best transfection efficiencies were observed for polyplexes

made with PAMAM G5-(PEG $N_3)_{3.5}$-(SO1861)$_{0.5eq}$ regardless whether SO1861 was equipped on the PAMAM's surface or distributed evenly (Figure 13B-C, respectively). Importantly, the latter polyplexes did not cause any toxicity while being more efficient in transfection than the gold standard transfection reagent Lipofectamine (Figure 13F). Furthermore, the results emphasize the importance of SO1861 conjugation to PAMAM since co-administration at the same concentration does not affect transfection efficiency. The conjugation does not only increase transfection but also mitigates toxicity. In addition, lower doses are needed compared to the co-administration.

**Example 6. Transfection of non-targeted polyplexes made with PAMAM scaffold in A431 cells**

Next, nanoplasmid npSaporin that encodes for a ribosome inactivating toxin known as saporin, was transfected into A431 cancer cells in a form of a polyplex made with a polyplexing agent either being PAMAM G5-(PEG-$N_3)_{62}$ or PAMAM G5-(PEG-$N_3)_{12.4}$ alone or conjugated with 0.5 equivalents of SO1861 (i.e. PAMAM G5-(PEG-$N_3)_{6.2}$(SO1861)$_{0.5}$ or PAMAM G5-(PEG-$N_3)_{12.4}$(SO1861)$_{0.5}$, respectively), SO1861 being conjugated before polyplexation. The results are shown in Figure 14 and demonstrate that saporin DNA nanoplasmid polyplexes made with PAMAM G5-(PEG-$N_3)_{6.2}$ or with PAMAM G5-(PEG-$N_3)_{12.4}$ did not induce saporin toxin mediated cell killing, which means that they were incapable of delivering the saporin plasmid into the cells' cytoplasm allowing for the saporin toxin production. Interestingly, however, scaffolds PAMAM G5-(PEG-$N_3)_{6.2}$(S01861)$_{0.5}$ and PAMAM G5-(PEG-$N_3)_{12.4}$(S01861)$_{0.5}$ that carry EEE saponin conjugated with an acid-sensitive bond that releases it in the endosome, were capable of effectuating an efficient cytoplasmic saporin plasmid delivery, as demonstrated by cell killing (IC50 npSaporin: 60 pM (PEG6.2) and 200pM (PEG12.4)). Interestingly, a higher pegylation degree (12.4 vs 6.2) on the PAMAM G5 decreased the efficiency of cytoplasmic delivery, presumably due to a reduction in cellular uptake of the product.

**Example 7. Transfection of non-targeted polyplexes made with peptide poly-lysine scaffold (K16C) in A431 cells**

Next, transfection experiments in A431 cells were performed with the peptide poly-lysine scaffold (K16C) that was polyplexed with saporin-encoding nanoplasmid npSaporin. The K16C peptide was either used alone, with free acid-sensitive saponin derivative (SO1861-EMCH), or was conjugated with 0.25 equivalents of saponin [K16C-(SO1861)$_{0.25eq}$]. The results of this treatment on cell viability are shown in Figure 15, which indicate that K16C peptide scaffold polyplexed with the saporin-encoding nano-plasmid DNA shows practically no cell killing activity up to about 5000 pM of plasmids in the absence of a free saponin derivative or acid-releasable saponin conjugated to the peptide scaffold. Co-administration of free SO1861-EMCH with the K16C-polyplexes does result in an enhanced cell killing activity in A431 cells, but importantly, polyplexes made with K16C-(SO1861)$_{0.25seq}$ were the most efficient in saporin gene delivery into the cytoplasm from all conditions tested.

**Example 8. Transfection efficiency of non-targeted polyplexes made with poly-lysine scaffolds (K16C) in different cell lines**

Neuro2A, HEK293FT, Hepa1-6, and MDA-MB 468 cells were seeded at 5.000-10.000 cells in 100 μL cell culture medium per well in 96-well-plates (Greiner Bio-One, Frickenhausen, Germany) and cultivated for 24 h under the standard conditions described in 'Cell culture conditions' section above. Polyplexes were formulated freshly before transfection by admixing same volumes of peptide scaffold solution (1 mg/mL stock in ultrapure water, diluted with 10 mM HEPES, pH 7.1) and pEGFP-N3 solution in 10 mM HEPES, pH 7.1, followed by a 30 min incubation period at room temperature. All polyplexes were formed with an N/P ratio of 10. Lipofectamine control was prepared with the same DNA concentrations according to the manufacturers protocol. After the incubation, the polyplex solution was diluted with complete cell culture medium to a final concentration of 1 ng complexed DNA/μL in the transfection medium. Each polyplex was tested with and without the addition of free SO1861-EMCH in the transfection medium, so in the first case the transfection medium was supplemented with SO1861-EMCH to a final concentration of 2 μg/mL (966 nM). After the 24 hour incubation period, the cell culture medium was exchanged against the transfection medium. 100 μL transfection medium were added per well, resulting in 100 ng complexed pEGFP-N3 plasmid per well. Cells were incubated for 48 hours under the normal culture conditions before measuring the transfection efficiency. The culture medium was removed and the cells were detached using trypsin. The obtained cell suspensions were kept on ice until they were analysed using flow cytometry. All cells exhibiting higher FITC-H (indicating eGFPexpression) signals than the untreated control cell population were considered transfected. 10000 single cells were included in the analysis for each sample, those were gated based on their forward scatter. Transfection efficiency was calculated as ratio of the number of transfected cells and total number of cells.

Each experiment was performed three times. Data for Neuro2A and HEK293FT cell line is expressed as mean of three measurements, normal distribution of the data was confirmed with Shapiro-Wilk-test, significant differences were calculated with Student-t-test (two-sided, *p<0.05, **p<0.01, ***p<0.001, ****p<0.0001). Data for Hepa1-6 and

MDA-MB 468 cell line is expressed as mean of nine measurements (three independent experiments with each 3 wells per condition), significant differences were calculated with U-Test (*$p<0.05$, **$p<0.01$, ***$p<0.001$, ****$p<0.0001$).

The results are shown in Figure 16 and reveal significantly higher transfection efficiencies for the SO1861-loaded polyplexes (i.e. those that were formed with SO1861-equipped peptide scaffolds) than for the polyplexes with free SO1861-EMCH supplementation in HEK293FT, Hepa1-6, and MDA-MB 468 cells. In Neuro2A cells external SO1861-EMCH supplementation resulted in a similar transfection rate. Additional supplementation of external SO186-EMCH (dotted bars) to the SO1861-loaded polyplexes did not increase their transfection efficiencies further. Depending on the cell-line, SO1861-loaded polyplexes exhibit a comparable or even better transfection efficiency than lipofectamine which is used as positive control. In addition to the above, toxicity assessment using impedance-based cell viability measurement (graph not shown) preformed on HEK293FT cells, confirmed the observation that transfection of the EEE-loaded polyplexes did not have a negative impact on cell growth and viability.

**Example 9. Transfection of Gal-NAc targeted polyplexes made with PAMAM scaffold**

Next, PAMAM-based scaffolds were functionalised with GalNAc as a ligand for targeting hepatocytes, which are cells that express ASGR1 receptor. External loading of 0.5 equivalents of SO1861 per PAMAM G5-(PEG $N_3)_{4.0}$ was used as an exemplary setting. The scaffolds with SO1861 and/or GalNAc ligand (10%) were then polyplexed with a DNA construct encoding for the human coagulation factor IX (hFIX) as effector gene. The resulting polyplexes were subsequently transfected to primary murine hepatocytes, as an ex *vivo* model of hFIX delivery for treating haemophilia B. After 72 h, the medium was collected from the transfected primary murine hepatocytes and the secreted hFIX levels where analyzed through a hFIX-specific ELISA assay.

The data is shown in Figure 17 indicating that hFIX expression levels were synergistically higher when both a liver-cell targeting ligand GalNAc and a surface SO1861-EMCH were conjugated together to the PAMAM G5-(PEG $N_3)_{4.0}$ model scaffold, as compared to only GalNAc conjugation or SO1861-EMCH conjugation alone.This preliminary data suggests that a targeted delivery of therapeutic genes based on the presented herein systems could be applicable for hemophilia B and other broad ranges of diseases, such as cancer, in function of the choice of an appropriate targeting ligand.

**Example 10. Stability of PAMAM and peptide polyplexes in blood**

Sufficient stability in blood is required for the polyplexes to sustain prolonged circulation in the blood following systemic administration into the patient's body. To measure the stability of the described herein polyplexes when exposed to human blood, an assay was designed as shown in Figure 18A. The assay starts with incubation of the polyplexes in blood serum for 1 h at 37 °C. Afterwards, intermediate steps are performed for the sample purification and polyplex isolation. A heat shock procedure was used for blood protein precipitation, followed by a centrifugation to separate the solid and the liquid part of the sample. The supernatant was then mixed with heparin to release the plasmid from the polyplex. In a final step, PCR and gel electrophoresis were used for qualitative assessments and product size control (Figure 18B). The results show that both the PAMAM- or peptide-based polyplexes were stable in human blood serum at body temperature for at least 1 hr.

**Example 11. Toxicity tests of PAMAM and peptide scaffolds or polyplexes in blood**

To get a first estimation of the haemolytic activity of the scaffolds and its potential to induce aggregation of red blood cells, possible disruption of red blood cells was investigated by measuring the release of haemoglobin into the supernatant according to the scheme shown in Figure 19A.

First, the red blood cells (RBCs) were treated with solutions containing PAMAM- or peptide-based scaffolds or polyplexes obtained therewith at different concentrations (2.5-80 $\mu$g/mL). The incubation was performed for 3 h at 37°C. RBCs treated with PBS under same conditions were used as a negative control while samples treated with Triton X-100 (10%) were used as a positive control corresponding to conditions of full haemolysis. After centrifugation, the absorbance at 540 nm ($OD_{540}$) of the supernatants was recorded with a spectrometer. The following equation was used for the calculation of the percentages of haemolysis:

$$Hemolysis\ (\%) = \left(\frac{OD(sample) - OD(0)}{OD(100) - OD(0)}\right) \times\ 100\%$$

The results of haemolytic activity of two different scaffolds (PAMAM G5 and peptide $K_{16}C$), and polyplexes obtained therewith are shown in Figure 19B and indicate a surprisingly low haemolysis degree for all cases.

As a next step, the erythrocyte aggregation propensity of the scaffolds was examined under an optical microscope to visualize possible interactions of the scaffolds and polyplexes with RBCs and their surface (Figure 19C).

The results show that polyplexes of K16C or PAMAM and the scaffold molecule K16C did not cause any RBC aggregation. Only a mild degree of irregular transformation of the cell surfaces was observed an only at the highest concentrations tested. In contrast, under this experiment set-up, the scaffold molecule PAMAM caused massive blood clotting reaction at higher concentrations, likely due to the interaction of its positively charged surface with the negatively charged cell membranes.

Next, the impact on haemostasis was investigated. It is known that to stop bleeding from blood vessels, three major steps are involved in haemostasis. These are vascular spasm, the platelet plug formation, and coagulation. It is widely accepted that two pathways are involved in clot formation in mammals; being the intrinsic and the extrinsic pathway. The activated partial thromboplastin time (aPTT) is a parameter used to detect disruptions in the intrinsic pathway and the prothrombin time (PT) reveals problems in the extrinsic part of the coagulation cascade. Consequently, to investigate the impact of the PAMAM- and peptide-based scaffolds on the patient's blood coagulation, these two parameters were measured in fresh whole blood after incubation of nanoparticles at different concentrations (0 nM-5 μM). The results for PAMAM or peptide K16C scaffolds or polyplexes made with them (as indicated) are shown in Table 4 containing Partial thromboplastin time (PTT) values (the reference is 26-40 s), and in Table 4 and 5 containing Prothrombin time (PT) values (The reference for PT is 70-130%). In both of the Tables, the values within the reference range are highlighted in grey, while the values in the remaining non-highlighted boxes are prolonged or not detectable. The extended coagulation times in Table 5 can likely be explained by electrostatic interactions between the positively charged scaffold molecules and the negatively charged coagulation factor proteins, which are responsible for the clot formation at the end of the common pathway of the cascade. The results indicate that systemic delivery of polyplexes using the presented herein scaffolds will likely not only be sufficiently stable in the blood but also well tolerated by the patient.

*Table 4.*

| Concentration / Nanomaterials | 0 nM | 0.1 μM | 0.5 μM | 1 μM | 5 μM |
|---|---|---|---|---|---|
| PAMAM G5 | 32.48 s | 34.15 s | 60.85 s | - | - |
| PAMAM G5-polyplexes | 31.5 s | 38 s | - | - | - |
| K16C | 31 s | 38.5 | - | - | - |
| K16C-polyplexes | 31.6 s | 30.15 | - | - | - |

*Table 5.*

| Concentration / Nanomaterials | 0 nM | 0.1 μM | 0.5 μM | 1 μM | 5 μM |
|---|---|---|---|---|---|
| PAMAM G5 | 100 % | 62 % | 29 % | 29 % | 0 % |
| PAMAM G5-polyplexes | 100 % | 71 % | 31.5 % | 27 % | 0 % |
| K16C | 100 % | 100 % | 82 % | 61 % | 33.5 % |
| K16C-polyplexes | 100 % | 62 % | 44 % | 41.5 % | 27 % |

| | |
|---|---|
| 100 % | - 13.9 s |
| 50 % | - 18.5 s |
| 25 % | - 29.5 s |
| 10% | - 65.8 s |

**Example 12 The in vivo toxicity assessment**

The *in vivo* toxicity of the prototypes was investigated in mice by determining the non-observed adverse effect level (NOAEL) for intravenous administration (data not shown). Severe side effects were not observed, neither for the highest dose (45 μg of plasmid) of the dendrimer-based prototype nor for the peptide-based prototype (15 μg). Only a locally limited inflammation was observed at the injection site. For performing histological analyses of mouse organs after treatments, mice were treated eight times with 5 μg nanoplasmid DNA encoding for saporin, which was either polyplexed by a peptide-based scaffold or a dendrimer-based scaffold. Spleens and livers were observed. The while pulpa of the spleens was activated in both cases. The liver of the animal that was treated with the peptide-based prototype showed single cell necrosis, while in the liver of the animal treated with the dendrimer-based scaffold, necrotic cell groups were observed. The results indicate only very low systemic intoxication after intravenous administration.

**Conclusions**

The data in general shows that plasmids polyplexed with either PAMAM or peptide scaffolds that are conjugated with releasable SO1861 via acid-sensitive linker can efficiently induce endosomal escape and cytoplasmic delivery of the plasmids, as demonstrated by GFP expression delivered in polyplexed pEGFP-N3 plasmids or by saporin-mediated cell killing in response to delivery of npSaporin.

The experiments further show that non-targeted polyplexes comprising a scaffold conjugated with releasable EEE saponin are efficient in delivering plasmids to cells *in vitro* and on their own show very low cytotoxicity. The results emphasize that conjugation of SO1861 to the scaffold provides an enormous augmentation to the plasmid delivery at a low dose, which in turn suggests that the presented herein technology can likely be translated to other effector genes such as genome editing cassettes, for which a transient expression in the specific cells is highly desirable.

Secondly, the preliminary data on targeted counterparts of these polyplexes indicates that these targeted polyplexes also appear to have the advantage of being less toxic to cells as compared to transfections performed with free saponin at comparable concentration levels. Consequently, it can be concluded that a prototype platform for creation of targeted polyplexes with EEE properties was successfully created and can be tested in gene therapy delivery *in vivo,* preferably using systemic administration. The latter is further supported by promising stability results and low haemolytic activity of the polyplexes as investigated in human blood.

**LITERATURE**

Agarwal, S., et al., 2012. PDMAEMA based gene delivery materials. Mater. Today 15 (9), 388-393.

Berkhout B, Liu YP. Towards improved shRNA and miRNA reagents as inhibitors of HIV-1 replication. Future Microbiol 9:561-571 (2014)

Böttger S - PhD thesis (2013): Untersuchungen zur synergistischen Zytotoxizität zwischen Saponinen und Ribosomen inaktivierenden Proteinen Typ I,

Clifford, A., et al., 2019. Biomimetic modification of poly-L-lysine and electrodeposition of nanocomposite coatings for orthopaedic applications. Colloids Surf. B 176, 115-121.

Clochard J et al, A new acetylated triterpene saponin from Agrostemma githago L. modulates gene delivery efficiently and shows a high cellular tolerance, International Journal of Pharmaceutics, Volume 589, 15 November 2020, 119822.

Démoulins, T., et al., 2016. Polyethylenimine-based polyplex delivery of self-replicating RNA vaccines. Nanomedicine 12 (3), 711-722.

Dufes C, et al. (2005) Adv Drug Deliv Rev 57:2177. DOI: 10.1016/j.addr.2005.09.017.

Fleck et al. (2019) in Table 1, row no. 28 - Juliane Deise Fleck, Andresa Heemann Betti, Francini Pereira da Silva, Eduardo Artur Troian, Cristina Olivaro, Fernando Ferreira and Simone Gasparin Verza, Saponins from Quillaja saponaria and Quillaja brasiliensis: Particular Chemical Characteristics and Biological Activities, Molecules 2019, 24, 171; doi:10.3390/molecules24010171

Fuchs H, et al. Glycosylated Triterpenoids as Endosomal Escape Enhancers in Targeted Tumor Therapies. Biomedicines 5(2017)

George LA, et al. Hemophilia B Gene Therapy with a High-Specific-Activity Factor IX Variant. N Engl J Med 377:2215-2227 (2017)

Gilleron J, et al. Image based analysis of lipid nanoparticle-mediated siRNA delivery, intracellular trafficking and endosomal escape. Nat Biotechnol 31:638-646 (2013)

He, Y., et al., 2013. Polyethyleneimine/DNA polyplexes with reduction-sensitive hyaluronic acid derivatives shielding for targeted gene delivery. Biomaterials 34 (4), 1235-1245.

Hess, K.L., et al., 2017. Polyplexes assembled from self-peptides and regulatory nucleic acids blunt toll-like receptor signaling to combat autoimmunity. Biomaterials 118, 51-62.

Holliger, P, et al. (1993) Proc. Natl. Acad. Sci. USA 90:6444-6448;

Poljak, R. J., et al. (1994) Structure 2:1121-1123

Jia et al., Major Triterpenoid Saponins from Saponaria officinalis, J. Nat. Prod. 1998, 61, 11, 1368-1373, Publication Date: September 19, 1998, https://doi.org/10.1021/np980167u

Kabanov, A.V., Kabanov, V.A., 1995. DNA complexes with polycations for the delivery of genetic material into cells. Bioconjug Chem 6 (1), 7-20.

Kabat, E.A., Wu, T.T., Perry, H.M., Gottesman, K.S. and Foeller, C. (1991) Sequences of Proteins of Immunological Interest. NIH publication No. 91-3242, Bethesda.

Kipriyanov, S. M., et al. (1994) Mol. Immunol. 31:1047-1058

Kipriyanov, S. M., et al. (1995) Human Antibodies and Hybridomas 6:93-101

Kochetkov N.K.; Khorlin A.J.; Ovodov J.S (1963). The structure of gypsoside - triterpenic saponin from gypsophila pacifica kom.. , 4(8), 477-482. doi:10.1016/s0040-4039(01)90658-6

Kodama, Y., et al., 2014. Biodegradable nanoparticles composed of dendrigraft poly-Llysine for gene delivery. Eur. J. Pharm. Biopharmaceutics 87 (3), 472-479.

Koping-Hoggard, M., et al., 2004. Improved chitosan-mediated gene delivery based on easily dissociated chitosan polyplexes of highly defined chitosan oligomers. Gene Ther. 11 (19), 1441-1452.

Kozielski, K.L., Rui, Y., Green, J.J., 2016. Non-viral nucleic acid containing nanoparticles as cancer therapeutics. Expert Opin Drug Deliv 13 (10), 1475-1487.

Kwoh, D.Y., et al., 1999. Stabilization of poly-L-lysine/DNA polyplexes for in vivo gene delivery to the liver. Biochim. Biophys. Acta 1444 (2), 171-190.

Lee CS, et al. Adenovirus-Mediated Gene Delivery: Potential Applications for Gene and Cell based Therapies in the New Era of Personalized Medicine. Genes Dis 4:43-63 (2017).

Mali P, et al. RNA-guided human genome engineering via Cas9. Science 339:823-826 (2013)

Moniuszko-Szajwaj et al., Highly Polar Triterpenoid Saponins from the Roots of Saponaria officinalis L., Helv. Chim. Acta, V99, pp. 347 - 354, 2016 (doi.org/10.1002/hlca.201500224).

Puras, G., et al., 2013. Oligochitosan polyplexes as carriers for retinal gene delivery. Eur. J. Pharm.Sci. 48 (1), 323-331.

Rumschöttel, J., et al., 2017. DNA polyplexes with dendritic glycopolymer-entrapped gold nanoparticles. Colloids and Surf. B 154, 74-81.

Sama S et al., Sapofectosid - Ensuring non-toxic and effective DNA and RNA delivery, International Journal of Pharmaceutics, Volume 534, Issues 1-2, 20 December 2017, Pages 195-205 (dx.doi.org/10.1016/j.ijpharm.2017.10.016)

Sama S et al., Structure-Activity Relationship of Transfection-Modulating Saponins - A Pursuit for the Optimal Gene Trafficker, Planta Med. Volume 85, pp. 513-518, 2019 (doi:10.1055/a-0863-4795)

Sama S, et al. Targeted suicide gene transfections reveal promising results in nu/nu mice with aggressive neuroblastoma. J Control Release 275:208-216 (2018)

Tros de Ilarduya, C., Sun, Y., Düzgünes, N., 2010. Gene delivery by lipoplexes and polyplexes. Eur. J. Pharm. Sci. 40 (3), 159-170.

van Setten (1995), Table 2 [Dirk C. van Setten, Gerrit van de Werken, Gijsbert Zomer and Gideon F. A. Kersten, Glycosyl Compositions and Structural Characteristics of the Potential Immuno-adjuvant Active Saponins in the Quillaja saponaria Molina Extract Quil A, RAPID COMMUNICATIONS IN MASS SPECTROMETRY, VOL. 9,660-666 (1995)].

Vasiliu, T., et al., 2017. Optimization of polyplex formation between DNA oligonucleotide and Poly(l-Lysine): experimental study and modeling approach. Int. J. Mol. Sci. 18 (6), 1291.

Wu, G.Y., Wu, C.H., 1987. Receptor-mediated in vitro gene transformation by a soluble DNA carrier system. J. Biol. Chem. 262 (10), 4429-4432.

Zhang, X., et al., 2010. Poly(L-lysine) nanostructured particles for gene delivery and hormone stimulation. Biomaterials 31 (7), 1699-1706.

Ziady, A.-.G., et al., 2003. Transfection of airway epithelium by stable PEGylated poly-Llysine DNA nanoparticles in vivo. Mol. Ther. 8 (6), 936-947.

## Claims

**1.** A polyplex comprising

at least one nucleic acid,
and a polyplexing agent, wherein the polyplexing agent comprises

a polymeric scaffold, and
a saponin being a triterpenoid 12,13-dehydrooleanane-type saponin that in an unreacted state comprises an aldehyde group at position C-23 of the saponin's aglycone core structure,

wherein the saponin is covalently bound with the polymeric scaffold by a linker adapted to cleave and release the saponin from the polymeric scaffold under conditions present in an endosome or a lysosome.

**2.** The polyplex according to claim 1, further comprising a targeting ligand recognised by an endocytic receptor.

**3.** The polyplex according to claim 1 or 2, wherein the linker is an acid-sensitive linker or is a linker configured to be enzymatically cleaved by an enzyme present in the endosome or the lysosome, preferably wherein the linker is an acid-sensitive linker adapted to cleave and release the saponin from the polymeric scaffold under acidic conditions present in the endosome or the lysosome.

**4.** Polyplex according to any one of the preceding claims, wherein the amount of the saponin molecule equivalents per amount of molecules of the polymeric scaffold in the polyplexing agent is comprised between 0.05 and 4, preferably between 0.1 and 2, more preferably between 0.15 and 1, even more preferably between 0.2 and 0.75,

and most preferably is either equal to or about 0.5.

5. The polyplex according to any one of the preceding claims, wherein the polymeric scaffold is or comprises polyamidoamine dendrimer, further referred to as PAMAM, preferably wherein the PAMAM comprises ethylenediamine core and/or wherein the PAMAM is at least generation 3 PAMAM or higher, preferably is generation 5 PAMAM.

6. The polyplex according to claim 5, wherein the PAMAM comprises at least one pegylated group comprising at least one PEG adapter, preferably wherein the PEG adapter comprises 5-15 PEG units, more preferably being 6-12 PEG units; even more preferably wherein the pegylation degree of the PAMAM is comprised between on average 1.5-20 equivalents of PEG adapter per PAMAM core, preferably being on average 2-16 equivalents of PEG adapter per PAMAM core, even more preferably being on average 3-13 equivalents of PEG adapter per PAMAM core, most preferably being on average selected from any one of 3.2, 6.2, and 12.4 PEG adapter equivalents per PAMAM core.

7. The polyplex according to any one of the claims 5-6, wherein the amount of the saponin molecule equivalents per amount of molecules of the PAMAM in the polyplexing agent is comprised between 0.1 and 4, preferably between 0.15 and 2, more preferably between 0.2 and 1, even more preferably between 0.25 and 0.75, and most preferably is either equal to or about 0.5.

8. The polyplex according to any one of the claims 1-5, wherein the polymeric scaffold is or comprises a polypeptide comprising between 5 to 25 lysines and at least one cysteine, preferably being exactly one cysteine, more preferably wherein the polypeptide comprises exactly one cysteine flanked by a string of 1-7 glycines from each side, preferably 1-5 glycines from each side, more preferably 2-4 glycines, even more preferably wherein the exactly one cysteine is flanked by 3-7 glycines from N-terminal side, preferably being 4 glycines; and by 1-4 glycines from C-terminal side, preferably being 2 glycines, and wherein optionally the polypeptide comprises a C-terminal tyrosine, optionally wherein the polypeptide is further modified to comprise at least an azide group, preferably configured to display said single azide group for conjugation purpose, and/or

   wherein the polypeptide is represented by a general formula of: 2-25 Lys; 3-7 Gly; 1 Cys; 2-4 Gly, and possibly at least one Tyr, possibly followed by a residue comprising an azide group, preferably being azidolysine, more preferably 6-azido-L-lysine;
   preferably wherein the polypeptide is represented by an amino acid sequence given by SEQ ID NO. 1: KKKKKKKKKKKKKKKKGGGGCGGY or SEQ ID NO. 2: KKKKKKKKKKKKKKKKGGGGCGGYK* wherein K* is azidolysine, preferably 6-azido-L-lysine.

9. The polyplex according to claim 8, wherein the amount of the saponin molecule equivalents per amount of molecules of the polypeptide in the polyplexing agent is comprised between 0.1 and 1, preferably between 0.15 and 0.75, more preferably between 0.2 and 0.6, and most preferably is either equal to or about 0.5.

10. Polyplex according to any one of the preceding claims, wherein the saponin comprises an aglycone core structure selected from quillaic acid or gypsogenin, preferably wherein the aglycone core structure is quillaic acid.

11. Polyplex according to any one of the preceding claims, wherein the saponin is at least a monodesmosidic saponin comprising at least a first saccharide chain comprising at least three sugar residues in a branched configuration, or is at least a bidesmosidic saponin further comprising a second saccharide chain comprising a glucuronic acid residue, preferably being a terminal glucuronic acid residue;

   preferably wherein the first branched saccharide chain comprises a terminal fucose residue and/or a terminal rhamnose residue and preferably comprises at least four sugar residues, and/or wherein the second saccharide chain is Gal-(1$\rightarrow$2)-[Xyl-(1$\rightarrow$3)]-GlcA;
   more preferably wherein the saponin comprises the first saccharide chain at position C-28 of the saponin's aglycone core structure, and/or the second saccharide chain at position C-3 of the saponin's aglycone core structure;
   most preferably wherein the first saccharide chain is a carbohydrate substituent at the C-28-OH group of the saponin's aglycone core structure and/or wherein the second saccharide chain is a carbohydrate substituent at the C-3beta-OH group of the saponin's aglycone core structure.

12. Polyplex according to any one of the preceding claims, wherein the saponin is any one or more of:

a) saponin selected from any one or more of list A:

- *Quillaja saponaria* saponin mixture, or a saponin isolated from *Quillaja saponaria,* for example Quil-A, QS-17-api, QS-17-xyl, QS-21, QS-21A, QS-21B, QS-7-xyl;
- *Saponinum album* saponin mixture, or a saponin isolated from *Saponinum album;*
- *Saponaria officinalis* saponin mixture, or a saponin isolated from *Saponaria officinalis;* and
- Quillaja bark saponin mixture, or a saponin isolated from Quillaja bark, for example Quil-A, QS-17-api, QS-17-xyl, QS-21, QS-21A, QS-21B, QS-7-xyl; or

b) a saponin comprising a gypsogenin aglycone core structure, selected from list B:
SA1641, gypsoside A, NP-017772, NP-017774, NP-017777, NP-017778, NP-018109, NP-017888, NP-017889, NP-018108, SO1658 and Phytolaccagenin; or
c) a saponin comprising a quillaic acid aglycone core structure, selected from list C:
AG1856, AG1, AG2, Agrostemmoside E, GE1741, Gypsophila saponin 1 (Gyp1), NP-017674, NP-017810, NP-003881, NP-017676, NP-017677, NP-017705, NP-017706, NP-017773, NP-017775, SA1657, Saponarioside B, SO1542, SO1584, SO1674, SO1700, SO1730, SO1772, SO1832, SO1861, SO1862, SO1904, QS-7, QS-7 api, QS-17, QS-18, QS-21 A-apio, QS-21 A-xylo, QS-21 B-apio and QS-21 B-xylo; or

preferably, the saponin is any one or more of a saponin selected from list B or C, more preferably, a saponin selected from list C.

**13.** Polyplex according to any one of the preceding claims, wherein the saponin is any one or more of AG1856, GE1741, a saponin isolated from *Quillaja saponaria,* Quil-A, QS-17, QS-21, QS-7, SA1641, a saponin isolated from *Saponaria officinalis,* SO1542, SO1584, SO1658, SO1674, SO1700, SO1730, SO1772,Saponarioside B, SO1832, SO1861, SO1862 and SO1904;

preferably wherein the saponin is any one or more of QS-21, SO1832, SO1861, SA1641 and GE1741; more preferably wherein the saponin is QS-21, SO1832 or SO1861;
most preferably being SO1861.

**14.** Polyplex according to any one of the preceding claims, wherein the saponin is a saponin isolated from *Saponaria officinalis,* preferably wherein the saponin is any one or more of SO1542, SO1584, SO1658, SO1674, SO1700, SO1730, SO1772, Saponarioside B, SO1832, SO1861, SO1862 and SO1904;

more preferably wherein the saponin is any one or more of SO1832, SO1861 and SO1862;
even more preferably wherein the saponin is SO1832 and SO1861;
most preferably being SO1861.

**15.** Polyplex according to any one of the preceding claims 2-14, wherein the endocytic receptor is selected from any of the following: epidermal growth factor receptor (EGFR) and receptor tyrosine-protein kinase erbB-2 (Her-2).

**16.** Polyplex according to any one of the claims 2-15, wherein the targeting ligand is an antibody or a binding fragment thereof, preferably is a monoclonal antibody such as Cetuximab or Panitumumab.

**17.** Polyplex according to any one of the preceding claims, wherein the nucleic acid is selected from a plasmid, a minicircle DNA, cDNA, mRNA, siRNA, oligonucleotide, and any modified equivalent thereof comprising one or more nucleotide analogues, preferably wherein the nucleic acid is a plasmid, more preferably wherein the nucleic acid comprises at least 0.25 kbp, more preferably at least 1 kbp, even more preferably at least 2.5 kbp, most preferably at least 5 kbp.

**18.** Polyplex according to any one of the preceding claims, wherein the linker is an acid-sensitive linker that comprises a covalent bond selected from any one or more of a hydrazone bond, an imine bond, an ester bond, a dioxalane bond, and an oxime bond, preferably wherein the acid-sensitive linker comprises a hydrazone bond.

**19.** Polyplex according to any one of the preceding claims, wherein the aldehyde group at position C-23 of the saponin's aglycone core structure has been engaged in forming the covalent bond with the linker.

**20.** Pharmaceutical composition comprising the polyplex according to any one of the preceding claims, preferably further

comprising a pharmaceutically acceptable excipient and/or pharmaceutically acceptable diluent.

**21.** Pharmaceutical composition according to claim 20, selected from any one or more of the following:

- a composition comprising the polyplex according to any one of the claims 1-19, wherein the targeting ligand is Cetuximab and the nucleic acid preferably encodes for a toxin, more preferably saporin or dianthin for use in the treatment of EGFR positive cancer, possibly being selected from any one of colon cancer, breast cancer, and head and neck cancer; or
- a composition comprising the polyplex according to any one of the claims 1-19, wherein the nucleic acid is encoding for factor IX or a functional fragment thereof for use in the treatment of haemophilia B.

**22.** A polyplexing agent comprising a polymeric scaffold and a saponin being a triterpenoid 12,13-dehydrooleanane-type saponin that in an unreacted state comprises an aldehyde group at position C-23 of the saponin's aglycone core structure,

wherein the saponin is covalently bound with the polymeric scaffold by a linker adapted to cleave and release the saponin from the polymeric scaffold under conditions present in an endosome or a lysosome, preferably being an acid-sensitive linker adapted to cleave and release the saponin from the polymeric scaffold under acidic conditions present in an endosome or a lysosome, and

preferably wherein the amount of the saponin molecule equivalents per amount of molecules of the polymeric scaffold in the polyplexing agent is comprised between 0.05 and 4, preferably between 0.1 and 2, more preferably between 0.15 and 1, even more preferably between 0.2 and 0.75, and most preferably is either equal to or about 0.5.

**23.** The polyplexing agent according to claim 22, wherein the polymeric scaffold is or comprises PAMAM, preferably being at least generation 3 PAMAM or higher, and/or preferably is a PAMAM of ethylenediamine core, more preferably wherein the PAMAM comprises at least one pegylated group comprising at least one PEG adapter preferably comprising 5-15 PEG units, and/or preferably wherein the pegylation degree of the PAMAM is comprised between on average 1.5-20 equivalents of PEG adapter per PAMAM core; and
preferably wherein the amount of the saponin molecule equivalents per amount of molecules of the PAMAM in the polyplexing agent is comprised between 0.1 and 4, preferably between 0.15 and 2, more preferably between 0.2 and 1, even more preferably between 0.25 and 0.75, and most preferably is either equal to or about 0.5.

**24.** The polyplexing agent according to claim 22, wherein the polymeric scaffold is or comprises a polypeptide comprising between 5 to 25 lysines and at least one cysteine, preferably being exactly one cysteine, more preferably wherein the polypeptide comprises exactly one cysteine, preferably wherein the exactly one cysteine is flanked by a string of 1-7 glycines from each side, preferably 1-5 glycines from each side, more preferably 2-4 glycines, most preferably is flanked by 3-7 glycines from N-terminal side, preferably being 4 glycines; and by 1-4 glycines from C-terminal side, preferably being 2 glycines, and wherein optionally the polypeptide comprises a C-terminal tyrosine, optionally wherein the polypeptide is further modified to comprise at least an azide group, preferably configured to display said single azide group for conjugating of the targeting ligand, most preferably wherein the polypeptide is represented by a general formula of, from N-terminus to C-terminus: 2-25 Lys; 3-7 Gly; 1 Cys; 2-4 Gly, and possibly at least one Tyr, possibly followed by a residue comprising azide group, preferably being azidolysine, preferably 6-azido-L-lysine; preferably wherein the polypeptide is represented by an amino acid sequence given by SEQ ID NO. 1: KKKKKKKKKKKKKKKKGGGGCGGY or SEQ ID NO. 2: KKKKKKKKKKKKKKKKGGGGCGGYK* wherein K* is azidolysine, preferably 6-azido-L-lysine,
preferably wherein the amount of the saponin molecule equivalents per amount of molecules of the polypeptide in the polyplexing agent is comprised between 0.1 and 1, preferably between 0.15 and 0.75, more preferably between 0.2 and 0.6, and most preferably is either equal to or about 0.5.

**25.** The polyplexing agent according to any one of claims 22-24, further comprising a targeting ligand recognised by an endocytic receptor.

**26.** Kit of parts for delivering a nucleic acid into a cell *in vitro* comprising the polyplexing agent according to any one of the claims 22-25, and optionally further comprising instructions for use, preferably containing instructions for combining of the polyplexing agent with a nucleic acid for preparation of a polyplex for delivering the nucleic acid into a cell.

**27.** A method of preparation of the polyplex according to any one of the claims 1-19, the method comprising the step

of conjugating of a polymeric scaffold with a saponin to obtain a polyplexing agent, wherein the saponin is a triterpenoid 12,13-dehydrooleanane-type saponin that in an unreacted state comprises an aldehyde group at position C-23 of the saponin's aglycone core structure, wherein said conjugating is either

- performed after mixing an at least one nucleic acid with the polymeric scaffold to obtain the polyplex;

or

- performed before mixing the at least one nucleic acid with the polymeric scaffold to obtain the polyplex;

preferably, wherein the amount of the saponin molecule equivalents per amount of molecules of the polymeric scaffold in the polyplexing agent is adjusted during the conjugating to be between 0.05 and 4, preferably between 0.1 and 2, more preferably between 0.15 and 1, even more preferably between 0.2 and 0.75, and most preferably is either equal to or about 0.5.

**28.** Method according to claim 27, further comprising conjugation of a targeting ligand recognised by an endocytic receptor to the polymeric scaffold, wherein the targeting ligand either is covalently linked to the polymeric scaffold before the preparation of the polyplex, or is covalently linked to the polymeric scaffold after the preparation of the polyplex, preferably wherein the conjugation of targeting ligand uses click-chemistry; more preferably wherein the conjugation of targeting ligand comprises reaction with an azide group, possibly being a reaction involving a terminal azidolysine residue, preferably being azido-L-lysine residue.

**29.** Method according to any one of claims 27-28, wherein the polymeric scaffold is or comprises PAMAM, preferably being at least generation 3 PAMAM or higher, and/or preferably being a PAMAM of ethylenediamine core,

more preferably wherein the PAMAM comprises at least one pegylated group comprising at least one PEG adapter preferably comprising 5-15 PEG units, and/or preferably wherein the pegylation degree of the PAMAM is comprised between on average 1.5-20 equivalents of PEG adapter per PAMAM core, and preferably wherein the amount of the saponin molecule equivalents per amount of molecules of the PAMAM in the polyplexing agent is adjusted during the conjugating to be between 0.1 and 4, preferably between 0.15 and 2, more preferably between 0.2 and 1, even more preferably between 0.25 and 0.75, and most preferably is either equal to or about 0.5;
or
wherein the polymeric scaffold is or comprises a polypeptide comprising between 5 to 25 lysines and at least one cysteine, preferably being exactly one cysteine,
more preferably wherein the polypeptide comprises exactly one cysteine,
preferably wherein the exactly one cysteine is flanked by a string of 1-7 glycines from each side, preferably 1-5 glycines from each side, more preferably 2-4 glycines, most preferably is flanked by 3-7 glycines from N-terminal side, preferably being 4 glycines; and by 1-4 glycines from C-terminal side, preferably being 2 glycines, and wherein optionally the polypeptide comprises a C-terminal tyrosine, most preferably wherein the polypeptide is represented by a general formula of, from N-terminus to C-terminus: 2-25 Lys; 3-7 Gly; 1 Cys; 2-4 Gly, and possibly at least one Tyr, possibly followed by a residue comprising azide group, preferably being azidolysine, preferably 6-azido-L-lysine;
preferably wherein the polypeptide is represented by an amino acid sequence given by SEQ ID NO. 1: KKKKKKKKKKKKKKKKGGGGCGGY or SEQ ID NO. 2: KKKKKKKKKKKKKKKKGGGGCGGYK* wherein K* is azidolysine, preferably 6-azido-L-lysine;
and preferably wherein the amount of the saponin molecule equivalents per amount of molecules of the polypeptide in the polyplexing agent is adjusted during the conjugating to be between 0.1 and 1, preferably between 0.15 and 0.75, more preferably between 0.2 and 0.6, and most preferably is either equal to or about 0.5.

**30.** A method of preparation of the polyplexing agent according to any one of the claims 22-25, the method comprising the step of conjugating of a polymeric scaffold with a saponin being a triterpenoid 12,13-dehydrooleanane-type saponin that in an unreacted state comprises an aldehyde group at position C-23 of the saponin's aglycone core structure,

preferably wherein the amount of the saponin molecule equivalents per amount of molecules of the polymeric scaffold in the thus obtained polyplexing agent is adjusted during the conjugating to be between 0.05 and 4, preferably between 0.1 and 2,

more preferably between 0.15 and 1, even more preferably between 0.2 and 0.75, and most preferably is either equal to or about 0.5.

# FIG. 1

## A

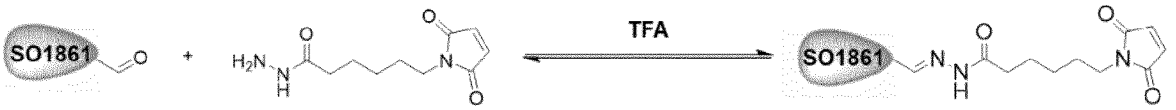


SO1861 EMCH SO1861-EMCH

## B

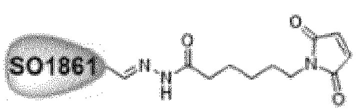

SO1861-EMCH

=

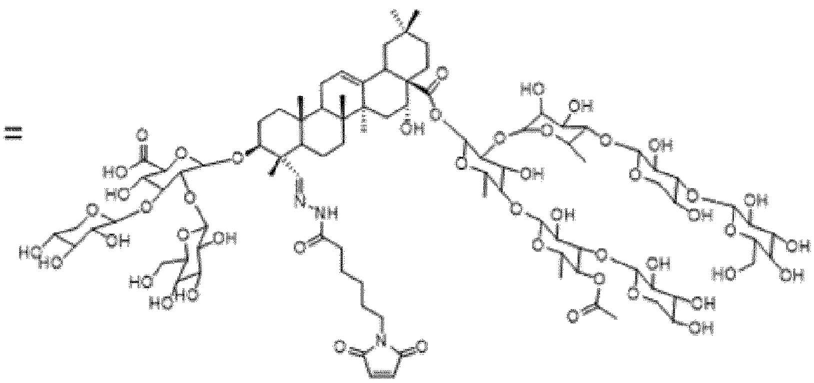

FIG. 1

C

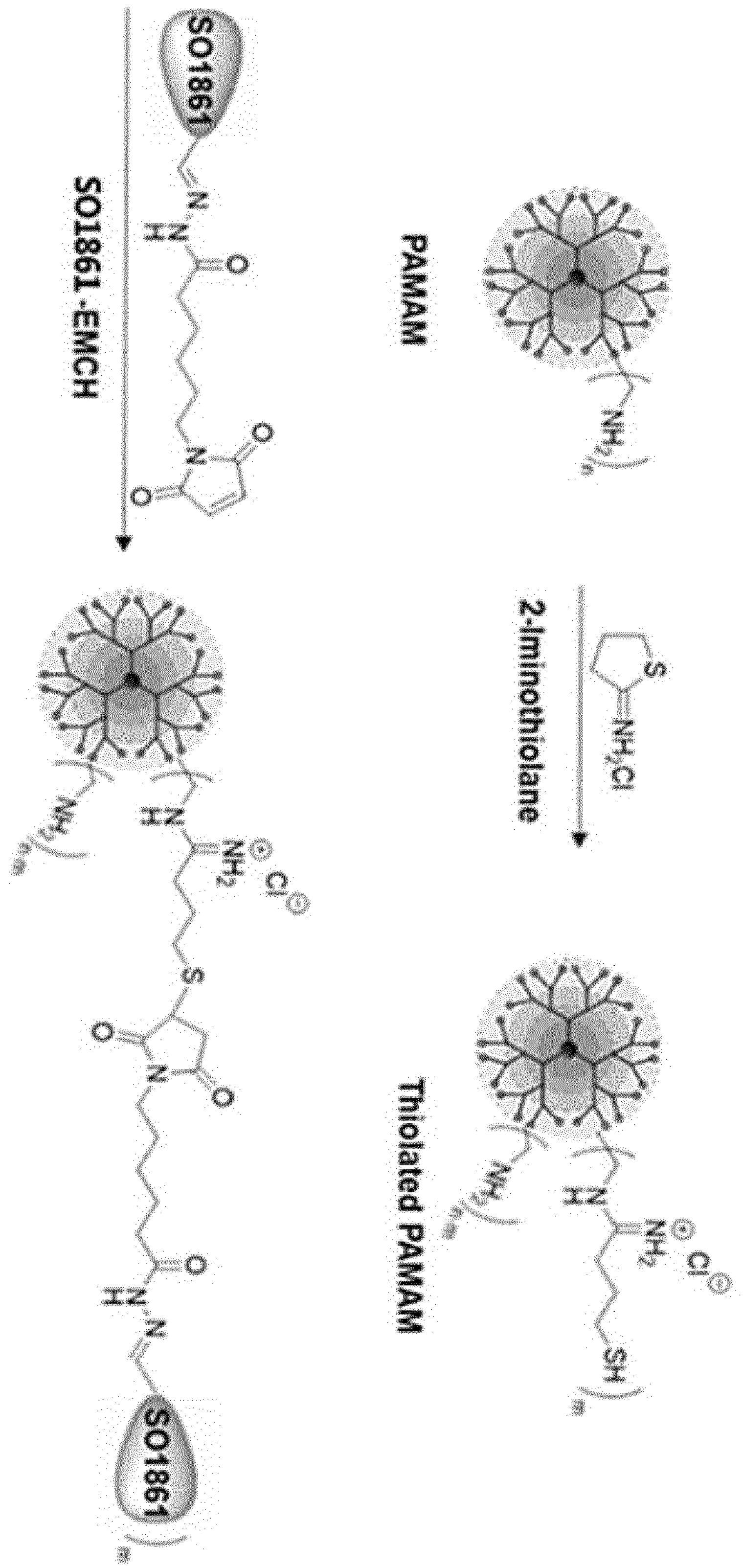
PAMAM
2-Iminothiolane
Thiolated PAMAM
SO1861-EMCH
PAMAM-SO1861

**FIG. 2**

**A**

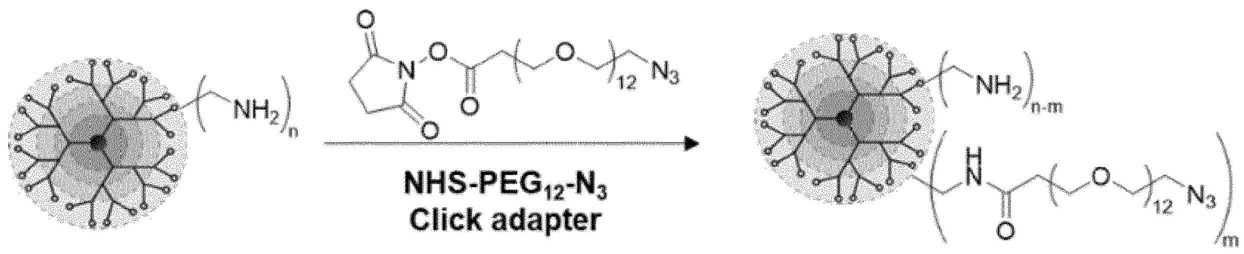


PAMAM

PAMAM with click adapter

**B**

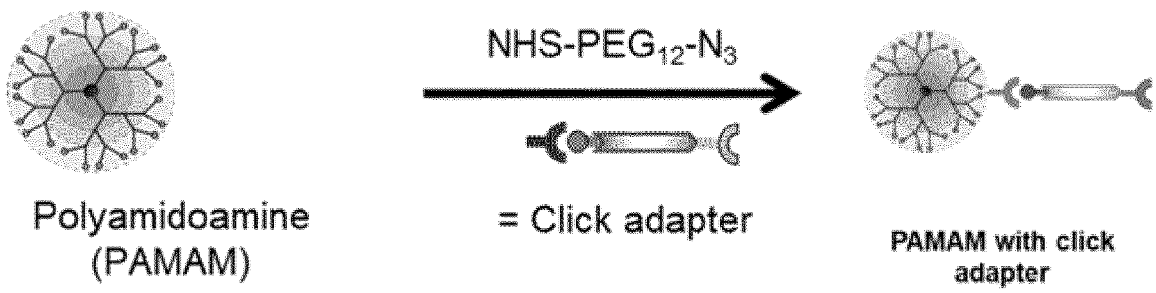

FIG. 3

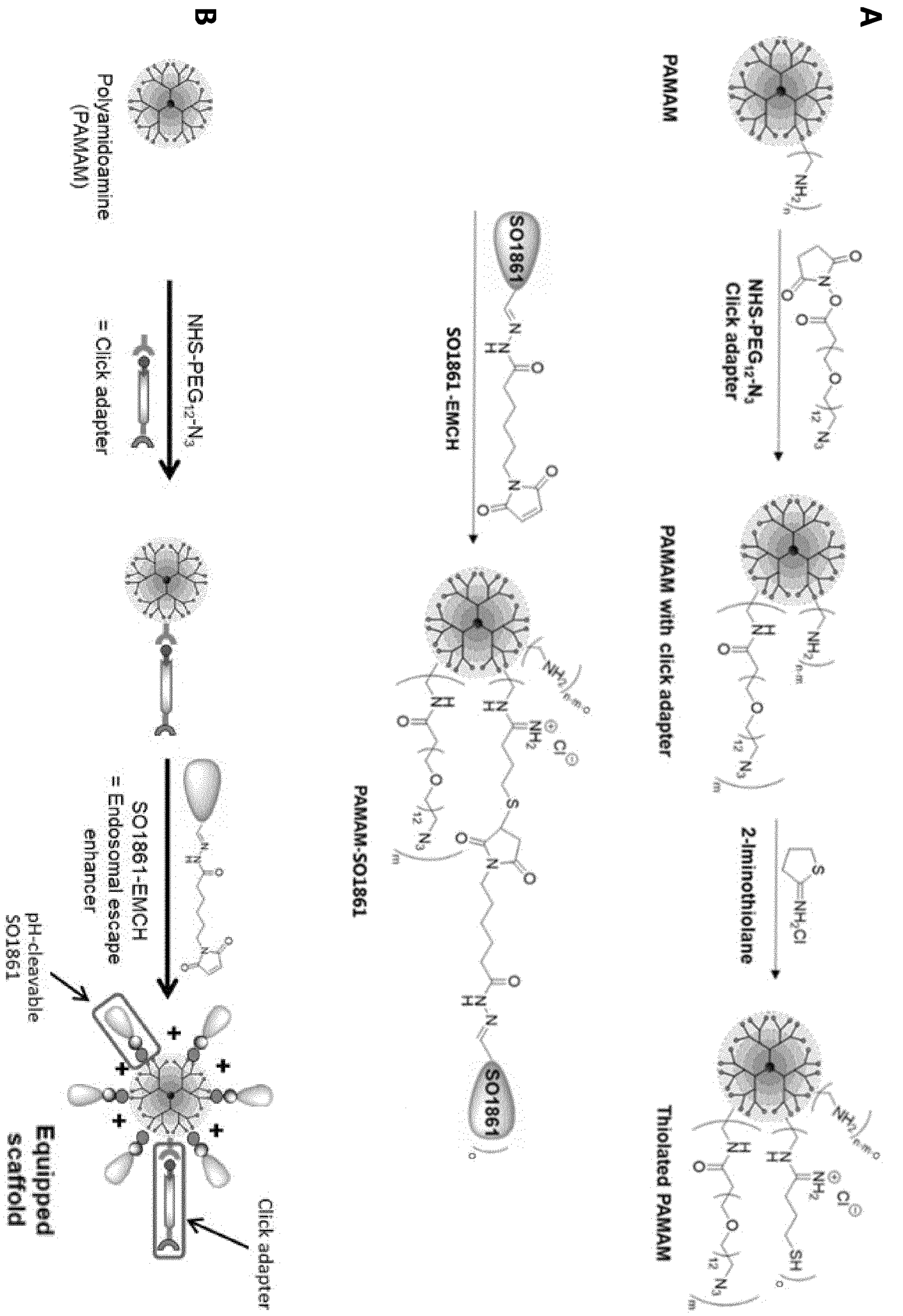
A
PAMAM
NHS-PEG$_{12}$-N$_3$
Click adapter
PAMAM with click adapter
2-Iminothiolane
Thiolated PAMAM
SO1861
SO1861-EMCH
PAMAM-SO1861
B
Polyamidoamine (PAMAM)
NHS-PEG$_{12}$-N$_3$
= Click adapter
SO1861-EMCH
= Endosomal escape enhancer
pH-cleavable SO1861
Click adapter
Equipped scaffold

# FIG. 4

## A

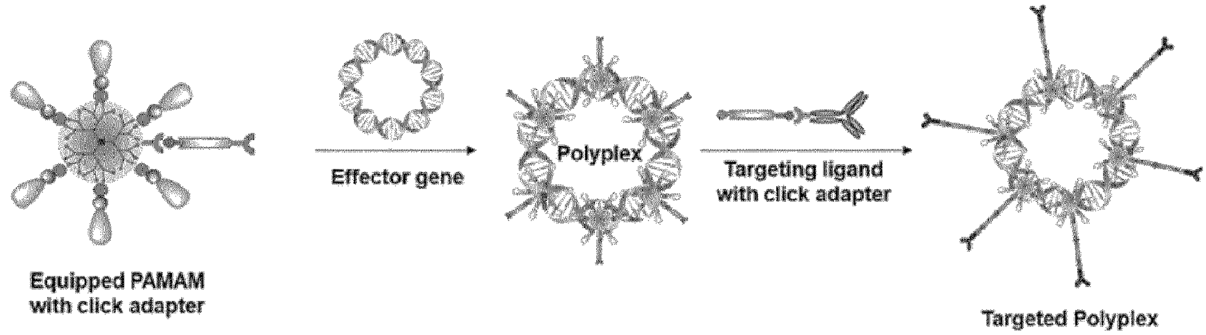

Effector gene
Polyplex
Targeting ligand
with click adapter
Equipped PAMAM
with click adapter
Targeted Polyplex


## B

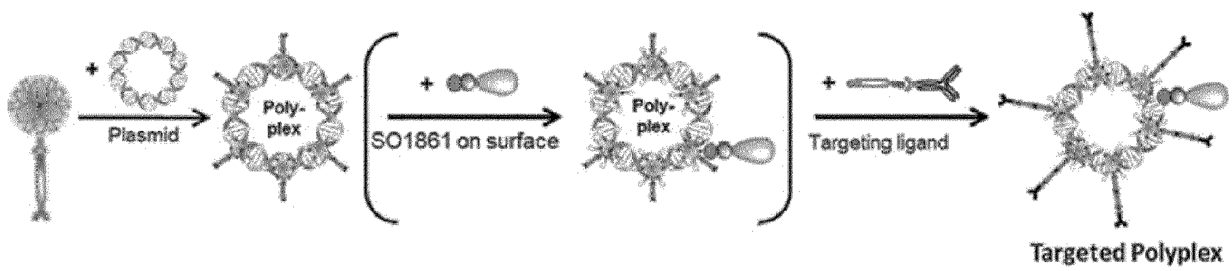

+
Plasmid
Poly-
plex
+
SO1861 on surface
Poly-
plex
+
Targeting ligand
Targeted Polyplex


## C

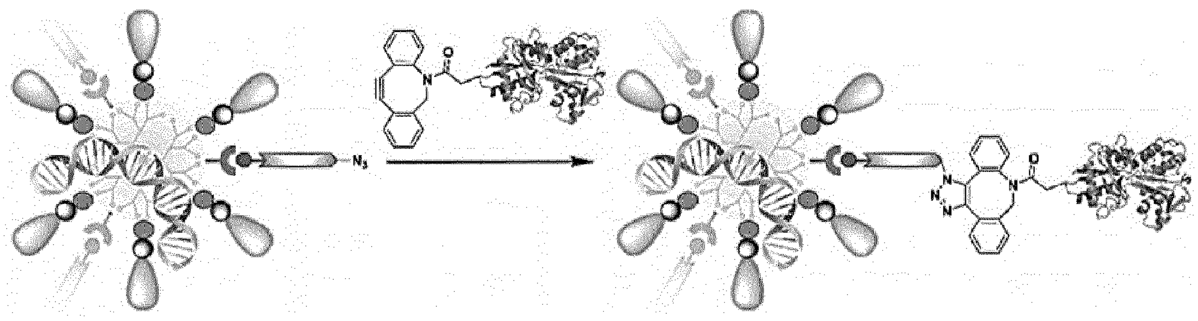

FIG. 5

A

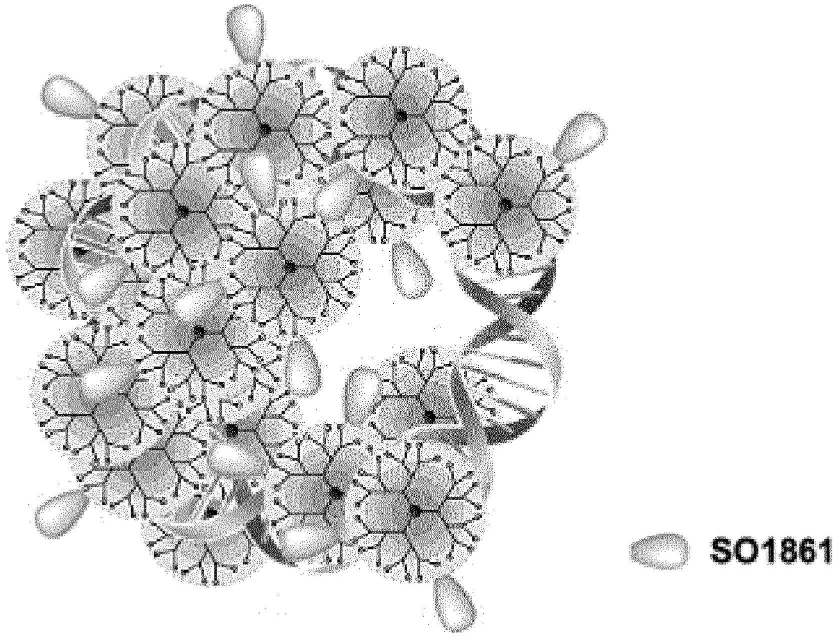


B

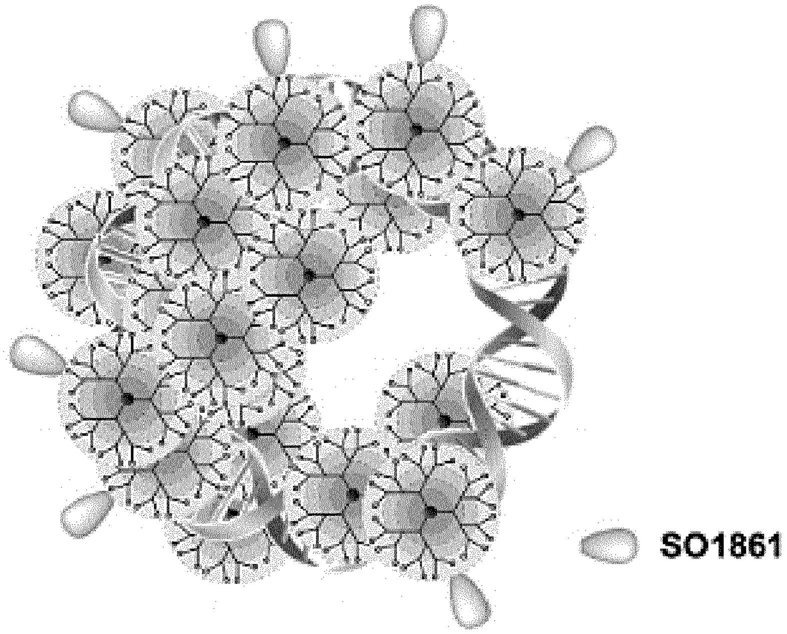

FIG. 6

A

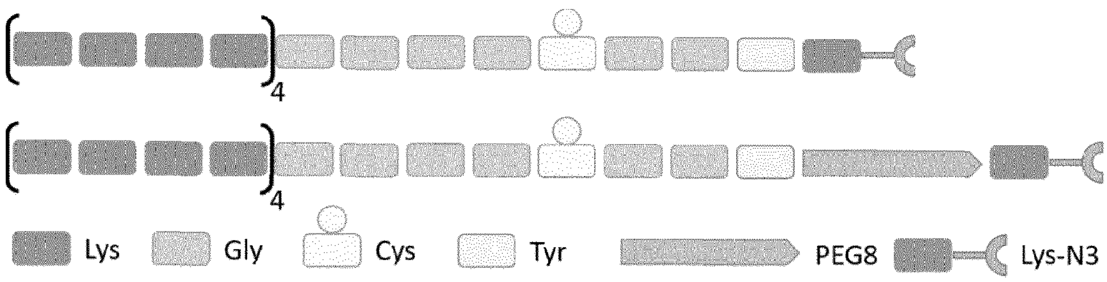
4
4
Lys
Gly
Cys
Tyr
PEG8
Lys-N3

B

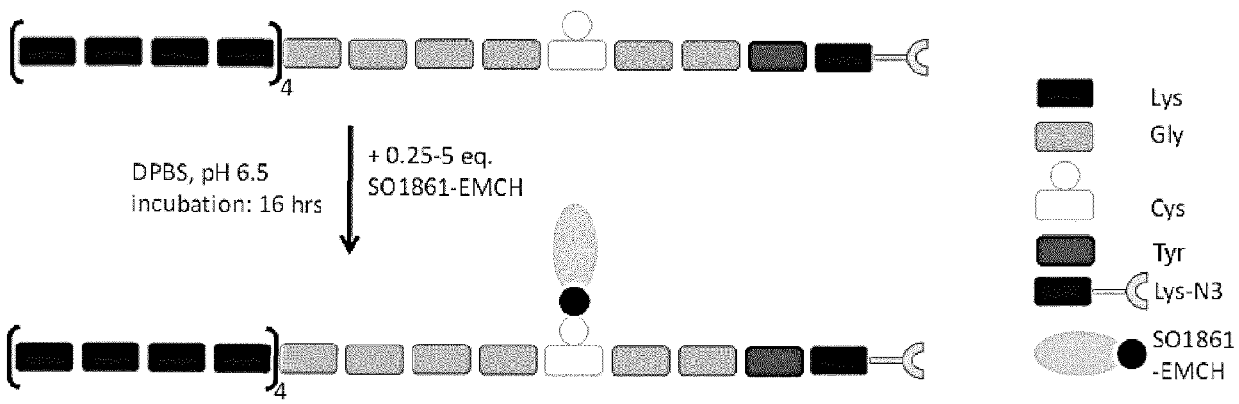
4
DPBS, pH 6.5
incubation: 16 hrs
+ 0.25-5 eq.
SO1861-EMCH
4
Lys
Gly
Cys
Tyr
Lys-N3
SO1861
-EMCH

FIG. 7

A

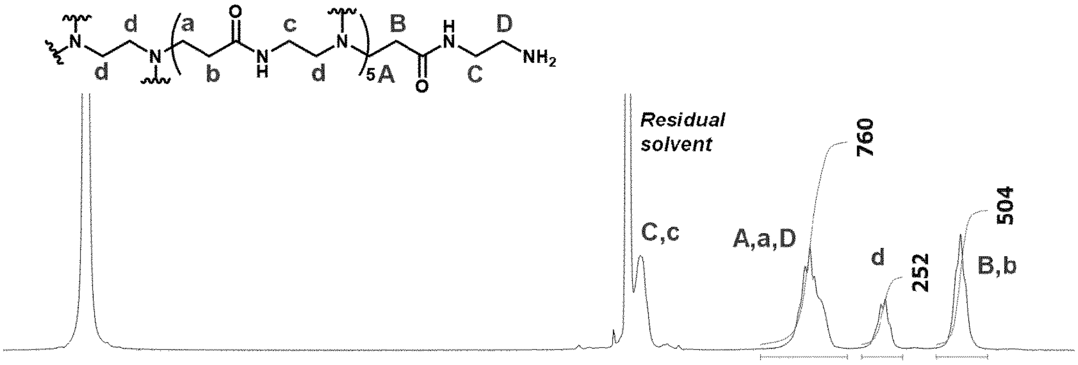
Residual solvent
C,c
A,a,D
760
d
252
504
B,b

B

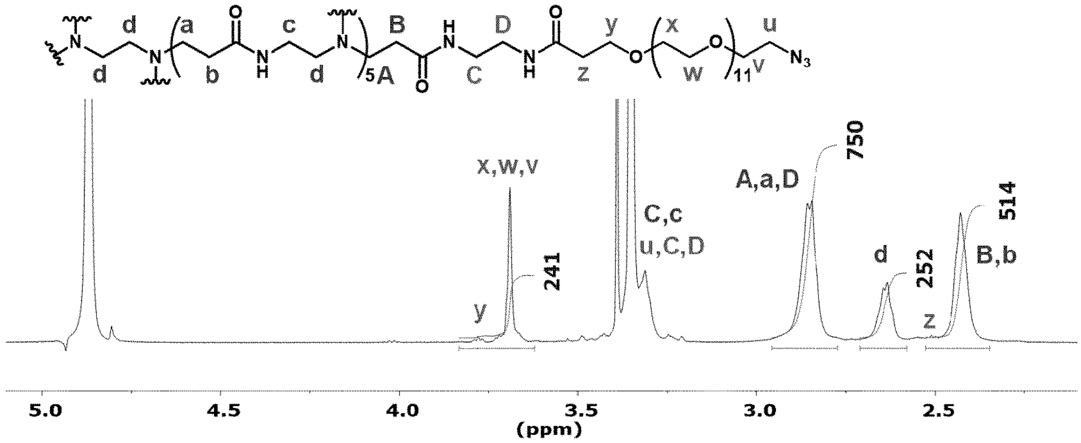
x,w,v
241
y
C,c
u,C,D
A,a,D
750
d
252
z
514
B,b
5.0
4.5
4.0
3.5
3.0
2.5
(ppm)

C

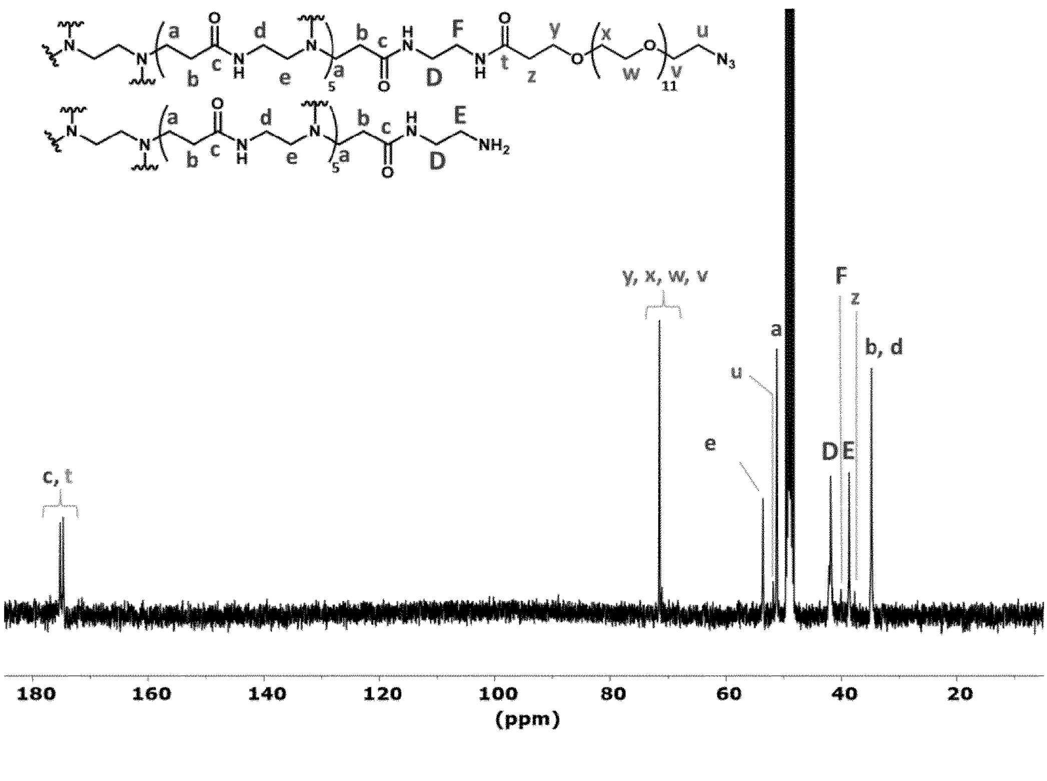
y, x, w, v
c, t
e
u
a
F
z
b, d
D E
180
160
140
120
100
80
60
40
20
(ppm)

FIG. 7

D

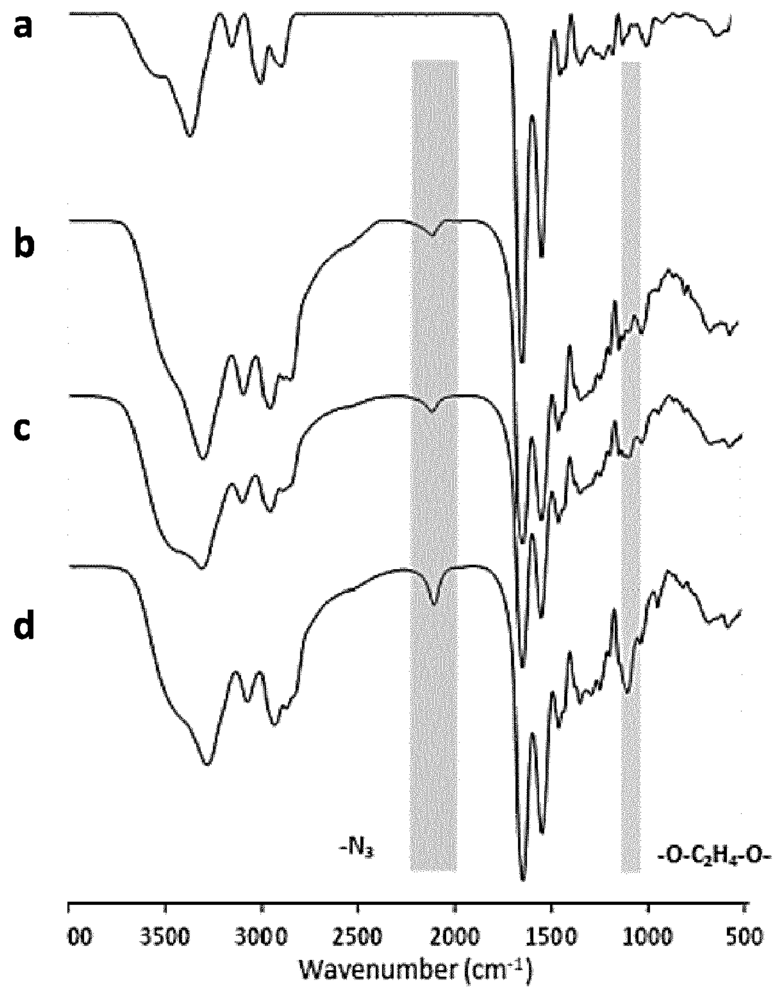
a
b
c
d
-N3
-O-C2H4-O-
00
3500
3000
2500
2000
1500
1000
500
Wavenumber (cm-1)

E

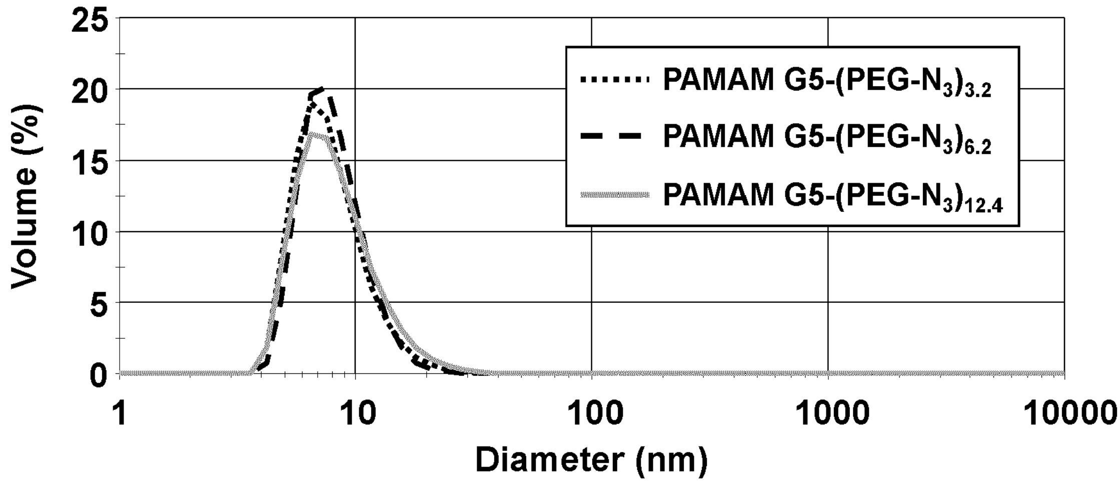
25
20
15
10
5
0
Volume (%)
PAMAM G5-(PEG-N3)3.2
PAMAM G5-(PEG-N3)6.2
PAMAM G5-(PEG-N3)12.4
1
10
100
1000
10000
Diameter (nm)

**FIG. 7**

**F**

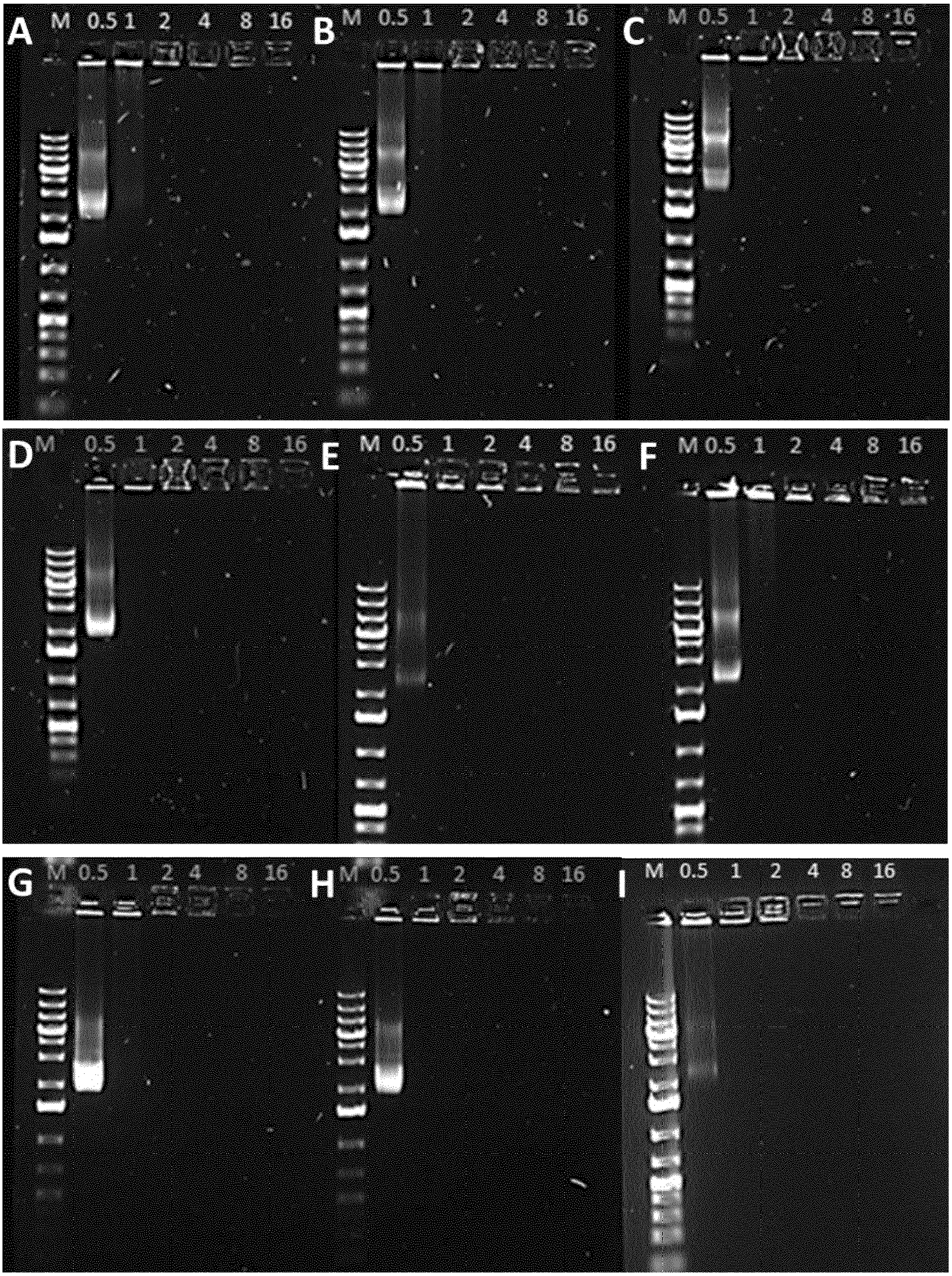
A M 0.5 1 2 4 8 16
B M 0.5 1 2 4 8 16
C M 0.5 1 2 4 8 16
D M 0.5 1 2 4 8 16
E M 0.5 1 2 4 8 16
F M 0.5 1 2 4 8 16
G M 0.5 1 2 4 8 16
H M 0.5 1 2 4 8 16
I M 0.5 1 2 4 8 16

**FIG. 7**

**G**

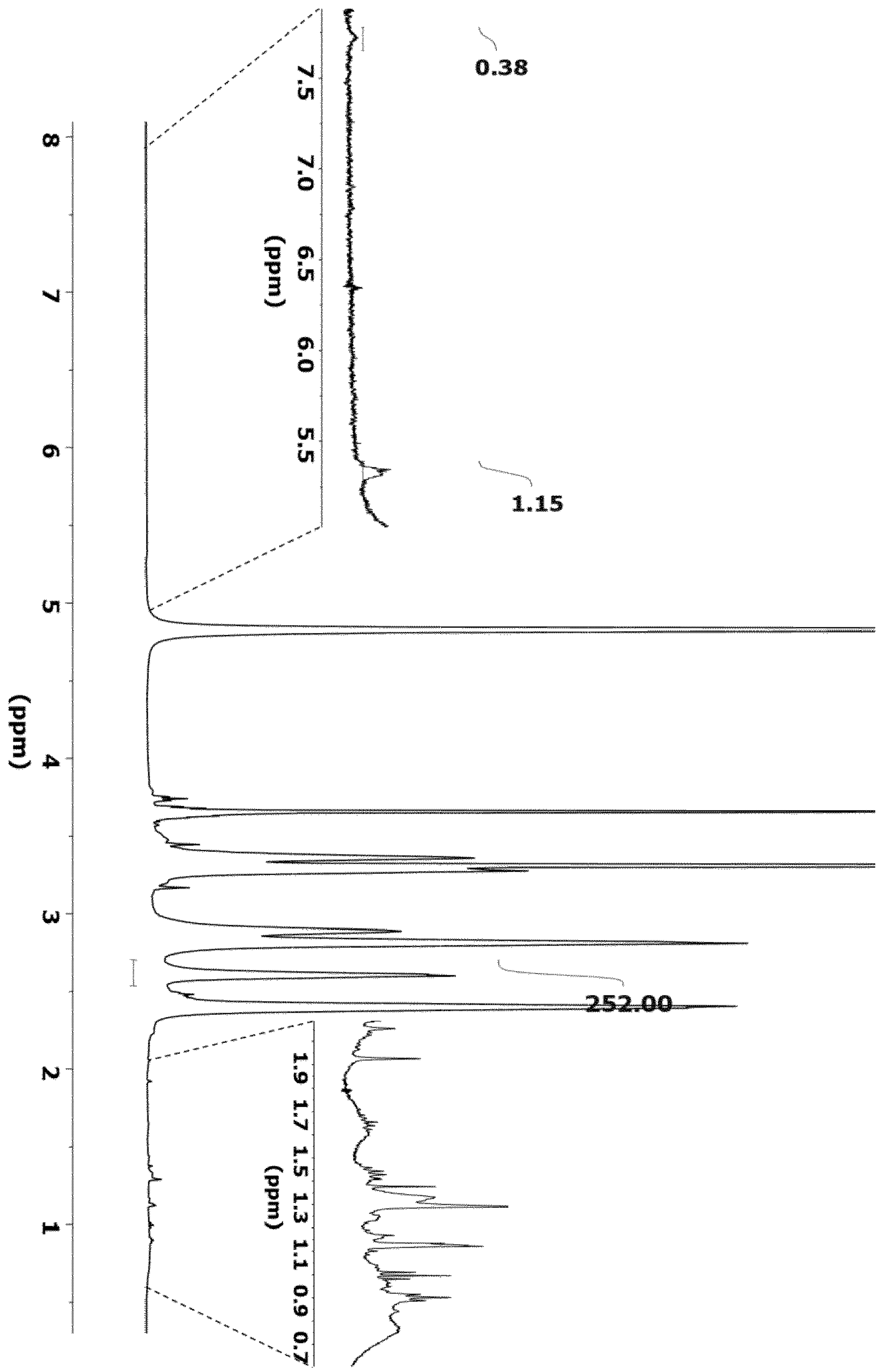

H

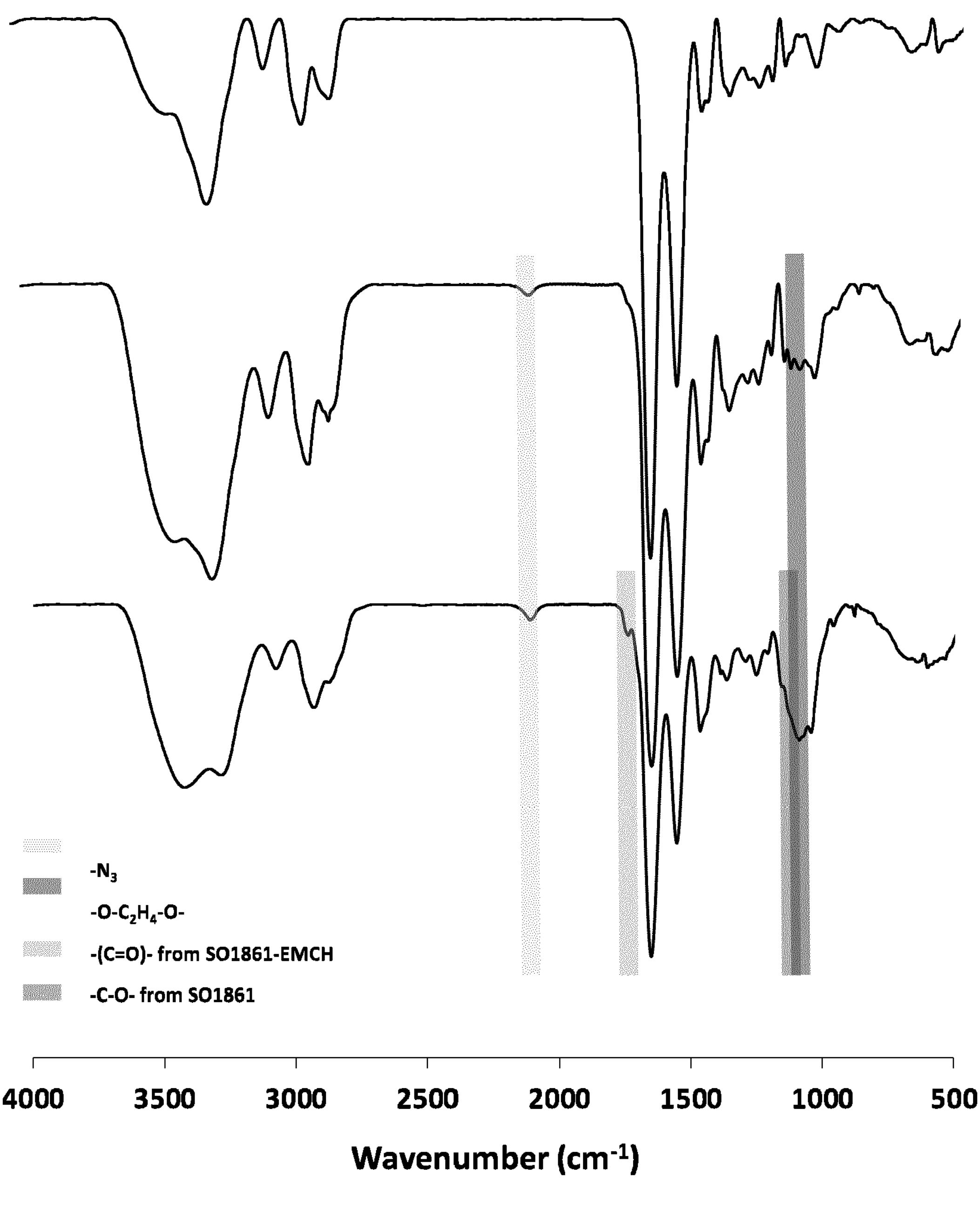
a
b
c
-N3
-O-C2H4-O-
-(C=O)- from SO1861-EMCH
-C-O- from SO1861
4000
3500
3000
2500
2000
1500
1000
500
Wavenumber (cm-1)

FIG. 7

I

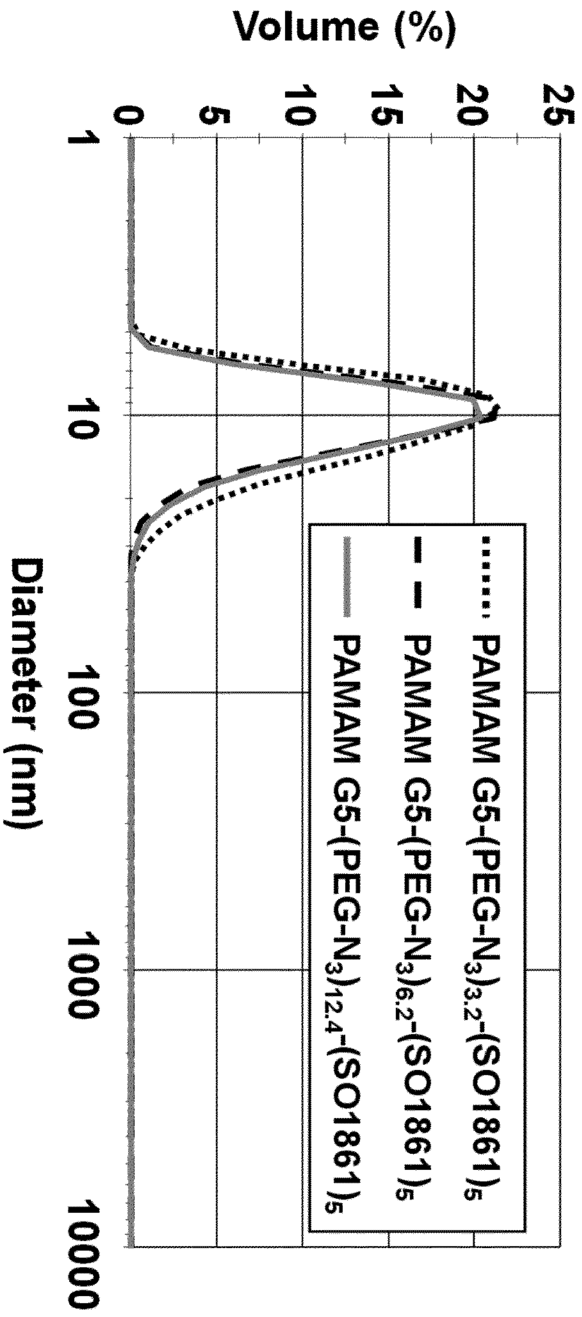
Volume (%)
0
5
10
15
20
25
1
10
100
1000
10000
Diameter (nm)
PAMAM G5-(PEG-N3)3.2-(SO1861)5
PAMAM G5-(PEG-N3)6.2-(SO1861)5
PAMAM G5-(PEG-N3)12.4-(SO1861)5

**FIG. 8**

**A**

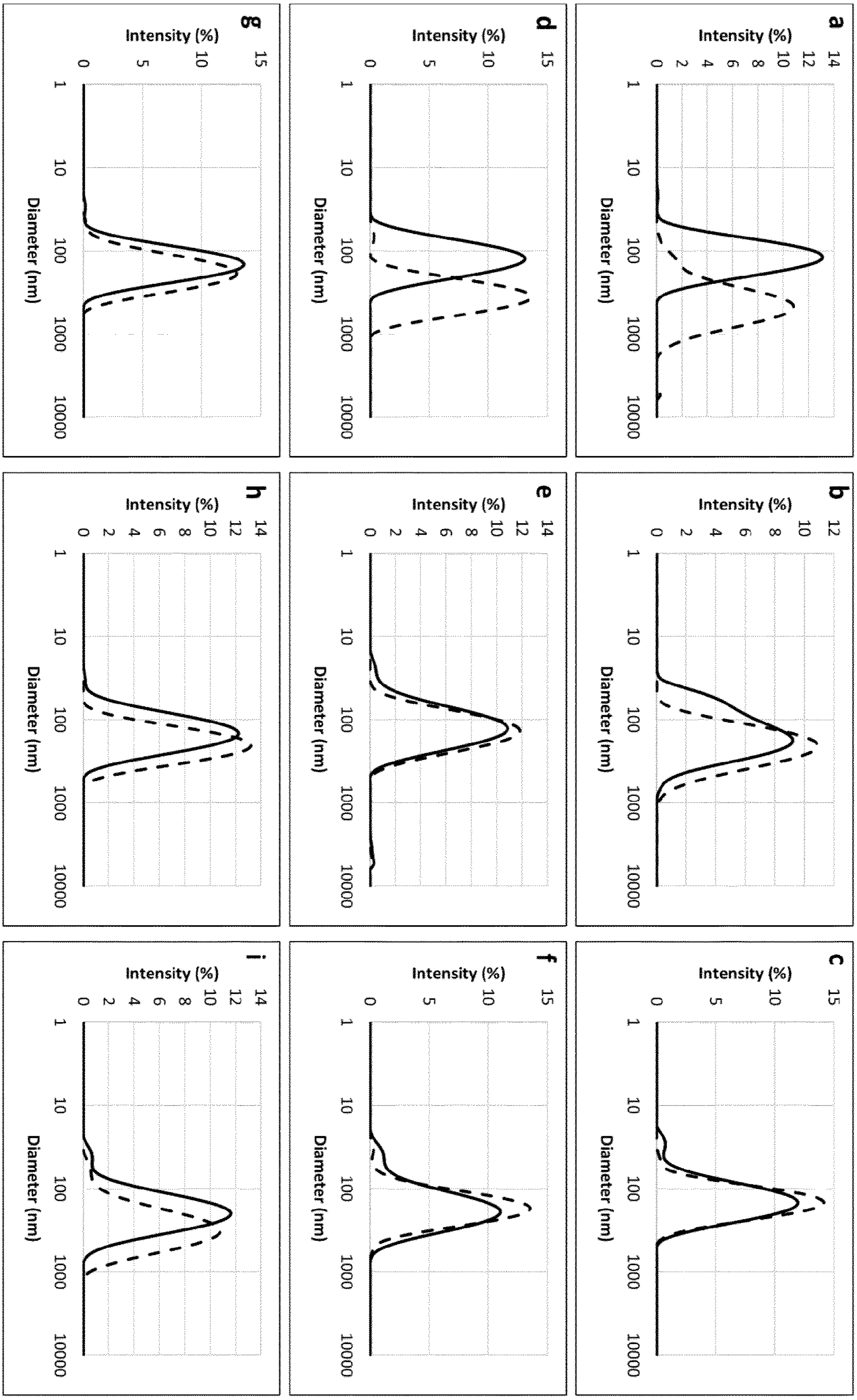

**B**

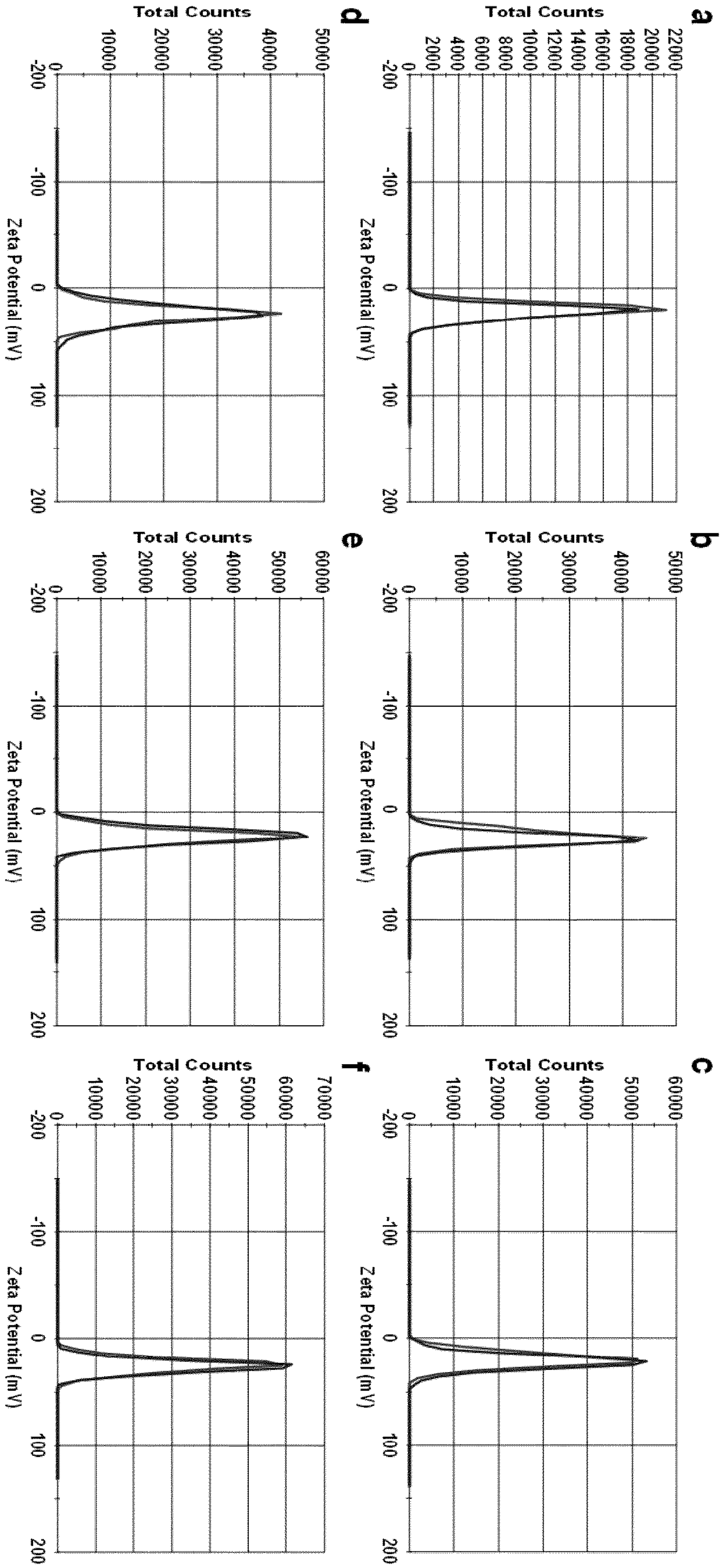

C

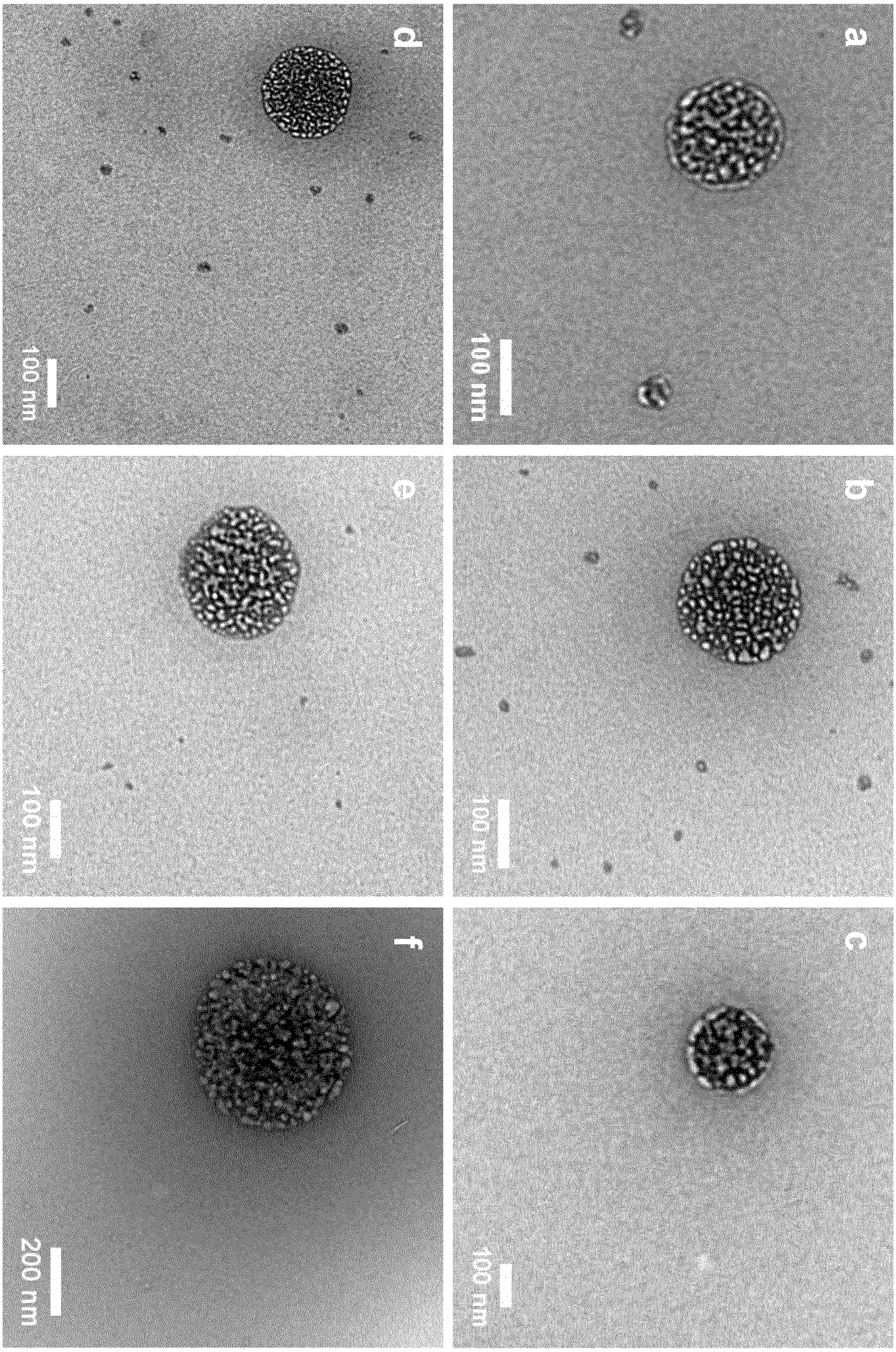

**FIG. 9**

**A**

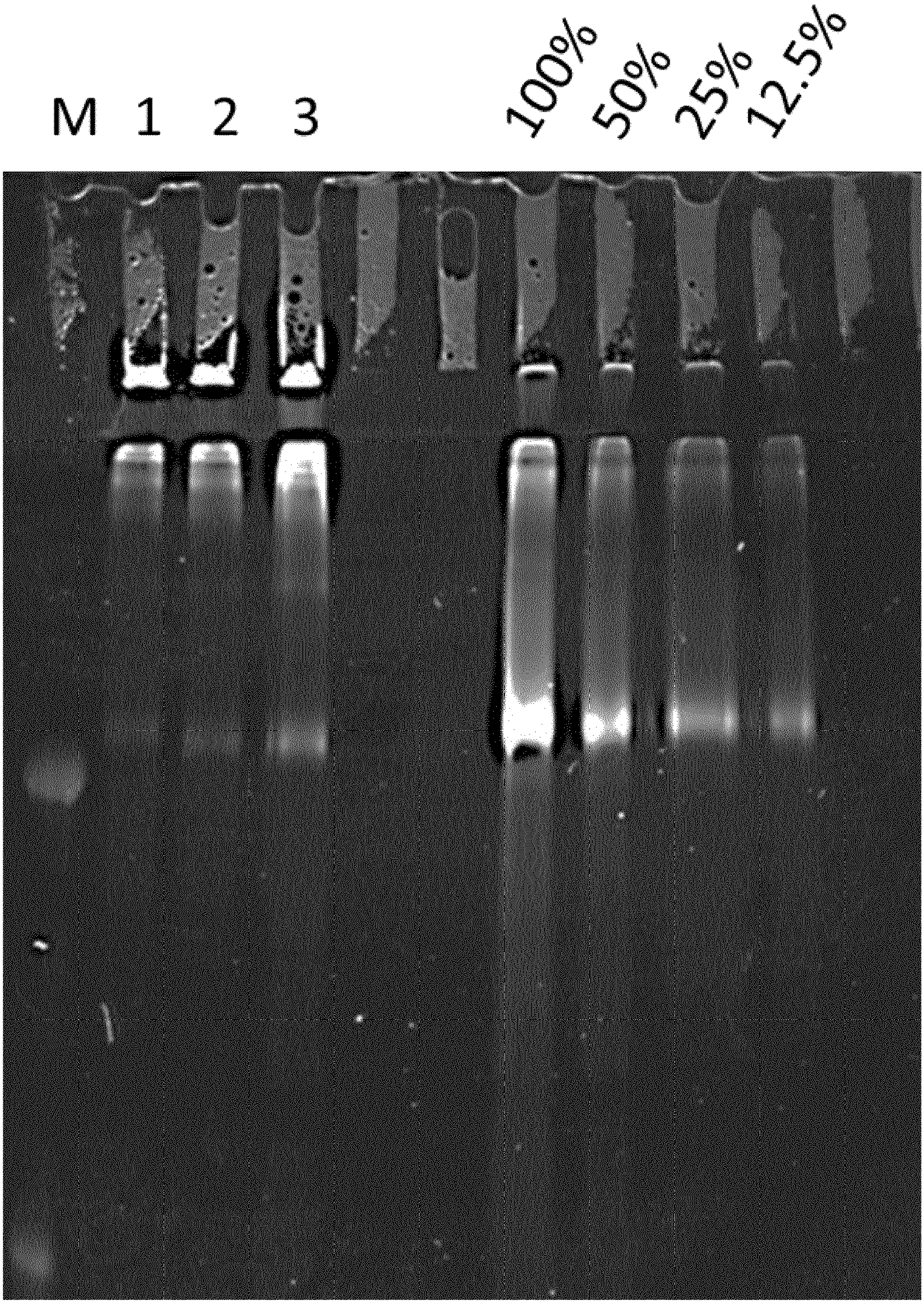

**Fig. 9**

**B**

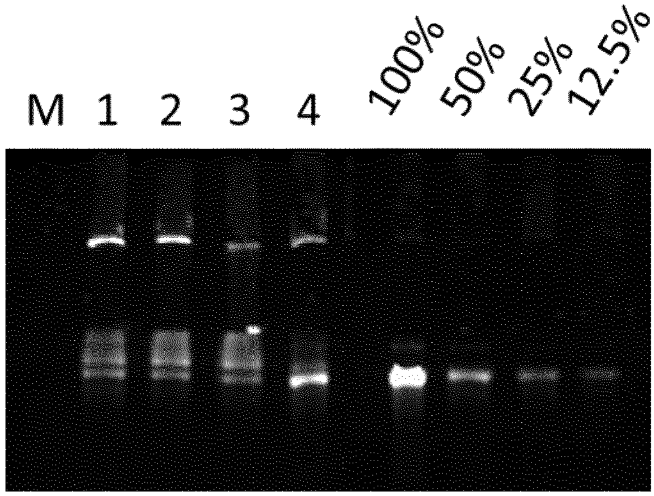


**C**

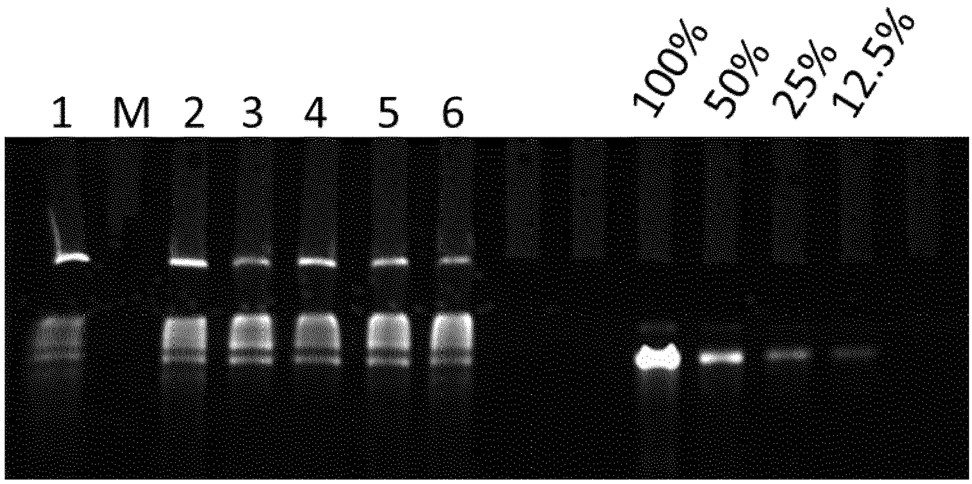

**Fig. 10**

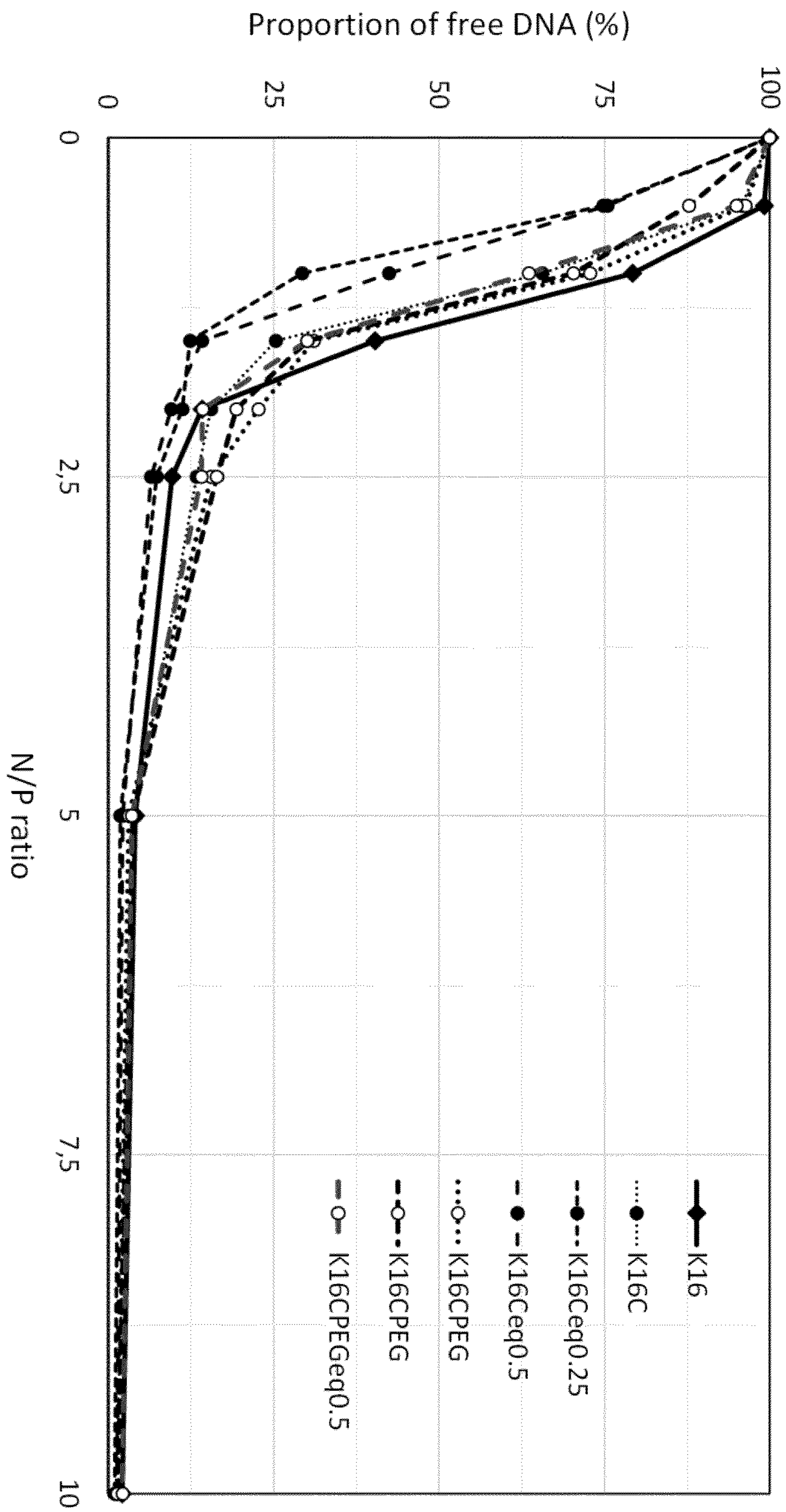
Proportion of free DNA (%)
0
25
50
75
100
N/P ratio
0
2,5
5
7,5
10
K16
K16C
K16Ceq0.25
K16Ceq0.5
K16CPEG
K16CPEG
K16CPEGeq0.5

**FIG. 11**

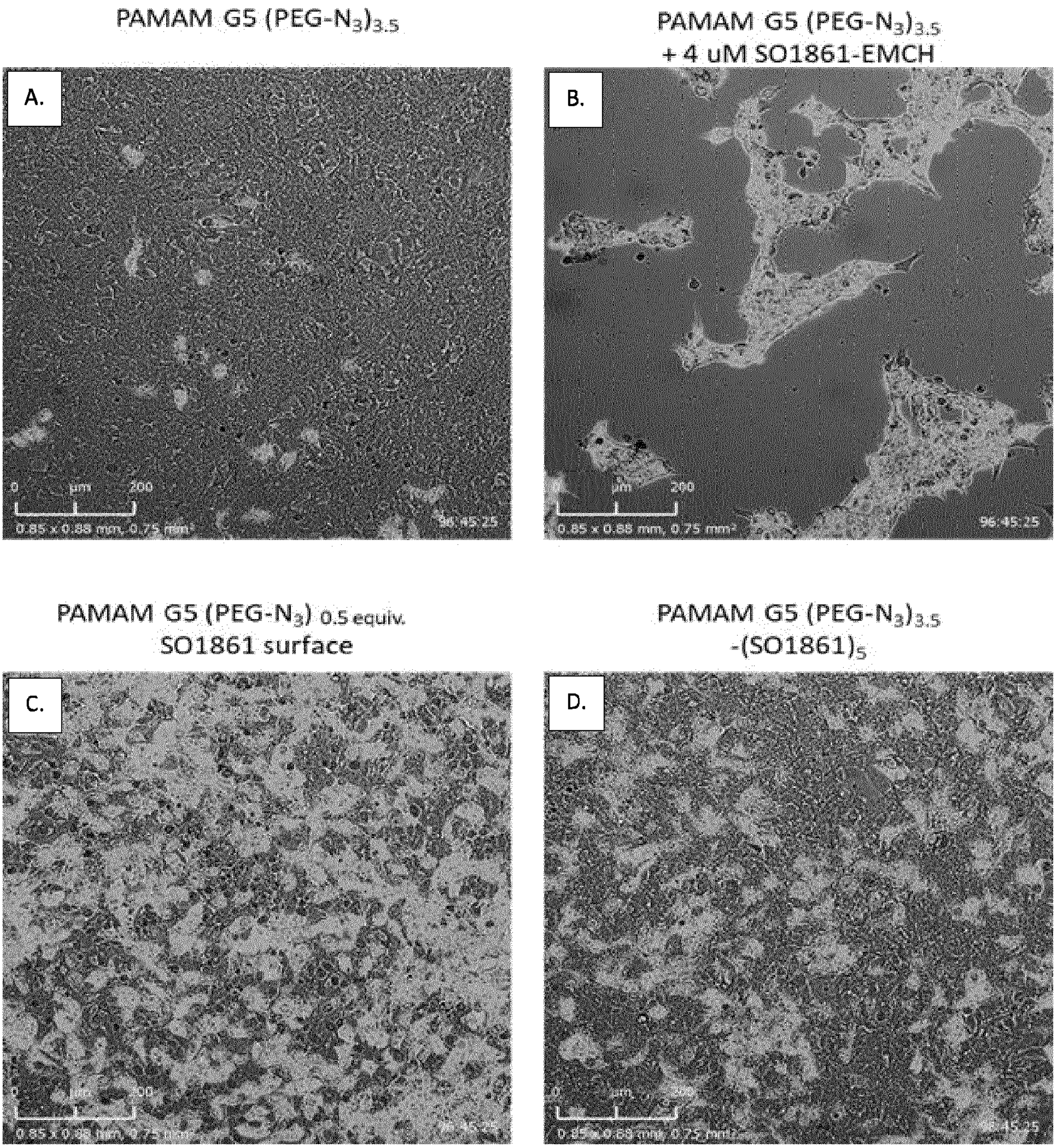
PAMAM G5 (PEG-$N_3$)$_{3.5}$
A.
0 µm 200
0.85 x 0.88 mm, 0.75 mm²
96:45:25
PAMAM G5 (PEG-$N_3$)$_{3.5}$
+ 4 uM SO1861-EMCH
B.
0 µm 200
0.85 x 0.88 mm, 0.75 mm²
96:45:25
PAMAM G5 (PEG-$N_3$) 0.5 equiv.
SO1861 surface
C.
PAMAM G5 (PEG-$N_3$)$_{3.5}$
-(SO1861)$_5$
D.

**FIG. 11**

**E**

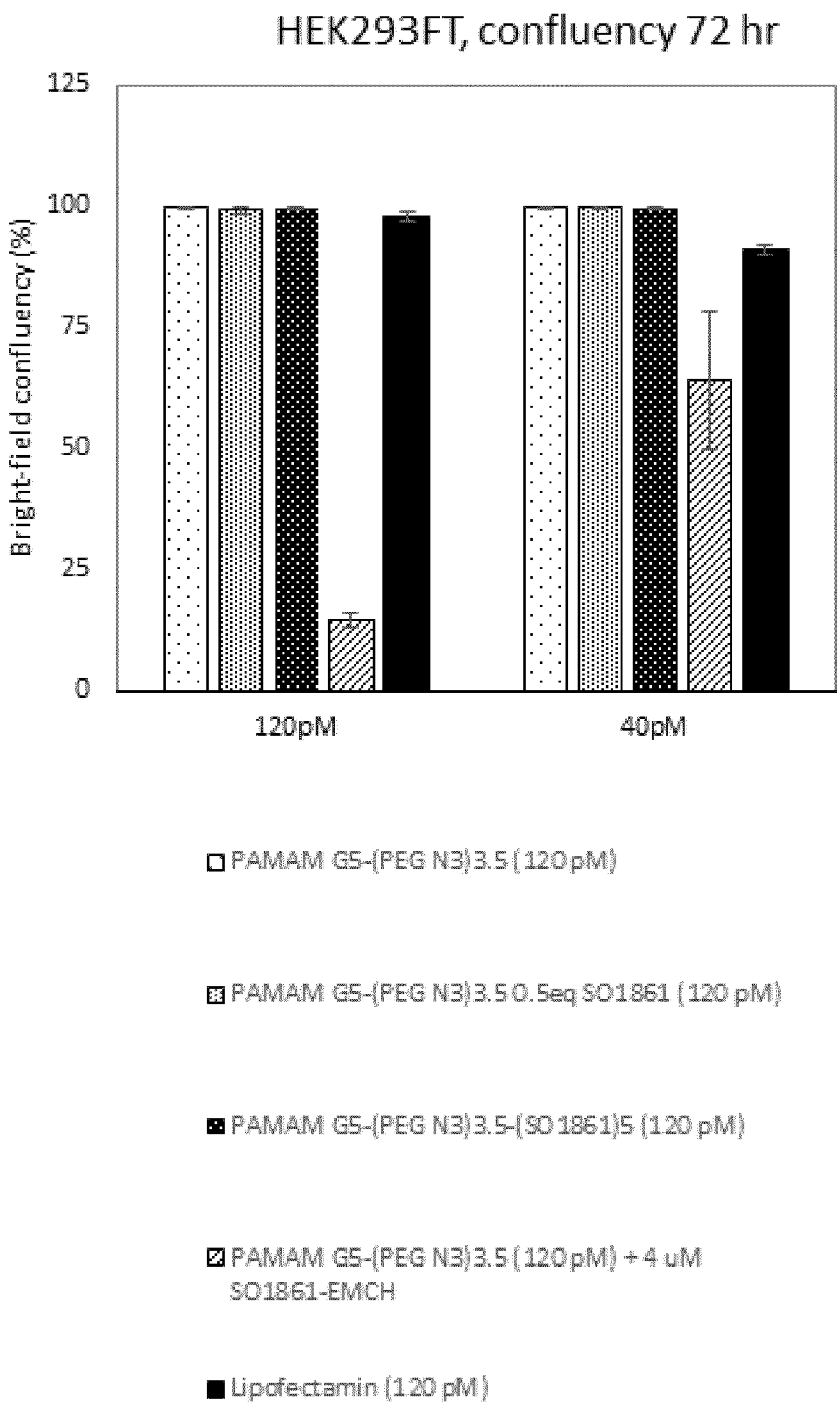
HEK293FT, confluency 72 hr
Bright-field confluency (%)
125
100
75
50
25
0
120pM
40pM
PAMAM G5-(PEG N3)3.5 (120 pM)
PAMAM G5-(PEG N3)3.5 0.5eq SO1861 (120 pM)
PAMAM G5-(PEG N3)3.5-(SO1861)5 (120 pM)
PAMAM G5-(PEG N3)3.5 (120 pM) + 4 uM SO1861-EMCH
Lipofectamin (120 pM)

**FIG. 11**

**F**

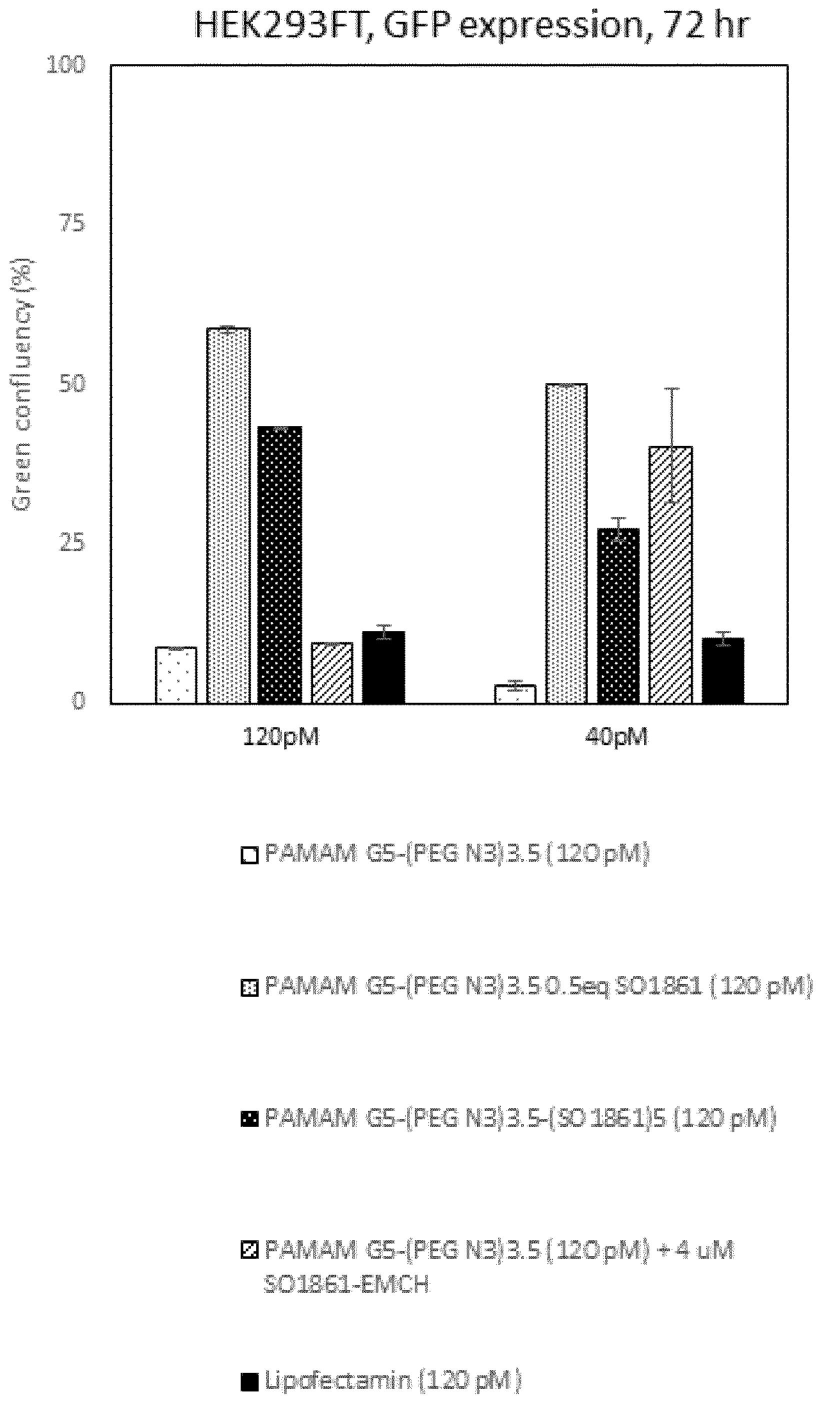
HEK293FT, GFP expression, 72 hr
Green confluency (%)
100
75
50
25
0
120pM
40pM
PAMAM G5-(PEG N3)3.5 (120 pM)
PAMAM G5-(PEG N3)3.5 0.5eq SO1861 (120 pM)
PAMAM G5-(PEG N3)3.5-(SO 1861)5 (120 pM)
PAMAM G5-(PEG N3)3.5 (120 pM) + 4 uM SO1861-EMCH
Lipofectamin (120 pM)

FIG. 12

A

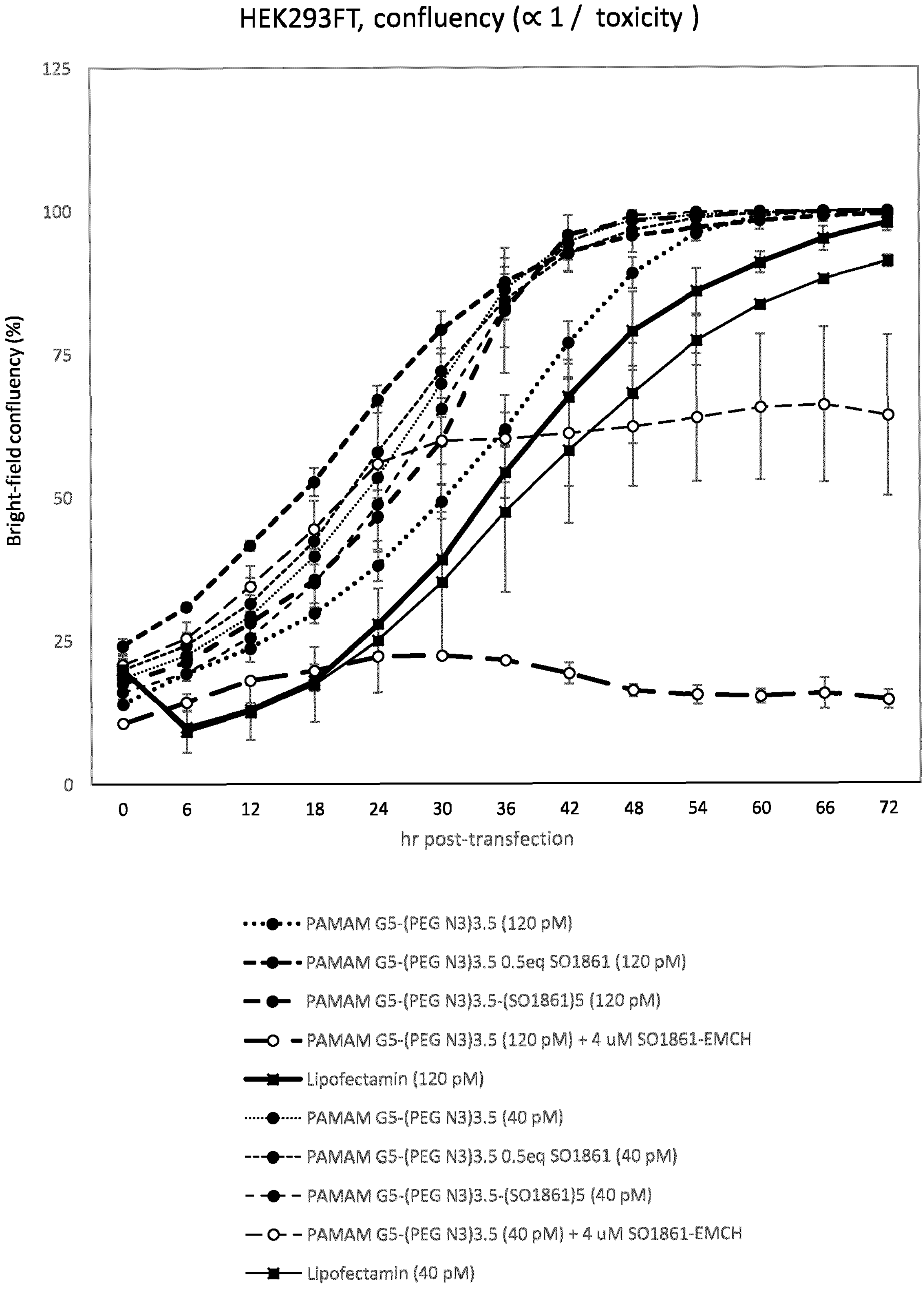
HEK293FT, confluency (∝ 1 / toxicity )
Bright-field confluency (%)
125
100
75
50
25
0
0
6
12
18
24
30
36
42
48
54
60
66
72
hr post-transfection
PAMAM G5-(PEG N3)3.5 (120 pM)
PAMAM G5-(PEG N3)3.5 0.5eq SO1861 (120 pM)
PAMAM G5-(PEG N3)3.5-(SO1861)5 (120 pM)
PAMAM G5-(PEG N3)3.5 (120 pM) + 4 uM SO1861-EMCH
Lipofectamin (120 pM)
PAMAM G5-(PEG N3)3.5 (40 pM)
PAMAM G5-(PEG N3)3.5 0.5eq SO1861 (40 pM)
PAMAM G5-(PEG N3)3.5-(SO1861)5 (40 pM)
PAMAM G5-(PEG N3)3.5 (40 pM) + 4 uM SO1861-EMCH
Lipofectamin (40 pM)

# FIG. 12

## B

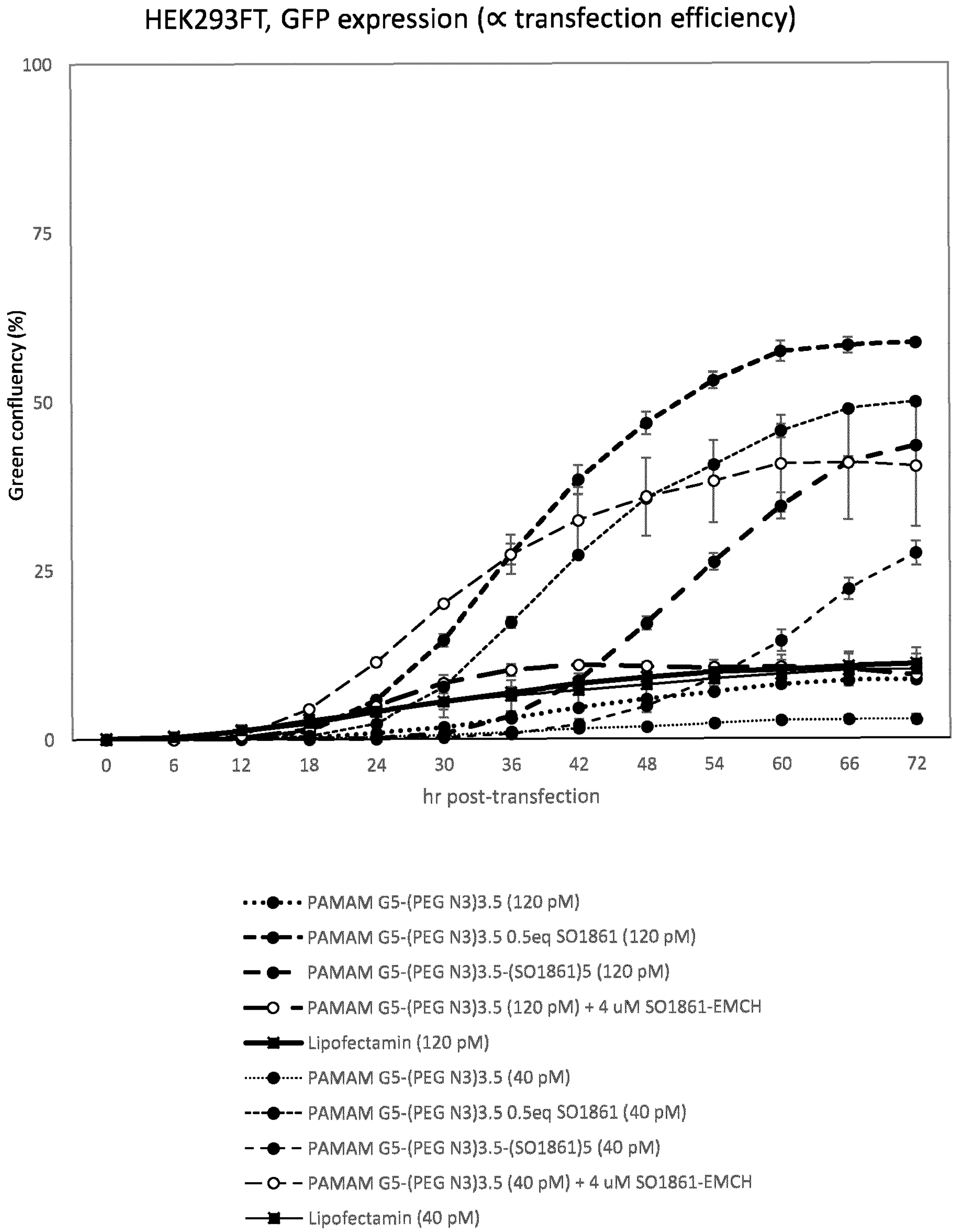
HEK293FT, GFP expression (∝ transfection efficiency)
Green confluency (%)
100
75
50
25
0
0
6
12
18
24
30
36
42
48
54
60
66
72
hr post-transfection
PAMAM G5-(PEG N3)3.5 (120 pM)
PAMAM G5-(PEG N3)3.5 0.5eq SO1861 (120 pM)
PAMAM G5-(PEG N3)3.5-(SO1861)5 (120 pM)
PAMAM G5-(PEG N3)3.5 (120 pM) + 4 uM SO1861-EMCH
Lipofectamin (120 pM)
PAMAM G5-(PEG N3)3.5 (40 pM)
PAMAM G5-(PEG N3)3.5 0.5eq SO1861 (40 pM)
PAMAM G5-(PEG N3)3.5-(SO1861)5 (40 pM)
PAMAM G5-(PEG N3)3.5 (40 pM) + 4 uM SO1861-EMCH
Lipofectamin (40 pM)

**FIG. 12**

**C**

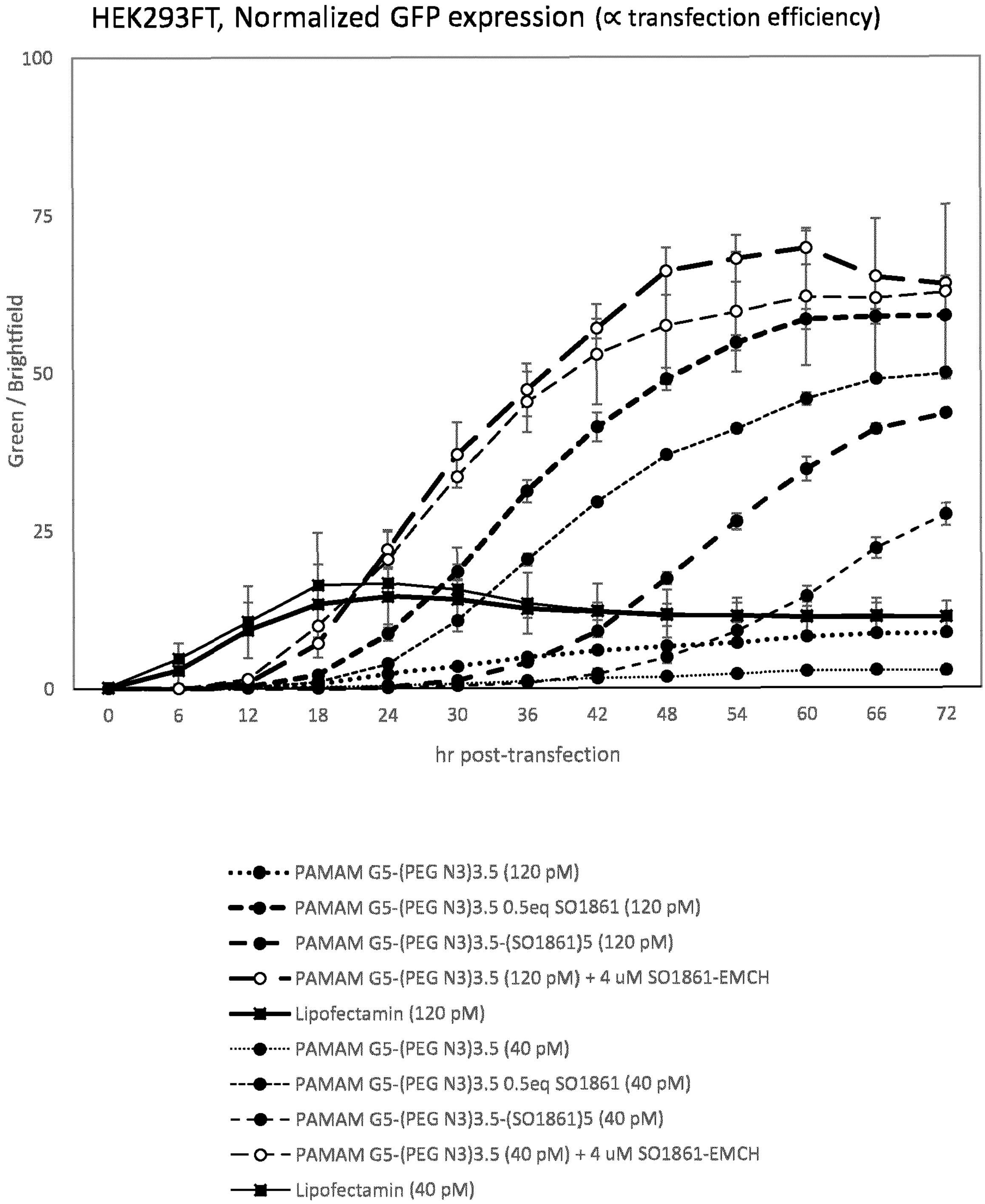
HEK293FT, Normalized GFP expression (∝ transfection efficiency)
100
75
50
25
0
Green / Brightfield
0
6
12
18
24
30
36
42
48
54
60
66
72
hr post-transfection
PAMAM G5-(PEG N3)3.5 (120 pM)
PAMAM G5-(PEG N3)3.5 0.5eq SO1861 (120 pM)
PAMAM G5-(PEG N3)3.5-(SO1861)5 (120 pM)
PAMAM G5-(PEG N3)3.5 (120 pM) + 4 uM SO1861-EMCH
Lipofectamin (120 pM)
PAMAM G5-(PEG N3)3.5 (40 pM)
PAMAM G5-(PEG N3)3.5 0.5eq SO1861 (40 pM)
PAMAM G5-(PEG N3)3.5-(SO1861)5 (40 pM)
PAMAM G5-(PEG N3)3.5 (40 pM) + 4 uM SO1861-EMCH
Lipofectamin (40 pM)

**FIG. 13**

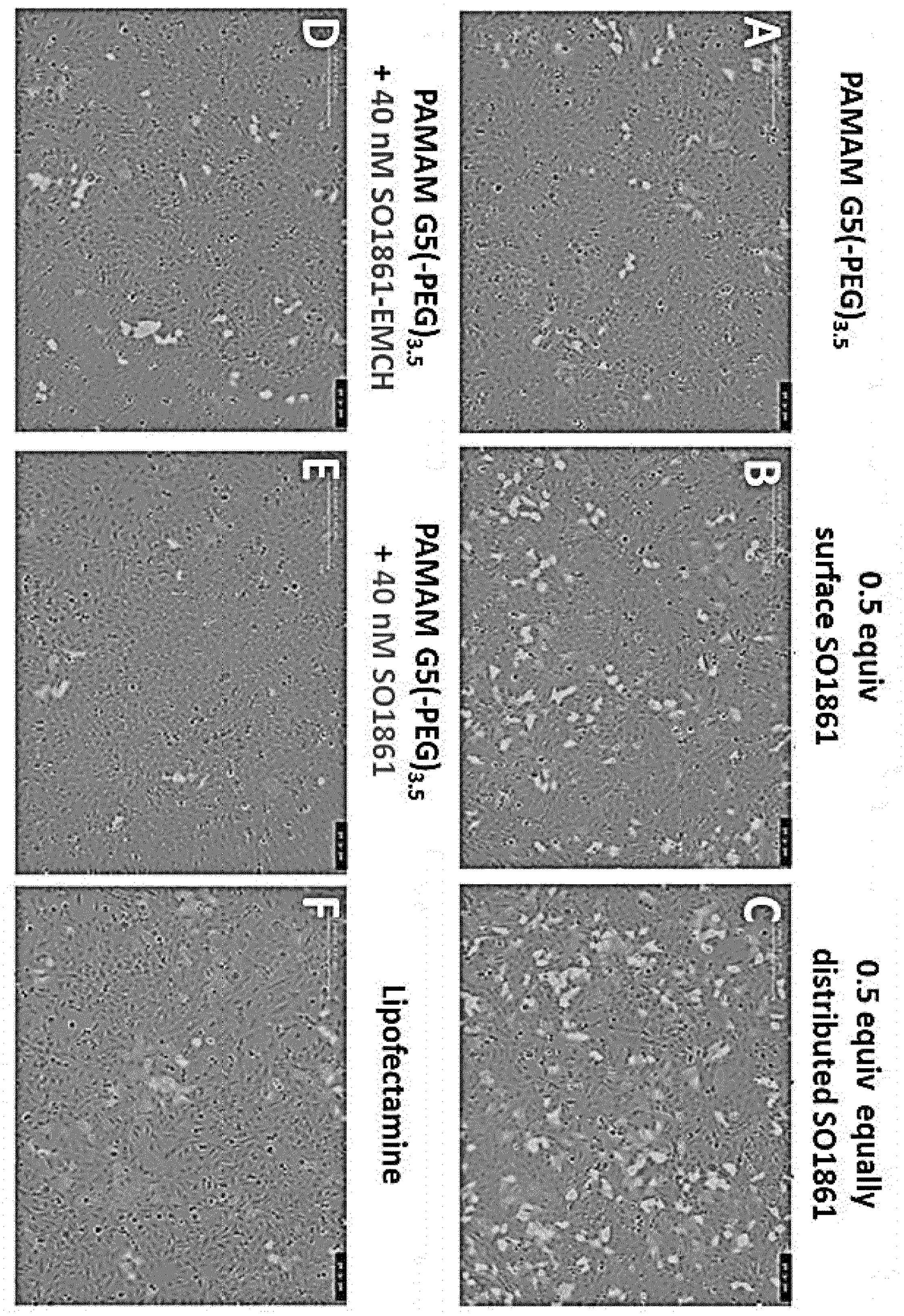
A
PAMAM G5(-PEG)3.5
B
0.5 equiv
surface SO1861
C
0.5 equiv equally
distributed SO1861
D
PAMAM G5(-PEG)3.5
+ 40 nM SO1861-EMCH
E
PAMAM G5(-PEG)3.5
+ 40 nM SO1861
F
Lipofectamine

FIG. 14

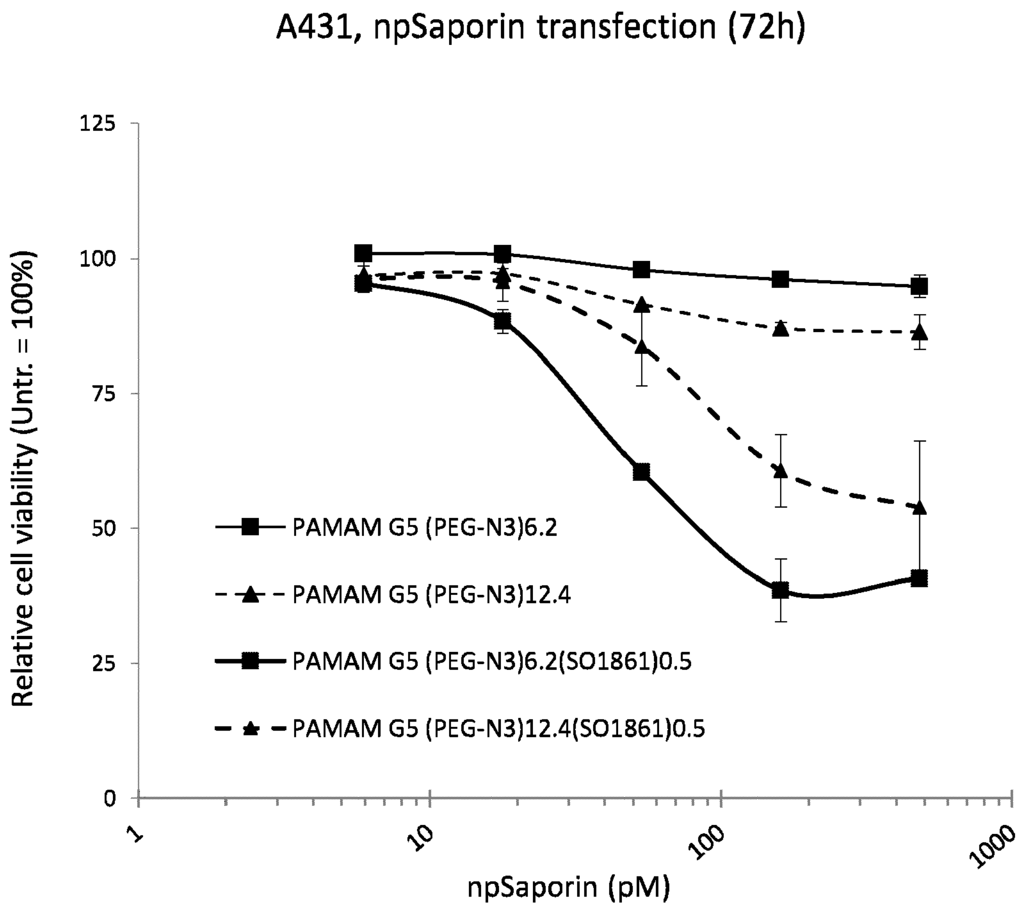
A431, npSaporin transfection (72h)
Relative cell viability (Untr. = 100%)
125
100
75
50
25
0
1
10
100
1000
npSaporin (pM)
PAMAM G5 (PEG-N3)6.2
PAMAM G5 (PEG-N3)12.4
PAMAM G5 (PEG-N3)6.2(SO1861)0.5
PAMAM G5 (PEG-N3)12.4(SO1861)0.5

**FIG. 15**

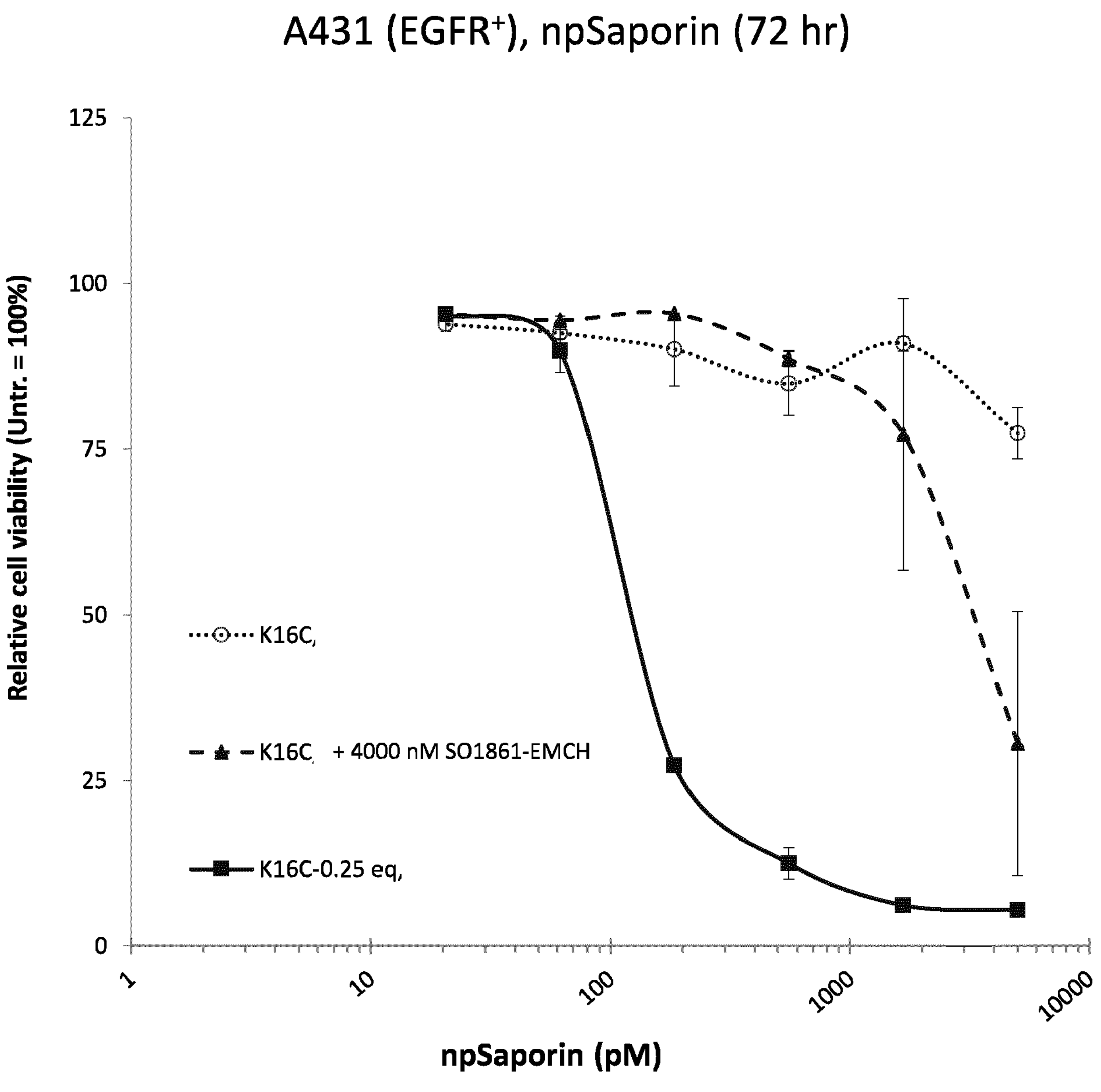
A431 (EGFR+), npSaporin (72 hr)
125
100
75
50
25
0
Relative cell viability (Untr. = 100%)
K16C,
K16C, + 4000 nM SO1861-EMCH
K16C-0.25 eq,
1
10
100
1000
10000
npSaporin (pM)

# FIG. 16

## A

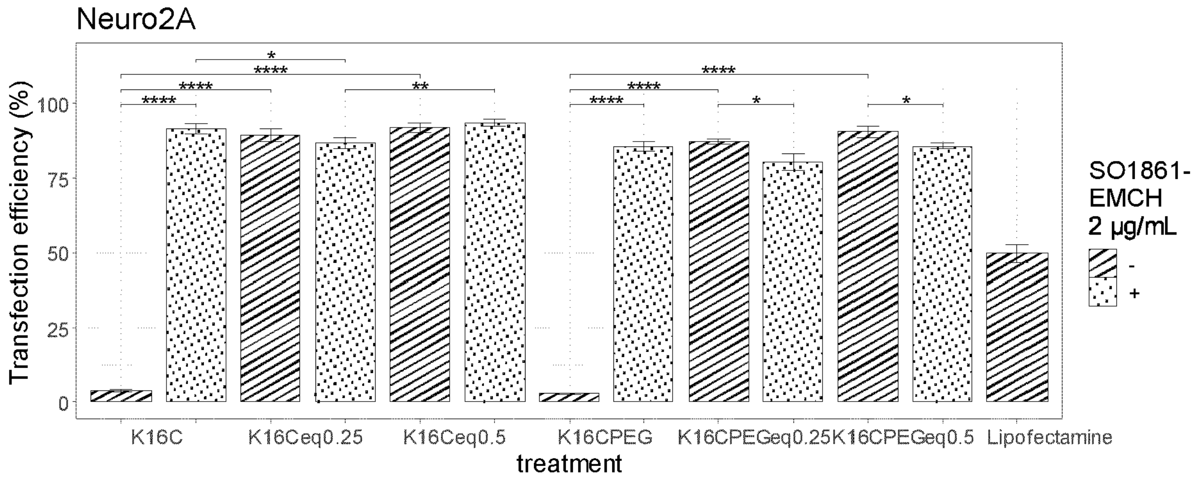
Neuro2A
Transfection efficiency (%)
0
25
50
75
100
****
*
**
SO1861-
EMCH
2 µg/mL
-
+
K16C
K16Ceq0.25
K16Ceq0.5
K16CPEG
K16CPEGeq0.25
K16CPEGeq0.5
Lipofectamine
treatment

## B

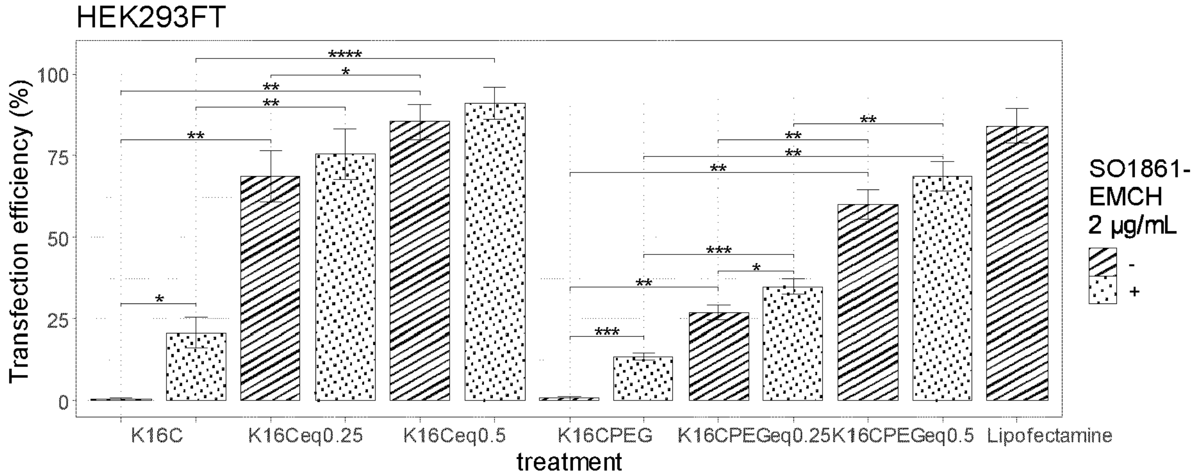
HEK293FT
Transfection efficiency (%)
0
25
50
75
100
****
***
**
*
SO1861-
EMCH
2 µg/mL
-
+
K16C
K16Ceq0.25
K16Ceq0.5
K16CPEG
K16CPEGeq0.25
K16CPEGeq0.5
Lipofectamine
treatment

**FIG. 16**

**C**

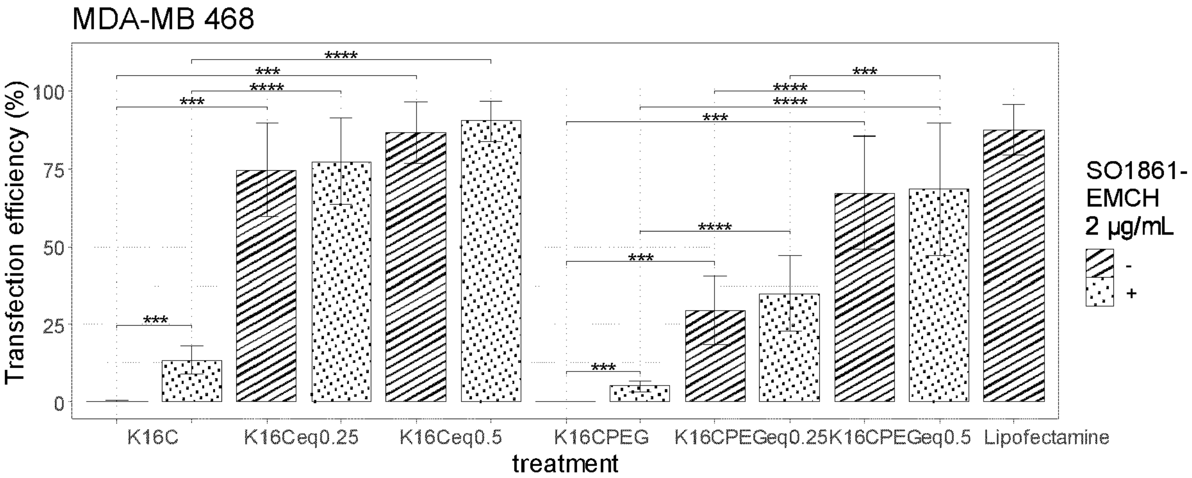
MDA-MB 468
Transfection efficiency (%)
0
25
50
75
100
***
****
K16C
K16Ceq0.25
K16Ceq0.5
K16CPEG
K16CPEGeq0.25
K16CPEGeq0.5
Lipofectamine
treatment
SO1861-EMCH 2 µg/mL
-
+

**D**

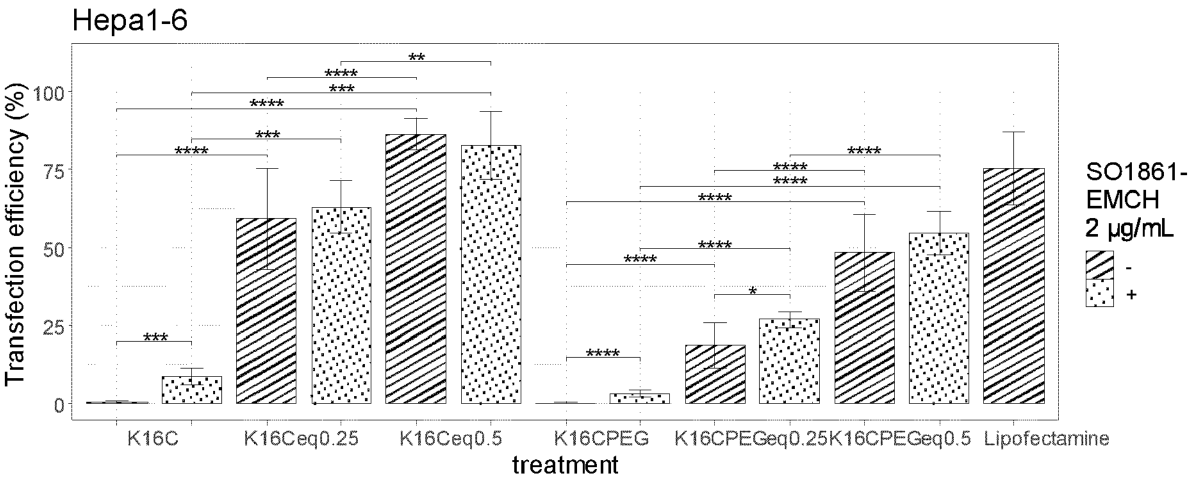
Hepa1-6
Transfection efficiency (%)
0
25
50
75
100
*
**
***
****
K16C
K16Ceq0.25
K16Ceq0.5
K16CPEG
K16CPEGeq0.25
K16CPEGeq0.5
Lipofectamine
treatment
SO1861-EMCH 2 µg/mL
-
+

FIG. 17

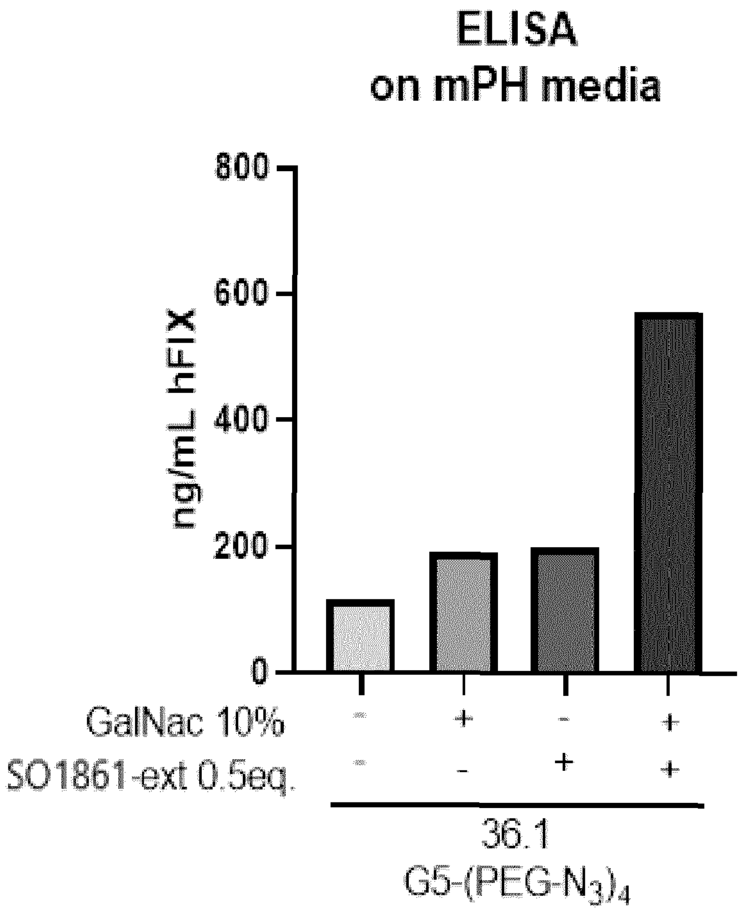
ELISA
on mPH media
ng/mL hFIX
800
600
400
200
0
GalNac 10%
- + - +
SO1861-ext 0.5eq.
- - + +
36.1
G5-(PEG-N3)4

FIG. 18

A

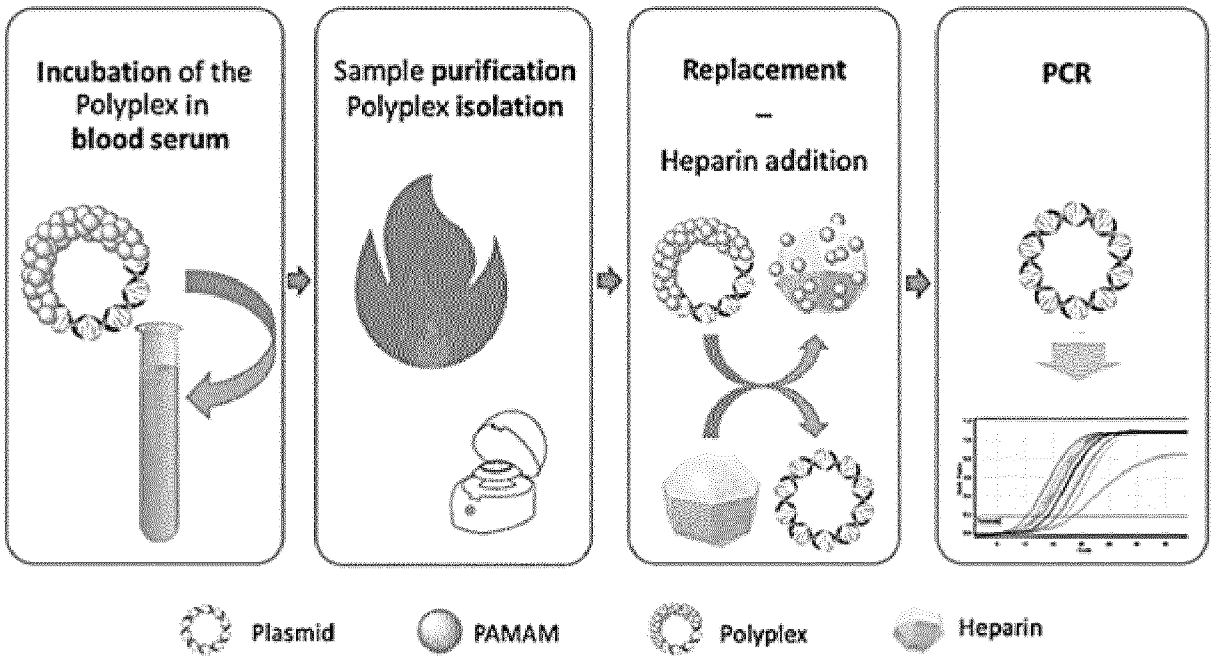
Incubation of the Polyplex in blood serum
Sample purification Polyplex isolation
Replacement
–
Heparin addition
PCR
Plasmid
PAMAM
Polyplex
Heparin

B

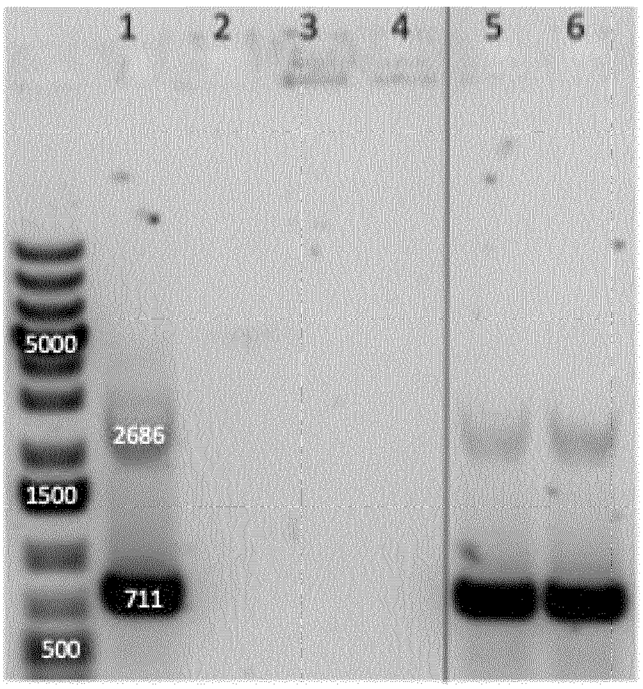
1
2
3
4
5
6
5000
2686
1500
711
500

**FIG. 19**

**A**

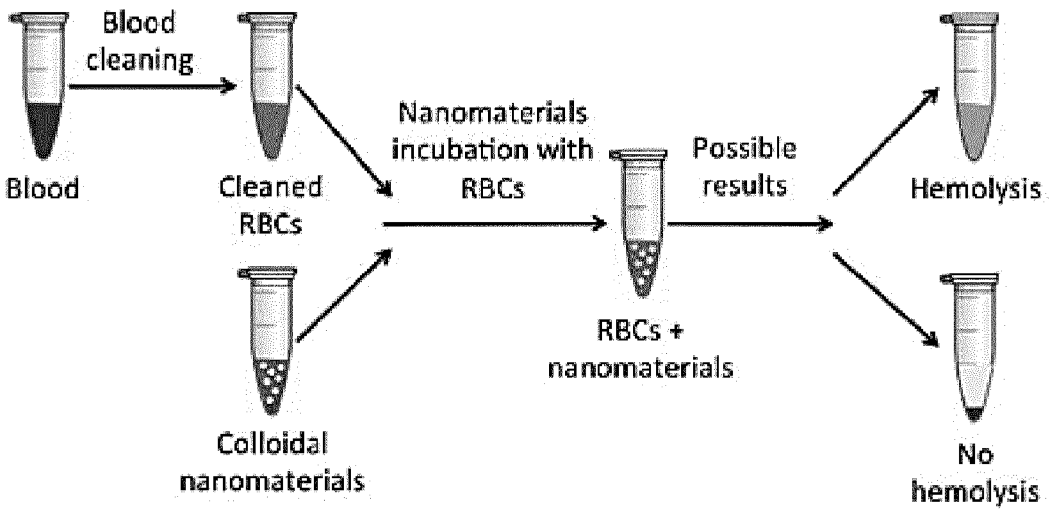
Blood cleaning
Blood
Cleaned RBCs
Colloidal nanomaterials
Nanomaterials incubation with RBCs
RBCs + nanomaterials
Possible results
Hemolysis
No hemolysis

**B**

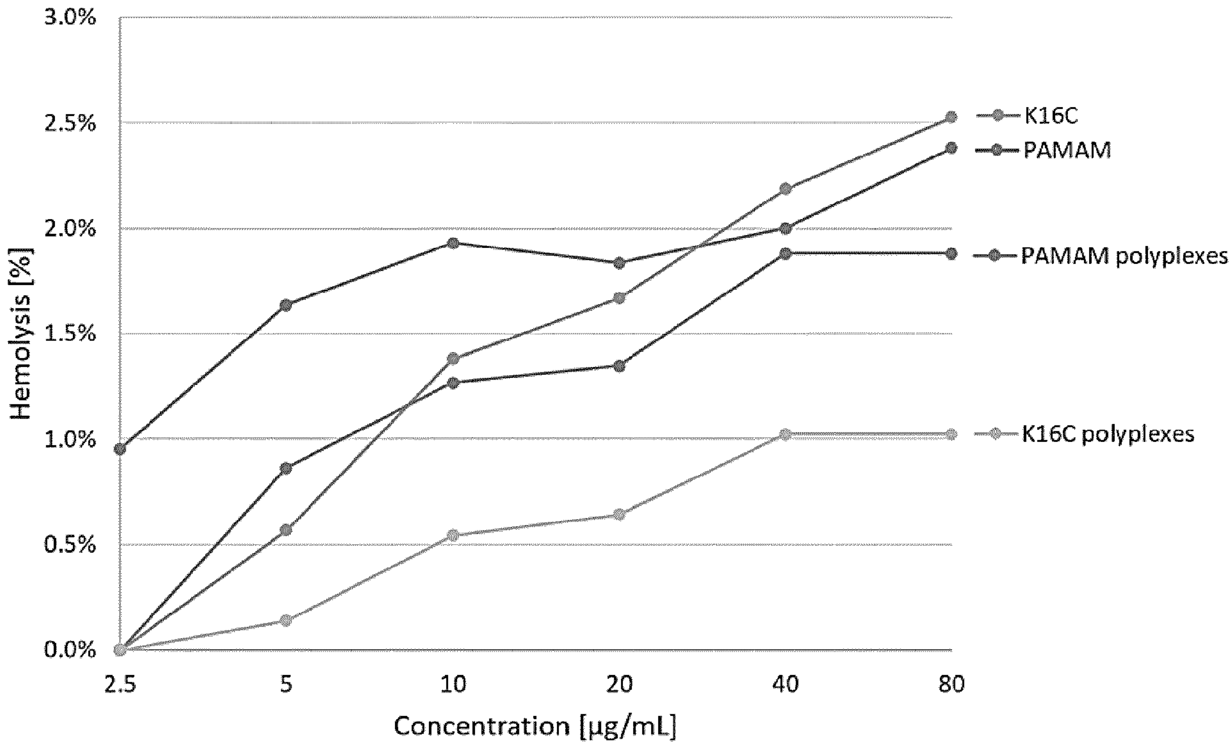
3.0%
2.5%
2.0%
1.5%
1.0%
0.5%
0.0%
Hemolysis [%]
2.5
5
10
20
40
80
Concentration [µg/mL]
K16C
PAMAM
PAMAM polyplexes
K16C polyplexes

**FIG. 19**

**C**

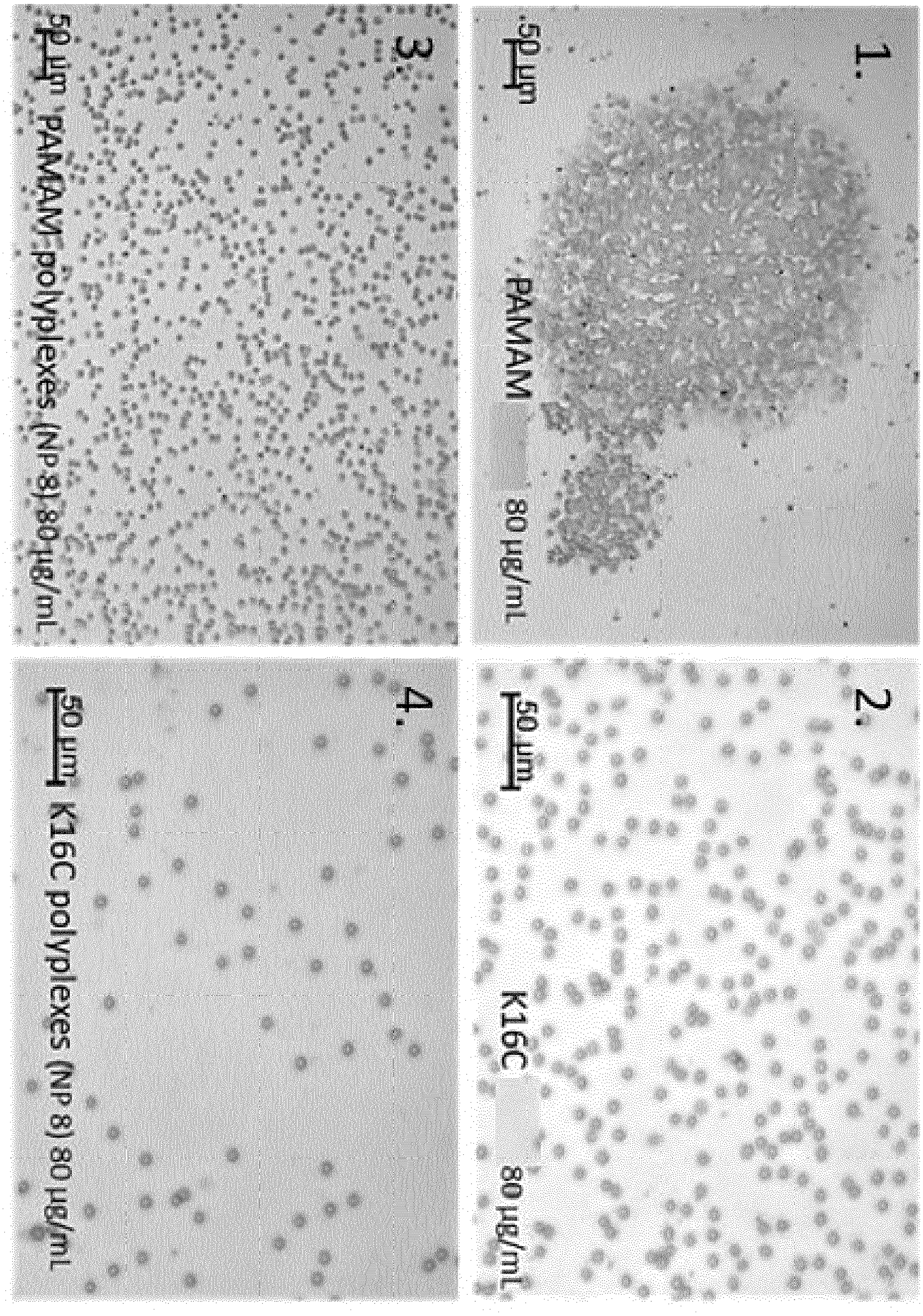
1.
50 µm
PAMAM 80 µg/mL
2.
50 µm
K16C 80 µg/mL
3.
50 µm
PAMAM-polyplexes (NP 8) 80 µg/mL
4.
50 µm
K16C polyplexes (NP 8) 80 µg/mL

Europäisches Patentamt
European Patent Office
Office européen des brevets

# EUROPEAN SEARCH REPORT

Application Number
EP 22 19 3416

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 2020/126064 A1 (SAPREME TECH BV [NL]; UNIV BERLIN CHARITE [DE]) 25 June 2020 (2020-06-25)<br>* figures 68, 70 *<br>* claims 1, 5, 13, 33, 34, 36, 38, 42 *<br>* page 60, line 15 to page 61, line 26 *<br>* page 65, lines 10-19 *<br>* example 14 *<br>----- | 22,23, 26,30<br>1-30 | INV.<br>A61K47/59<br>A61K47/64<br>A61K47/68<br>A61P35/00<br>A61P7/00 |
| Y | QI RONG ET AL: "PEG-conjugated PAMAM Dendrimers Mediate Efficient Intramuscular Gene Expression", THE AAPS JOURNAL, SPRINGER US, BOSTON, vol. 11, no. 3, 29 May 2009 (2009-05-29), pages 395-405, XP035719034, DOI: 10.1208/S12248-009-9116-1 [retrieved on 2009-05-29]<br>* the whole document *<br>----- | 1-30 | |
| Y | KWOH D Y ET AL: "Stabilization of poly-l-lysine/DNA polyplexes for in vivo gene delivery to the liver", BIOCHIMICA ET BIOPHYSICA ACTA . GENE STRUCTURE AND EXPRESSION, ELSEVIER, AMSTERDAM, NL, vol. 1444, no. 2, 16 February 1999 (1999-02-16), pages 171-190, XP004275308, ISSN: 0167-4781, DOI: 10.1016/S0167-4781(98)00274-7<br>* whole document and in particular section 3.4 *<br>----- | 1-30 | TECHNICAL FIELDS SEARCHED (IPC)<br>A61K<br>A61P |

The present search report has been drawn up for all claims

| Place of search | Date of completion of the search | Examiner |
|---|---|---|
| The Hague | 15 February 2023 | Birikaki, Lemonia |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 22 19 3416

This annex lists the patent family members relating to the patent documents cited in the above-mentioned European search report. The members are as contained in the European Patent Office EDP file on
The European Patent Office is in no way liable for these particulars which are merely given for the purpose of information.

15-02-2023

| Patent document cited in search report | Publication date | Patent family member(s) | Publication date |
|---|---|---|---|
| WO 2020126064 A1 | 25-06-2020 | AU 2019400630 A1 | 12-08-2021 |
| | | AU 2019407234 A1 | 19-08-2021 |
| | | AU 2019408811 A1 | 12-08-2021 |
| | | AU 2019411276 A1 | 12-08-2021 |
| | | AU 2019411278 A1 | 12-08-2021 |
| | | AU 2019411289 A1 | 12-08-2021 |
| | | CA 3123978 A1 | 25-06-2020 |
| | | CA 3124064 A1 | 25-06-2020 |
| | | CA 3124065 A1 | 25-06-2020 |
| | | CA 3124129 A1 | 25-06-2020 |
| | | CA 3124151 A1 | 25-06-2020 |
| | | CA 3124406 A1 | 25-06-2020 |
| | | CN 113453722 A | 28-09-2021 |
| | | CN 113474009 A | 01-10-2021 |
| | | CN 113474010 A | 01-10-2021 |
| | | CN 113498350 A | 12-10-2021 |
| | | CN 113507941 A | 15-10-2021 |
| | | CN 113747923 A | 03-12-2021 |
| | | DK 3773737 T3 | 04-10-2021 |
| | | EP 3773737 A1 | 17-02-2021 |
| | | EP 3808379 A1 | 21-04-2021 |
| | | EP 3856254 A2 | 04-08-2021 |
| | | EP 3897735 A1 | 27-10-2021 |
| | | EP 3897738 A1 | 27-10-2021 |
| | | EP 3897739 A1 | 27-10-2021 |
| | | EP 3897741 A1 | 27-10-2021 |
| | | EP 3897742 A2 | 27-10-2021 |
| | | EP 3897743 A1 | 27-10-2021 |
| | | EP 3915587 A1 | 01-12-2021 |
| | | EP 4015003 A1 | 22-06-2022 |
| | | ES 2899612 T3 | 14-03-2022 |
| | | IL 284265 A | 31-08-2021 |
| | | IL 284272 A | 31-08-2021 |
| | | IL 284273 A | 31-08-2021 |
| | | IL 284274 A | 31-08-2021 |
| | | IL 284276 A | 31-08-2021 |
| | | IL 284278 A | 31-08-2021 |
| | | IL 284279 A | 31-08-2021 |
| | | IL 284280 A | 31-08-2021 |
| | | JP 2022515250 A | 17-02-2022 |
| | | JP 2022515251 A | 17-02-2022 |
| | | JP 2022515797 A | 22-02-2022 |
| | | JP 2022516043 A | 24-02-2022 |
| | | JP 2022516044 A | 24-02-2022 |
| | | JP 2022516045 A | 24-02-2022 |
| | | KR 20210107073 A | 31-08-2021 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 22 19 3416

This annex lists the patent family members relating to the patent documents cited in the above-mentioned European search report. The members are as contained in the European Patent Office EDP file on
The European Patent Office is in no way liable for these particulars which are merely given for the purpose of information.

15-02-2023

| Patent document cited in search report | Publication date | Patent family member(s) | Publication date |
|---|---|---|---|
| | | KR 20210110323 A | 07-09-2021 |
| | | KR 20210117274 A | 28-09-2021 |
| | | KR 20210117275 A | 28-09-2021 |
| | | KR 20210117276 A | 28-09-2021 |
| | | KR 20210119976 A | 06-10-2021 |
| | | KR 20210119978 A | 06-10-2021 |
| | | KR 20210119979 A | 06-10-2021 |
| | | SG 11202106572Q A | 29-07-2021 |
| | | SG 11202106574U A | 29-07-2021 |
| | | SG 11202106575Y A | 29-07-2021 |
| | | SG 11202106603U A | 29-07-2021 |
| | | SG 11202106606R A | 29-07-2021 |
| | | SG 11202106612Q A | 29-07-2021 |
| | | SG 11202106664P A | 29-07-2021 |
| | | SG 11202106672S A | 29-07-2021 |
| | | US 2022023433 A1 | 27-01-2022 |
| | | US 2022054643 A1 | 24-02-2022 |
| | | US 2022072149 A1 | 10-03-2022 |
| | | US 2022111066 A1 | 14-04-2022 |
| | | US 2022218837 A1 | 14-07-2022 |
| | | US 2022313834 A1 | 06-10-2022 |
| | | WO 2020126064 A1 | 25-06-2020 |
| | | WO 2020126600 A1 | 25-06-2020 |
| | | WO 2020126604 A1 | 25-06-2020 |
| | | WO 2020126609 A2 | 25-06-2020 |
| | | WO 2020126610 A1 | 25-06-2020 |
| | | WO 2020126620 A2 | 25-06-2020 |
| | | WO 2020126626 A1 | 25-06-2020 |
| | | WO 2020126627 A1 | 25-06-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5693780 A
- EP 3773737 A


### Non-patent literature cited in the description


- **HOLLIGER, P et al.** *Proc. Natl. Acad. Sci. USA,* 1993, vol. 90, 6444-6448
- **POLJAK, R. J. et al.** *Structure,* 1994, vol. 2, 1121-1123
- **KIPRIYANOV, S. M. et al.** *Human Antibodies and Hybridomas,* 1995, vol. 6, 93-101
- **KIPRIYANOV, S. M. et al.** *Mol. Immunol.,* 1994, vol. 31, 1047-1058
- **DIRK C. ; VAN SETTEN ; , GERRIT VAN DE WERKEN ; GIJSBERT ZOMER ; GIDEON F. A. KERSTEN.** Glycosyl Compositions and Structural Characteristics of the Potential Immuno-adjuvant Active Saponins in the Quillaja saponaria Molina Extract Quil A. *RAPID COMMUNICATIONS IN MASS SPECTROMETRY,* 1995, vol. 9, 660-666
- **JULIANE DEISE FLECK ; ANDRESA HEEMANN BETTI ; FRANCINI PEREIRA DA SILVA ; EDUARDO ARTUR TROIAN ; CRISTINA OLIVARO ; FERNANDO FERREIRA ; SIMONE GASPARIN VERZA.** Saponins from Quillaja saponaria and Quillaja brasiliensis: Particular Chemical Characteristics and Biological Activities. *Molecules,* 2019, vol. 24, 171
- **JIA et al.** Major Triterpenoid Saponins from Saponaria officinalis. *J. Nat. Prod.,* 19 September 1998, vol. 61 (11), 1368-1373, https://doi.org/10.1021/np980167u
- **J. CLOCHARD et al.** A new acetylated triterpene saponin from Agrostemma githago L. modulates gene delivery efficiently and shows a high cellular tolerance. *International Journal of Pharmaceutics,* 15 November 2020, vol. 589, 119822
- **MONIUSZKO-SZAJWAJ et al.** Highly Polar Triterpenoid Saponins from the Roots of Saponaria officinalis L. *Helv. Chim. Acta,* 2016, vol. 99, 347-354
- **DR STEFAN BÖTTGER.** Untersuchungen zur synergistischen Zytotoxizität zwischen Saponinen und Ribosomen inaktivierenden Proteinen Typ I. *thesis,* 2013
- **SAMA et al.** Structure-Activity Relationship of Transfection-Modulating Saponins - A Pursuit for the Optimal Gene Trafficke. *Planta Med.,* 2019, vol. 85, 513-518
- **FUCHS et al.** Glycosylated Triterpenoids as Endosomal Escape Enhancers in Targeted Tumor Therapies. *Biomedicine,* 2017, vol. 5 (14
- **SAMA.** Sapofectosid - Ensuring non-toxic and effective DNA and RNA delivery. *INTERNATIONAL JORNAL OF PHARMACEUTICS,* 20 December 2017, vol. 534 (1-2), 195-205
- *Tetrahedron Letters,* 1963, vol. 8, 477-482
- **AGARWAL, S. et al.** PDMAEMA based gene delivery materials. *Mater. Today,* 2012, vol. 15 (9), 388-393
- **BERKHOUT B ; LIU YP.** Towards improved shRNA and miRNA reagents as inhibitors of HIV-1 replication. *Future Microbiol,* 2014, vol. 9, 561-571
- **BÖTTGER S.** Untersuchungen zur synergistischen Zytotoxizität zwischen Saponinen und Ribosomen inaktivierenden Proteinen Typ I. *PhD thesis,* 2013
- **CLIFFORD, A. et al.** Biomimetic modification of poly-L-lysine and electrodeposition of nanocomposite coatings for orthopaedic applications. *Colloids Surf. B,* 2019, vol. 176, 115-121
- **CLOCHARD J et al.** A new acetylated triterpene saponin from Agrostemma githago L. modulates gene delivery efficiently and shows a high cellular tolerance. *International Journal of Pharmaceutics,* 15 November 2020, vol. 589, 119822
- **DÉMOULINS, T. et al.** Polyethylenimine-based polyplex delivery of self-replicating RNA vaccines. *Nanomedicine,* 2016, vol. 12 (3), 711-722
- **DUFES C et al.** *Adv Drug Deliv Rev,* 2005, vol. 57, 2177
- **FLECK ; ULIANE DEISE FLECK ; ANDRESA HEEMANN BETTI ; FRANCINI PEREIRA DA SILVA ; EDUARDO ARTUR TROIAN ; CRISTINA OLIVARO ; FERNANDO FERREIRA ; SIMONE GASPARIN VERZA et al.** Saponins from Quillaja saponaria and Quillaja brasiliensis: Particular Chemical Characteristics and Biological Activities. *Molecules,* 2019, vol. 24, 171
- **FUCHS H et al.** Glycosylated Triterpenoids as Endosomal Escape Enhancers in Targeted Tumor Therapies. *Biomedicines,* 2017, vol. 5

- **GEORGE LA et al.** Hemophilia B Gene Therapy with a High-Specific-Activity Factor IX Variant. *N Engl J Med,* 2017, vol. 377, 2215-2227
- **GILLERON J et al.** Image based analysis of lipid nanoparticle-mediated siRNA delivery, intracellular trafficking and endosomal escape. *Nat Biotechnol,* 2013, vol. 31, 638-646
- **HE, Y. et al.** Polyethyleneimine/DNA polyplexes with reduction-sensitive hyaluronic acid derivatives shielding for targeted gene delivery. *Biomaterials,* 2013, vol. 34 (4), 1235-1245
- **HESS, K.L. et al.** Polyplexes assembled from self-peptides and regulatory nucleic acids blunt toll-like receptor signaling to combat autoimmunity. *Biomaterials,* 2017, vol. 118, 51-62
- **KABANOV, A.V. ; KABANOV, V.A.** DNA complexes with polycations for the delivery of genetic material into cells. *Bioconjug Chem,* 1995, vol. 6 (1), 7-20
- **KABAT, E.A. ; WU, T.T. ; PERRY, H.M. ; GOTTESMAN, K.S. ; FOELLER, C.** Sequences of Proteins of Immunological Interest. 1991
- **KOCHETKOV N.K. ; KHORLIN A.J. ; OVODOV J.S.** *The structure of gypsoside - triterpenic saponin from gypsophila pacifica kom..,* 1963, vol. 4 (8), 477-482
- **KODAMA, Y. et al.** Biodegradable nanoparticles composed of dendrigraft poly-Llysine for gene delivery. *Eur. J. Pharm. Biopharmaceutics,* 2014, vol. 87 (3), 472-479
- **KOPING-HOGGARD, M. et al.** Improved chitosan-mediated gene delivery based on easily dissociated chitosan polyplexes of highly defined chitosan oligomers. *Gene Ther.,* 2004, vol. 11 (19), 1441-1452
- **KOZIELSKI, K.L. ; RUI, Y. ; GREEN, J.J.** Non-viral nucleic acid containing nanoparticles as cancer therapeutics. *Expert Opin Drug Deliv,* 2016, vol. 13 (10), 1475-1487
- **KWOH, D.Y. et al.** Stabilization of poly-L-lysine/DNA polyplexes for in vivo gene delivery to the liver. *Biochim. Biophys. Acta,* 1999, vol. 1444 (2), 171-190
- **LEE CS et al.** Adenovirus-Mediated Gene Delivery: Potential Applications for Gene and Cell based Therapies in the New Era of Personalized Medicine. *Genes Dis,* 2017, vol. 4, 43-63
- **MALI P et al.** RNA-guided human genome engineering via Cas9. *Science,* 2013, vol. 339, 823-826
- **PURAS, G. et al.** Oligochitosan polyplexes as carriers for retinal gene delivery. *Eur. J. Pharm.Sci.,* 2013, vol. 48 (1), 323-331
- **RUMSCHÖTTEL, J. et al.** DNA polyplexes with dendritic glycopolymer-entrapped gold nanoparticles. *Colloids and Surf,* 2017, vol. 154, 74-81
- **SAMA S et al.** Sapofectosid - Ensuring non-toxic and effective DNA and RNA delivery. *International Journal of Pharmaceutics,* 20 December 2017, vol. 534 (1-2), 195-205
- **SAMA S et al.** Structure-Activity Relationship of Transfection-Modulating Saponins - A Pursuit for the Optimal Gene Trafficker. *Planta Med.,* 2019, vol. 85, 513-518
- **SAMA S et al.** Targeted suicide gene transfections reveal promising results in nu/nu mice with aggressive neuroblastoma. *J Control Release,* 2018, vol. 275, 208-216
- **TROS DE ILARDUYA, C. ; SUN, Y. ; DÜZGÜNES, N.** Gene delivery by lipoplexes and polyplexes. *Eur. J. Pharm. Sci.,* 2010, vol. 40 (3), 159-170
- **DIRK C. VAN SETTEN ; GERRIT VAN DE WERKEN ; GIJSBERT ZOMER ; GIDEON F. A. KERSTEN.** Glycosyl Compositions and Structural Characteristics of the Potential Immuno-adjuvant Active Saponins in the Quillaja saponaria Molina Extract Quil A. *RAPID COMMUNICATIONS IN MASS SPECTROMETRY,* 1995, vol. 9, 660-666
- **VASILIU, T. et al.** Optimization of polyplex formation between DNA oligonucleotide and Poly(l-Lysine): experimental study and modeling approach. *Int. J. Mol. Sci.,* 2017, vol. 18 (6), 1291
- **WU, G.Y. ; WU, C.H.** Receptor-mediated in vitro gene transformation by a soluble DNA carrier system. *J. Biol. Chem.,* 1987, vol. 262 (10), 4429-4432
- **ZHANG, X. et al.** Poly(L-lysine) nanostructured particles for gene delivery and hormone stimulation. *Biomaterials,* 2010, vol. 31 (7), 1699-1706
- **ZIADY, A.-.G. et al.** Transfection of airway epithelium by stable PEGylated poly-Llysine DNA nanoparticles in vivo. *Mol. Ther.,* 2003, vol. 8 (6), 936-947